# EUROPEAN PATENT SPECIFICATION

(11) **EP 3 606 546 B1**
(45) Date of publication and mention of the grant of the patent: **28.04.2021**
(21) Application number: 18715911.6
(22) Date of filing: 28.03.2018
(51) Int. Cl.: A61K 38/17, A61K 38/18, A61P 25/28, A61P 27/06, C12N 15/63, C12N 15/861

(54) **GENETIC CONSTRUCT FOR USE IN THE TREATMENT OF NEURODEGENERATIVE DISORDER OR STROKE**
GENETISCHES KONSTRUKT ZUR VERWENDUNG BEI DER BEHANDLUNG VON NEURODEGENERATIVER ERKRANKUNG ODER SCHLAGANFALL
CONSTRUCTION GÉNÉTIQUE DESTINÉE À ÊTRE UTILISÉE DANS LE TRAITEMENT D'UN TROUBLE NEURODÉGÉNÉRATIF OU D'UN ACCIDENT VASCULAIRE CÉRÉBRAL

(30) Priority: 05.04.2017 GB 201705484
(43) Date of publication of application: 12.02.2020
(73) Proprietor: Quethera Limited, Canterbury, Kent CT1 3DN (GB)
(72) Inventor: WIDDOWSON, Peter, Canterbury Kent CT1 3DN (GB); MARTIN, Keith, Canterbury Kent CT1 3DN (GB)
(74) Representative: Hutter, Anton
(86) International application number: PCT/GB2018/050824
(87) International publication number: WO 2018/185468

(56) References cited:
- WO-A1-2017/072498
- WO-A2-2009/120978
- US-A1- 2003 124 095
- Prweb: "PRWeb ebooks -Another online visibility tool from PRWeb Neuroprotective Therapy Company Quethera Reveals Gene Therapy Results at ARVO", , 18 May 2017 (2017-05-18), XP055474950, Retrieved from the Internet: URL:http://www.prweb.com/pdfdownload/14339 704.pdf [retrieved on 2018-05-15]
- ANDREW OSBORNE ET AL: "Design of a Novel Gene Therapy Construct to Achieve Sustained Brain-Derived Neurotrophic Factor Signaling in Neurons", HUMAN GENE THERAPY, 2 April 2018 (2018-04-02), XP055475037, & CONFERENCE ON CHANGING THE FACE OF MODERN MEDICINE - STEM CELLS AND GENE THERAPY; FLORENCE, ITALY; OCTOBER 18 -21, 2016 ISSN: 1043-0342, DOI: 10.1089/hum.2017.069
- GILL LUTHER C ET AL: "Functional recovery after cervical spinal cord injury: Role of neurotrophin and glutamatergic signaling in phrenic motoneurons", RESPIRATORY PHYSIOLOGY AND NEUROBIOLOGY, vol. 226, 18 May 2017 (2017-05-18), pages 128-136, XP029867116, ISSN: 1569-9048, DOI: 10.1016/J.RESP.2015.10.009
- CHENG L ET AL: "TrkB Gene Transfer Protects Retinal Ganglion Cells from Axotomy-Induced Death In Vivo", THE JOURNAL OF NEUROSCIENCE : THE OFFICIAL JOURNAL OF THE SOCIETY FOR NEUROSCIENCE,, vol. 22, no. 10, 15 May 2002 (2002-05-15), pages 3977-3986, XP008133696, ISSN: 0270-6474
- ALAN H. NAGAHARA ET AL: "Potential therapeutic uses of BDNF in neurological and psychiatric disorders", NATURE REVIEWS. DRUG DISCOVERY, vol. 10, no. 3, 1 March 2011 (2011-03-01), pages 209-219, XP55263635, GB ISSN: 1474-1776, DOI: 10.1038/nrd3366
- MARTIN KEITH R G ET AL: "Gene therapy with brain-derived neurotrophic factor as a protection: retinal ganglion cells in a rat glaucoma model", INVESTIGATIVE OPHTHALMOLOGY & VISUAL SCIENCE - IOVS, ASSOCIATION FOR RESEARCH IN VISION AND OPHTHALMOLOGY, US, vol. 44, no. 10, 1 October 2003 (2003-10-01), pages 4357-4365, XP009158081, ISSN: 0146-0404, DOI: 10.1167/IOVS.02-1332

### SEQUENCE LISTING

<110> Quethera Limited
<120> Genetic Construct
<130> 83344PCT1
<160> 108
<170> PatentIn version 3.5
<210> 1
   <211> 469
   <212> DNA
   <213> Homo sapiens
<400> 1
   ctgcagaggg ccctgcgtat gagtgcaagt gggttttagg accaggatga ggcggggtgg      60
   gggtgcctac ctgacgaccg accccgaccc actggacaag cacccaaccc ccattcccca     120
   aattgcgcat cccctatcag agagggggag gggaaacagg atgcggcgag gcgcgtgcgc     180
   actgccagct tcagcaccgc ggacagtgcc ttcgcccccg cctggcggcg cgcgccaccg     240
   ccgcctcagc actgaaggcg cgctgacgtc actcgccggt cccccgcaaa ctccccttcc     300
   cggccacctt ggtcgcgtcc gcgccgccgc cggcccagcc ggaccgcacc acgcgaggcg     360
   cgagataggg gggcacgggc gcgaccatct gcgctgcggc gccggcgact cagcgctgcc     420
   tcagtctgcg gtgggcagcg gaggagtcgt gtcgtgcctg agagcgcag                 469
<210> 2
   <211> 1733
   <212> DNA
   <213> Homo sapiens
<400> 2
   ctcgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtacgcccc ctattgacgt caatgacggt aaatggcccg 300
   cctggcatta tgcccagtac atgaccttat gggactttcc tacttggcag tacatctacg 360
   tattagtcat cgctattacc atggtcgagg tgagccccac gttctgcttc actctcccca 420
   tctccccccc ctccccaccc ccaattttgt atttatttat tttttaatta ttttgtgcag 480
   cgatgggggc gggggggggg ggggggcgcg cgccaggcgg ggcggggcgg ggcgaggggc 540
   ggggcggggc gaggcggaga ggtgcggcgg cagccaatca gagcggcgcg ctccgaaagt 600
   ttccttttat ggcgaggcgg cggcggcggc ggccctataa aaagcgaagc gcgcggcggg 660
   cgggagtcgc tgcgcgctgc cttcgccccg tgccccgctc cgccgccgcc tcgcgccgcc 720
   cgccccggct ctgactgacc gcgttactcc cacaggtgag cgggcgggac ggcccttctc 780
   ctccgggctg taattagcgc ttggtttaat gacggcttgt ttcttttctg tggctgcgtg 840
   aaagccttga ggggctccgg gagggccctt tgtgcggggg gagcggctcg gggggtgcgt 900
   gcgtgtgtgt gtgcgtgggg agcgccgcgt gcggctccgc gctgcccggc ggctgtgagc 960
   gctgcgggcg cggcgcgggg ctttgtgcgc tccgcagtgt gcgcgagggg agcgcggccg 1020
   ggggcggtgc cccgcggtgc ggggggggct gcgaggggaa caaaggctgc gtgcggggtg 1080
   tgtgcgtggg ggggtgagca gggggtgtgg gcgcgtcggt cgggctgcaa ccccccctgc 1140
   acccccctcc ccgagttgct gagcacggcc cggcttcggg tgcggggctc cgtacggggc 1200
   gtggcgcggg gctcgccgtg ccgggcgggg ggtggcggca ggtgggggtg ccgggcgggg 1260
   cggggccgcc tcgggccggg gagggctcgg gggaggggcg cggcggcccc cggagcgccg 1320
   gcggctgtcg aggcgcggcg agccgcagcc attgcctttt atggtaatcg tgcgagaggg 1380
   cgcagggact tcctttgtcc caaatctgtg cggagccgaa atctgggagg cgccgccgca 1440
   ccccctctag cgggcgcggg gcgaagcggt gcggcgccgg caggaaggaa atgggcgggg 1500
   agggccttcg tgcgtcgccg cgccgccgtc cccttctccc tctccagcct cggggctgtc 1560
   cgcgggggga cggctgcctt cgggggggac ggggcagggc ggggttcggc ttctggcgtg 1620
   tgaccggcgg ctctagagcc tctgctaacc atgttcatgc cttcttcttt ttcctacagc 1680
   tcctgggcaa cgtgctggtt attgtgctgt ctcatcattt tggcaaagaa ttg 1733
<210> 3
   <211> 664
   <212> DNA
   <213> Homo sapiens
<400> 3
   ctagatctga attcggtacc ctagttatta atagtaatca attacggggt cattagttca        60
   tagcccatat atggagttcc gcgttacata acttacggta aatggcccgc ctggctgacc       120
   gcccaacgac ccccgcccat tgacgtcaat aatgacgtat gttcccatag taacgccaat       180
   agggactttc cattgacgtc aatgggtgga ctatttacgg taaactgccc acttggcagt       240
   acatcaagtg tatcatatgc caagtacgcc ccctattgac gtcaatgacg gtaaatggcc       300
   cgcctggcat tatgcccagt acatgacctt atgggacttt cctacttggc agtacatcta       360
   cgtattagtc atcgctatta ccatggtcga ggtgagcccc acgttctgct tcactctccc       420
   catctccccc ccctccccac ccccaatttt gtatttattt attttttaat tattttgtgc       480
   agcgatgggg gcgggggggg ggggggggcg cgcgccaggc ggggcggggc ggggcgaggg       540
   gcggggcggg gcgaggcgga gaggtgcggc ggcagccaat cagagcggcg cgctccgaaa       600
   gtttcctttt atggcgaggc ggcggcggcg gcggccctat aaaaagcgaa gcgcgcggcg       660
   ggcg                                                                    664
<210> 4
   <211> 11
   <212> PRT
   <213> Homo sapiens
<400> 4
   Gln Ala Gly Asp Val Glu Glu Asn Pro Gly Pro
   1               5                   10
<210> 5
   <211> 63
   <212> DNA
   <213> Homo sapiens
<400> 5
   ggaagcggag ctactaactt cagcctgctg aaggctggag acgtggagga gaaccctgga        60
   cct                                                                      63
<210> 6
   <211> 21
   <212> PRT
   <213> Homo sapiens
<400> 6
   Gly Ser Gly Ala Thr Asn Phe Ser Leu Leu Gln Ala Gly Asp Val Glu
   1               5                   10                  15
   Glu Asn Pro Gly Pro
               20
<210> 7
   <211> 63
   <212> DNA
   <213> Homo sapiens
<400> 7
   agcggagcta ctaacttcag cctgctgaag caggctggag acgtggagga gaaccctgga      60
   cct                                                                   63
<210> 8
   <211> 21
   <212> PRT
   <213> Homo sapiens
<400> 8
   Ser Gly Ala Thr Asn Phe Ser Leu Leu Lys Gln Ala Gly Asp Val Glu
   1               5                   10                  15
   Glu Asn Pro Gly Pro
               20
<210> 9
   <211> 822
   <212> PRT
   <213> Homo sapiens
<400> 9
   Met Ser Ser Trp Ile Arg Trp His Gly Pro Ala Met Ala Arg Leu Trp
   1               5                   10                  15
   Gly Phe Cys Trp Leu Val Val Gly Phe Trp Arg Ala Ala Phe Ala Cys
               20                  25                  30
   Pro Thr Ser Cys Lys Cys Ser Ala Ser Arg Ile Trp Cys Ser Asp Pro
           35                  40                  45
   Ser Pro Gly Ile Val Ala Phe Pro Arg Leu Glu Pro Asn Ser Val Asp
       50                  55                  60
   Pro Glu Asn Ile Thr Glu Ile Phe Ile Ala Asn Gln Lys Arg Leu Glu
   65                  70                  75                  80
   Ile Ile Asn Glu Asp Asp Val Glu Ala Tyr Val Gly Leu Arg Asn Leu
                   85                  90                  95
   Thr Ile Val Asp Ser Gly Leu Lys Phe Val Ala His Lys Ala Phe Leu
               100                 105                 110
   Lys Asn Ser Asn Leu Gln His Ile Asn Phe Thr Arg Asn Lys Leu Thr
           115                 120                 125
   Ser Leu Ser Arg Lys His Phe Arg His Leu Asp Leu Ser Glu Leu Ile
       130                 135                 140
   Leu Val Gly Asn Pro Phe Thr Cys Ser Cys Asp Ile Met Trp Ile Lys
   145                 150                 155                 160
   Thr Leu Gln Glu Ala Lys Ser Ser Pro Asp Thr Gln Asp Leu Tyr Cys
                   165                 170                 175
   Leu Asn Glu Ser Ser Lys Asn Ile Pro Leu Ala Asn Leu Gln Ile Pro
               180                 185                 190
   Asn Cys Gly Leu Pro Ser Ala Asn Leu Ala Ala Pro Asn Leu Thr Val
           195                 200                 205
   Glu Glu Gly Lys Ser Ile Thr Leu Ser Cys Ser Val Ala Gly Asp Pro
       210                 215                 220
   Val Pro Asn Met Tyr Trp Asp Val Gly Asn Leu Val Ser Lys His Met
   225                 230                 235                 240
   Asn Glu Thr Ser His Thr Gln Gly Ser Leu Arg Ile Thr Asn Ile Ser
                   245                 250                 255
   Ser Asp Asp Ser Gly Lys Gln Ile Ser Cys Val Ala Glu Asn Leu Val
               260                 265                 270
   Gly Glu Asp Gln Asp Ser Val Asn Leu Thr Val His Phe Ala Pro Thr
           275                 280                 285
   Ile Thr Phe Leu Glu Ser Pro Thr Ser Asp His His Trp Cys Ile Pro
       290                 295                 300
   Phe Thr Val Lys Gly Asn Pro Lys Pro Ala Leu Gln Trp Phe Tyr Asn
   305                 310                 315                 320
   Gly Ala Ile Leu Asn Glu Ser Lys Tyr Ile Cys Thr Lys Ile His Val
                   325                 330                 335
   Thr Asn His Thr Glu Tyr His Gly Cys Leu Gln Leu Asp Asn Pro Thr
               340                 345                 350
   His Met Asn Asn Gly Asp Tyr Thr Leu Ile Ala Lys Asn Glu Tyr Gly
           355                 360                 365
   Lys Asp Glu Lys Gln Ile Ser Ala His Phe Met Gly Trp Pro Gly Ile
       370                 375                 380
   Asp Asp Gly Ala Asn Pro Asn Tyr Pro Asp Val Ile Tyr Glu Asp Tyr
   385                 390                 395                 400
   Gly Thr Ala Ala Asn Asp Ile Gly Asp Thr Thr Asn Arg Ser Asn Glu
                   405                 410                 415
   Ile Pro Ser Thr Asp Val Thr Asp Lys Thr Gly Arg Glu His Leu Ser
               420                 425                 430
   Val Tyr Ala Val Val Val Ile Ala Ser Val Val Gly Phe Cys Leu Leu
           435                 440                 445
   Val Met Leu Phe Leu Leu Lys Leu Ala Arg His Ser Lys Phe Gly Met
       450                 455                 460
   Lys Gly Pro Ala Ser Val Ile Ser Asn Asp Asp Asp Ser Ala Ser Pro
   465                 470                 475                 480
   Leu His His Ile Ser Asn Gly Ser Asn Thr Pro Ser Ser Ser Glu Gly
                   485                 490                 495
   Gly Pro Asp Ala Val Ile Ile Gly Met Thr Lys Ile Pro Val Ile Glu
               500                 505                 510
   Asn Pro Gln Tyr Phe Gly Ile Thr Asn Ser Gln Leu Lys Pro Asp Thr
           515                 520                 525
   Phe Val Gln His Ile Lys Arg His Asn Ile Val Leu Lys Arg Glu Leu
       530                 535                 540
   Gly Glu Gly Ala Phe Gly Lys Val Phe Leu Ala Glu Cys Tyr Asn Leu
   545                 550                 555                 560
   Cys Pro Glu Gln Asp Lys Ile Leu Val Ala Val Lys Thr Leu Lys Asp
                   565                 570                 575
   Ala Ser Asp Asn Ala Arg Lys Asp Phe His Arg Glu Ala Glu Leu Leu
               580                 585                 590
   Thr Asn Leu Gln His Glu His Ile Val Lys Phe Tyr Gly Val Cys Val
           595                 600                 605
   Glu Gly Asp Pro Leu Ile Met Val Phe Glu Tyr Met Lys His Gly Asp
       610                 615                 620
   Leu Asn Lys Phe Leu Arg Ala His Gly Pro Asp Ala Val Leu Met Ala
   625                 630                 635                 640
   Glu Gly Asn Pro Pro Thr Glu Leu Thr Gln Ser Gln Met Leu His Ile
                   645                 650                 655
   Ala Gln Gln Ile Ala Ala Gly Met Val Tyr Leu Ala Ser Gln His Phe
               660                 665                 670
   Val His Arg Asp Leu Ala Thr Arg Asn Cys Leu Val Gly Glu Asn Leu
           675                 680                 685
   Leu Val Lys Ile Gly Asp Phe Gly Met Ser Arg Asp Val Tyr Ser Thr
       690                 695                 700
   Asp Tyr Tyr Arg Val Gly Gly His Thr Met Leu Pro Ile Arg Trp Met
   705                 710                 715                 720
   Pro Pro Glu Ser Ile Met Tyr Arg Lys Phe Thr Thr Glu Ser Asp Val
                   725                 730                 735
   Trp Ser Leu Gly Val Val Leu Trp Glu Ile Phe Thr Tyr Gly Lys Gln
               740                 745                 750
   Pro Trp Tyr Gln Leu Ser Asn Asn Glu Val Ile Glu Cys Ile Thr Gln
           755                 760                 765
   Gly Arg Val Leu Gln Arg Pro Arg Thr Cys Pro Gln Glu Val Tyr Glu
       770                 775                 780
   Leu Met Leu Gly Cys Trp Gln Arg Glu Pro His Met Arg Lys Asn Ile
   785                 790                 795                 800
   Lys Gly Ile His Thr Leu Leu Gln Asn Leu Ala Lys Ala Ser Pro Val
                   805                 810                 815
   Tyr Leu Asp Ile Leu Gly
               820
<210> 10
   <211> 2466
   <212> DNA
   <213> Homo sapiens
<400> 10
   atgtcgtcct ggataaggtg gcatggaccc gccatggcgc ggctctgggg cttctgctgg        60
   ctggttgtgg gcttctggag ggccgctttc gcctgtccca cgtcctgcaa atgcagtgcc       120
   tctcggatct ggtgcagcga cccttctcct ggcatcgtgg catttccgag attggagcct       180
   aacagtgtag atcctgagaa catcaccgaa attttcatcg caaaccagaa aaggttagaa       240
   atcatcaacg aagatgatgt tgaagcttat gtgggactga gaaatctgac aattgtggat       300
   tctggattaa aatttgtggc tcataaagca tttctgaaaa acagcaacct gcagcacatc       360
   aattttaccc gaaacaaact gacgagtttg tctaggaaac atttccgtca ccttgacttg       420
   tctgaactga tcctggtggg caatccattt acatgctcct gtgacattat gtggatcaag       480
   actctccaag aggctaaatc cagtccagac actcaggatt tgtactgcct gaatgaaagc       540
   agcaagaata ttcccctggc aaacctgcag atacccaatt gtggtttgcc atctgcaaat       600
   ctggccgcac ctaacctcac tgtggaggaa ggaaagtcta tcacattatc ctgtagtgtg       660
   gcaggtgatc cggttcctaa tatgtattgg gatgttggta acctggtttc caaacatatg       720
   aatgaaacaa gccacacaca gggctcctta aggataacta acatttcatc cgatgacagt       780
   gggaagcaga tctcttgtgt ggcggaaaat cttgtaggag aagatcaaga ttctgtcaac 840
   ctcactgtgc attttgcacc aactatcaca tttctcgaat ctccaacctc agaccaccac 900
   tggtgcattc cattcactgt gaaaggcaac cccaaaccag cgcttcagtg gttctataac 960
   ggggcaatat tgaatgagtc caaatacatc tgtactaaaa tacatgttac caatcacacg 1020
   gagtaccacg gctgcctcca gctggataat cccactcaca tgaacaatgg ggactacact 1080
   ctaatagcca agaatgagta tgggaaggat gagaaacaga tttctgctca cttcatgggc 1140
   tggcctggaa ttgacgatgg tgcaaaccca aattatcctg atgtaattta tgaagattat 1200
   ggaactgcag cgaatgacat cggggacacc acgaacagaa gtaatgaaat cccttccaca 1260
   gacgtcactg ataaaaccgg tcgggaacat ctctcggtct atgctgtggt ggtgattgcg 1320
   tctgtggtgg gattttgcct tttggtaatg ctgtttctgc ttaagttggc aagacactcc 1380
   aagtttggca tgaaaggccc agcctccgtt atcagcaatg atgatgactc tgccagccca 1440
   ctccatcaca tctccaatgg gagtaacact ccatcttctt cggaaggtgg cccagatgct 1500
   gtcattattg gaatgaccaa gatccctgtc attgaaaatc cccagtactt tggcatcacc 1560
   aacagtcagc tcaagccaga cacatttgtt cagcacatca agcgacataa cattgttctg 1620
   aaaagggagc taggcgaagg agcctttgga aaagtgttcc tagctgaatg ctataacctc 1680
   tgtcctgagc aggacaagat cttggtggca gtgaagaccc tgaaggatgc cagtgacaat 1740
   gcacgcaagg acttccaccg tgaggccgag ctcctgacca acctccagca tgagcacatc 1800
   gtcaagttct atggcgtctg cgtggagggc gaccccctca tcatggtctt tgagtacatg 1860
   aagcatgggg acctcaacaa gttcctcagg gcacacggcc ctgatgccgt gctgatggct 1920
   gagggcaacc cgcccacgga actgacgcag tcgcagatgc tgcatatagc ccagcagatc 1980
   gccgcgggca tggtctacct ggcgtcccag cacttcgtgc accgcgattt ggccaccagg 2040
   aactgcctgg tcggggagaa cttgctggtg aaaatcgggg actttgggat gtcccgggac 2100
   gtgtacagca ctgactacta cagggtcggt ggccacacaa tgctgcccat tcgctggatg 2160
   cctccagaga gcatcatgta caggaaattc acgacggaaa gcgacgtctg gagcctgggg 2220
   gtcgtgttgt gggagatttt cacctatggc aaacagccct ggtaccagct gtcaaacaat 2280
   gaggtgatag agtgtatcac tcagggccga gtcctgcagc gaccccgcac gtgcccccag 2340
   gaggtgtatg agctgatgct ggggtgctgg cagcgagagc cccacatgag gaagaacatc 2400
   aagggcatcc ataccctcct tcagaacttg gccaaggcat ctccggtcta cctggacatt 2460
   ctaggc 2466
<210> 11
   <211> 838
   <212> PRT
   <213> Homo sapiens
<400> 11
<210> 12
   <211> 2514
   <212> DNA
   <213> Homo sapiens
<400> 12
   atgtcgtcct ggataaggtg gcatggaccc gccatggcgc ggctctgggg cttctgctgg      60
   ctggttgtgg gcttctggag ggccgctttc gcctgtccca cgtcctgcaa atgcagtgcc     120
   tctcggatct ggtgcagcga cccttctcct ggcatcgtgg catttccgag attggagcct     180
   aacagtgtag atcctgagaa catcaccgaa attttcatcg caaaccagaa aaggttagaa     240
   atcatcaacg aagatgatgt tgaagcttat gtgggactga gaaatctgac aattgtggat     300
   tctggattaa aatttgtggc tcataaagca tttctgaaaa acagcaacct gcagcacatc     360
   aattttaccc gaaacaaact gacgagtttg tctaggaaac atttccgtca ccttgacttg     420
   tctgaactga tcctggtggg caatccattt acatgctcct gtgacattat gtggatcaag     480
   actctccaag aggctaaatc cagtccagac actcaggatt tgtactgcct gaatgaaagc     540
   agcaagaata ttcccctggc aaacctgcag atacccaatt gtggtttgcc atctgcaaat     600
   ctggccgcac ctaacctcac tgtggaggaa ggaaagtcta tcacattatc ctgtagtgtg     660
   gcaggtgatc cggttcctaa tatgtattgg gatgttggta acctggtttc caaacatatg     720
   aatgaaacaa gccacacaca gggctcctta aggataacta acatttcatc cgatgacagt     780
   gggaagcaga tctcttgtgt ggcggaaaat cttgtaggag aagatcaaga ttctgtcaac     840
   ctcactgtgc attttgcacc aactatcaca tttctcgaat ctccaacctc agaccaccac     900
   tggtgcattc cattcactgt gaaaggcaac cccaaaccag cgcttcagtg gttctataac     960
   ggggcaatat tgaatgagtc caaatacatc tgtactaaaa tacatgttac caatcacacg     1020
   gagtaccacg gctgcctcca gctggataat cccactcaca tgaacaatgg ggactacact     1080
   ctaatagcca agaatgagta tgggaaggat gagaaacaga tttctgctca cttcatgggc     1140
   tggcctggaa ttgacgatgg tgcaaaccca aattatcctg atgtaattta tgaagattat     1200
   ggaactgcag cgaatgacat cggggacacc acgaacagaa gtaatgaaat cccttccaca     1260
   gacgtcactg ataaaaccgg tcgggaacat ctctcggtct atgctgtggt ggtgattgcg     1320
   tctgtggtgg gattttgcct tttggtaatg ctgtttctgc ttaagttggc aagacactcc     1380
   aagtttggca tgaaagattt ctcatggttt ggatttggga aagtaaaatc aagacaaggt     1440
   gttggcccag cctccgttat cagcaatgat gatgactctg ccagcccact ccatcacatc     1500
   tccaatggga gtaacactcc atcttcttcg gaaggtggcc cagatgctgt cattattgga     1560
   atgaccaaga tccctgtcat tgaaaatccc cagtactttg gcatcaccaa cagtcagctc     1620
   aagccagaca catttgttca gcacatcaag cgacataaca ttgttctgaa aagggagcta     1680
   ggcgaaggag cctttggaaa agtgttccta gctgaatgct ataacctctg tcctgagcag     1740
   gacaagatct tggtggcagt gaagaccctg aaggatgcca gtgacaatgc acgcaaggac     1800
   ttccaccgtg aggccgagct cctgaccaac ctccagcatg agcacatcgt caagttctat     1860
   ggcgtctgcg tggagggcga ccccctcatc atggtctttg agtacatgaa gcatggggac     1920
   ctcaacaagt tcctcagggc acacggccct gatgccgtgc tgatggctga gggcaacccg     1980
   cccacggaac tgacgcagtc gcagatgctg catatagccc agcagatcgc cgcgggcatg     2040
   gtctacctgg cgtcccagca cttcgtgcac cgcgatttgg ccaccaggaa ctgcctggtc     2100
   ggggagaact tgctggtgaa aatcggggac tttgggatgt cccgggacgt gtacagcact     2160
   gactactaca gggtcggtgg ccacacaatg ctgcccattc gctggatgcc tccagagagc     2220
   atcatgtaca ggaaattcac gacggaaagc gacgtctgga gcctgggggt cgtgttgtgg     2280
   gagattttca cctatggcaa acagccctgg taccagctgt caaacaatga ggtgatagag     2340
   tgtatcactc agggccgagt cctgcagcga ccccgcacgt gcccccagga ggtgtatgag     2400
   ctgatgctgg ggtgctggca gcgagagccc cacatgagga agaacatcaa gggcatccat     2460
   accctccttc agaacttggc caaggcatct ccggtctacc tggacattct aggc           2514
<210> 13
   <211> 822
   <212> PRT
   <213> Homo sapiens
<400> 13
   Met Ser Ser Trp Ile Arg Trp His Gly Pro Ala Met Ala Arg Leu Trp
   1               5                   10                  15
   Gly Phe Cys Trp Leu Val Val Gly Phe Trp Arg Ala Ala Phe Ala Cys
               20                  25                  30
   Pro Thr Ser Cys Lys Cys Ser Ala Ser Arg Ile Trp Cys Ser Asp Pro
           35                  40                  45
   Ser Pro Gly Ile Val Ala Phe Pro Arg Leu Glu Pro Asn Ser Val Asp
       50                  55                  60
   Pro Glu Asn Ile Thr Glu Ile Phe Ile Ala Asn Gln Lys Arg Leu Glu
   65                  70                  75                  80
   Ile Ile Asn Glu Asp Asp Val Glu Ala Tyr Val Gly Leu Arg Asn Leu
                   85                  90                  95
   Thr Ile Val Asp Ser Gly Leu Lys Phe Val Ala His Lys Ala Phe Leu
               100                 105                 110
   Lys Asn Ser Asn Leu Gln His Ile Asn Phe Thr Arg Asn Lys Leu Thr
           115                 120                 125
   Ser Leu Ser Arg Lys His Phe Arg His Leu Asp Leu Ser Glu Leu Ile
       130                 135                 140
   Leu Val Gly Asn Pro Phe Thr Cys Ser Cys Asp Ile Met Trp Ile Lys
   145                 150                 155                 160
   Thr Leu Gln Glu Ala Lys Ser Ser Pro Asp Thr Gln Asp Leu Tyr Cys
                   165                 170                 175
   Leu Asn Glu Ser Ser Lys Asn Ile Pro Leu Ala Asn Leu Gln Ile Pro
               180                 185                 190
   Asn Cys Gly Leu Pro Ser Ala Asn Leu Ala Ala Pro Asn Leu Thr Val
           195                 200                 205
   Glu Glu Gly Lys Ser Ile Thr Leu Ser Cys Ser Val Ala Gly Asp Pro
       210                 215                 220
   Val Pro Asn Met Tyr Trp Asp Val Gly Asn Leu Val Ser Lys His Met
   225                 230                 235                 240
   Asn Glu Thr Ser His Thr Gln Gly Ser Leu Arg Ile Thr Asn Ile Ser
                   245                 250                 255
   Ser Asp Asp Ser Gly Lys Gln Ile Ser Cys Val Ala Glu Asn Leu Val
   260 265 270
   Gly Glu Asp Gln Asp Ser Val Asn Leu Thr Val His Phe Ala Pro Thr
   275 280 285
   Ile Thr Phe Leu Glu Ser Pro Thr Ser Asp His His Trp Cys Ile Pro
   290 295 300
   Phe Thr Val Lys Gly Asn Pro Lys Pro Ala Leu Gln Trp Phe Tyr Asn
   305 310 315 320
   Gly Ala Ile Leu Asn Glu Ser Lys Tyr Ile Cys Thr Lys Ile His Val
   325 330 335
   Thr Asn His Thr Glu Tyr His Gly Cys Leu Gln Leu Asp Asn Pro Thr
   340 345 350
   His Met Asn Asn Gly Asp Tyr Thr Leu Ile Ala Lys Asn Glu Tyr Gly
   355 360 365
   Lys Asp Glu Lys Gln Ile Ser Ala His Phe Met Gly Trp Pro Gly Ile
   370 375 380
   Asp Asp Gly Ala Asn Pro Asn Tyr Pro Asp Val Ile Tyr Glu Asp Tyr
   385 390 395 400
   Gly Thr Ala Ala Asn Asp Ile Gly Asp Thr Thr Asn Arg Ser Asn Glu
   405 410 415
   Ile Pro Ser Thr Asp Val Thr Asp Lys Thr Gly Arg Glu His Leu Ser
   420 425 430
   Val Tyr Ala Val Val Val Ile Ala Ser Val Val Gly Phe Cys Leu Leu
   435 440 445
   Val Met Leu Phe Leu Leu Lys Leu Ala Arg His Ser Lys Phe Gly Met
   450 455 460
   Lys Gly Pro Ala Ser Val Ile Ser Asn Asp Asp Asp Ser Ala Ser Pro
   465 470 475 480
   Leu His His Ile Ser Asn Gly Ser Asn Thr Pro Ser Ser Ser Glu Gly
   485 490 495
   Gly Pro Asp Ala Val Ile Ile Gly Met Thr Lys Ile Pro Val Ile Glu
   500 505 510
   Asn Pro Gln Glu Phe Gly Ile Thr Asn Ser Gln Leu Lys Pro Asp Thr
           515                 520                 525
   Phe Val Gln His Ile Lys Arg His Asn Ile Val Leu Lys Arg Glu Leu
       530                 535                 540
   Gly Glu Gly Ala Phe Gly Lys Val Phe Leu Ala Glu Cys Tyr Asn Leu
   545                 550                 555                 560
   Cys Pro Glu Gln Asp Lys Ile Leu Val Ala Val Lys Thr Leu Lys Asp
                   565                 570                 575
   Ala Ser Asp Asn Ala Arg Lys Asp Phe His Arg Glu Ala Glu Leu Leu
               580                 585                 590
   Thr Asn Leu Gln His Glu His Ile Val Lys Phe Tyr Gly Val Cys Val
           595                 600                 605
   Glu Gly Asp Pro Leu Ile Met Val Phe Glu Tyr Met Lys His Gly Asp
       610                 615                 620
   Leu Asn Lys Phe Leu Arg Ala His Gly Pro Asp Ala Val Leu Met Ala
   625                 630                 635                 640
   Glu Gly Asn Pro Pro Thr Glu Leu Thr Gln Ser Gln Met Leu His Ile
                   645                 650                 655
   Ala Gln Gln Ile Ala Ala Gly Met Val Tyr Leu Ala Ser Gln His Phe
               660                 665                 670
   Val His Arg Asp Leu Ala Thr Arg Asn Cys Leu Val Gly Glu Asn Leu
           675                 680                 685
   Leu Val Lys Ile Gly Asp Phe Gly Met Ser Arg Asp Val Glu Ser Thr
       690                 695                 700
   Asp Glu Glu Arg Val Gly Gly His Thr Met Leu Pro Ile Arg Trp Met
   705                 710                 715                 720
   Pro Pro Glu Ser Ile Met Tyr Arg Lys Phe Thr Thr Glu Ser Asp Val
                   725                 730                 735
   Trp Ser Leu Gly Val Val Leu Trp Glu Ile Phe Thr Tyr Gly Lys Gln
               740                 745                 750
   Pro Trp Tyr Gln Leu Ser Asn Asn Glu Val Ile Glu Cys Ile Thr Gln
           755                 760                 765
   Gly Arg Val Leu Gln Arg Pro Arg Thr Cys Pro Gln Glu Val Tyr Glu
       770                 775                 780
   Leu Met Leu Gly Cys Trp Gln Arg Glu Pro His Met Arg Lys Asn Ile
   785                 790                 795                 800
   Lys Gly Ile His Thr Leu Leu Gln Asn Leu Ala Lys Ala Ser Pro Val
                   805                 810                 815
   Glu Leu Asp Ile Leu Gly
               820
<210> 14
   <211> 2466
   <212> DNA
   <213> Homo sapiens
<400> 14
   atgtcgtcct ggataaggtg gcatggaccc gccatggcgc ggctctgggg cttctgctgg 60
   ctggttgtgg gcttctggag ggccgctttc gcctgtccca cgtcctgcaa atgcagtgcc 120
   tctcggatct ggtgcagcga cccttctcct ggcatcgtgg catttccgag attggagcct 180
   aacagtgtag atcctgagaa catcaccgaa attttcatcg caaaccagaa aaggttagaa 240
   atcatcaacg aagatgatgt tgaagcttat gtgggactga gaaatctgac aattgtggat 300
   tctggattaa aatttgtggc tcataaagca tttctgaaaa acagcaacct gcagcacatc 360
   aattttaccc gaaacaaact gacgagtttg tctaggaaac atttccgtca ccttgacttg 420
   tctgaactga tcctggtggg caatccattt acatgctcct gtgacattat gtggatcaag 480
   actctccaag aggctaaatc cagtccagac actcaggatt tgtactgcct gaatgaaagc 540
   agcaagaata ttcccctggc aaacctgcag atacccaatt gtggtttgcc atctgcaaat 600
   ctggccgcac ctaacctcac tgtggaggaa ggaaagtcta tcacattatc ctgtagtgtg 660
   gcaggtgatc cggttcctaa tatgtattgg gatgttggta acctggtttc caaacatatg 720
   aatgaaacaa gccacacaca gggctcctta aggataacta acatttcatc cgatgacagt 780
   gggaagcaga tctcttgtgt ggcggaaaat cttgtaggag aagatcaaga ttctgtcaac 840
   ctcactgtgc attttgcacc aactatcaca tttctcgaat ctccaacctc agaccaccac 900
   tggtgcattc cattcactgt gaaaggcaac cccaaaccag cgcttcagtg gttctataac 960
   ggggcaatat tgaatgagtc caaatacatc tgtactaaaa tacatgttac caatcacacg 1020
   gagtaccacg gctgcctcca gctggataat cccactcaca tgaacaatgg ggactacact 1080
   ctaatagcca agaatgagta tgggaaggat gagaaacaga tttctgctca cttcatgggc 1140
   tggcctggaa ttgacgatgg tgcaaaccca aattatcctg atgtaattta tgaagattat 1200
   ggaactgcag cgaatgacat cggggacacc acgaacagaa gtaatgaaat cccttccaca 1260
   gacgtcactg ataaaaccgg tcgggaacat ctctcggtct atgctgtggt ggtgattgcg 1320
   tctgtggtgg gattttgcct tttggtaatg ctgtttctgc ttaagttggc aagacactcc 1380
   aagtttggca tgaaaggccc agcctccgtt atcagcaatg atgatgactc tgccagccca 1440
   ctccatcaca tctccaatgg gagtaacact ccatcttctt cggaaggtgg cccagatgct 1500
   gtcattattg gaatgaccaa gatccctgtc attgaaaatc cccaggaatt tggcatcacc 1560
   aacagtcagc tcaagccaga cacatttgtt cagcacatca agcgacataa cattgttctg 1620
   aaaagggagc taggcgaagg agcctttgga aaagtgttcc tagctgaatg ctataacctc 1680
   tgtcctgagc aggacaagat cttggtggca gtgaagaccc tgaaggatgc cagtgacaat 1740
   gcacgcaagg acttccaccg tgaggccgag ctcctgacca acctccagca tgagcacatc 1800
   gtcaagttct atggcgtctg cgtggagggc gaccccctca tcatggtctt tgagtacatg 1860
   aagcatgggg acctcaacaa gttcctcagg gcacacggcc ctgatgccgt gctgatggct 1920
   gagggcaacc cgcccacgga actgacgcag tcgcagatgc tgcatatagc ccagcagatc 1980
   gccgcgggca tggtctacct ggcgtcccag cacttcgtgc accgcgattt ggccaccagg 2040
   aactgcctgg tcggggagaa cttgctggtg aaaatcgggg actttgggat gtcccgggac 2100
   gtggaaagca ctgacgaaga aagggtcggt ggccacacaa tgctgcccat tcgctggatg 2160
   cctccagaga gcatcatgta caggaaattc acgacggaaa gcgacgtctg gagcctgggg 2220
   gtcgtgttgt gggagatttt cacctatggc aaacagccct ggtaccagct gtcaaacaat 2280
   gaggtgatag agtgtatcac tcagggccga gtcctgcagc gaccccgcac gtgcccccag 2340
   gaggtgtatg agctgatgct ggggtgctgg cagcgagagc cccacatgag gaagaacatc 2400
   aagggcatcc ataccctcct tcagaacttg gccaaggcat ctccggtcga actggacatt 2460
   ctaggc 2466
<210> 15
   <211> 229
   <212> PRT
   <213> Homo sapiens
<400> 15
   Ala Pro Met Lys Glu Ala Asn Ile Arg Gly Gln Gly Gly Leu Ala Tyr
   1               5                   10                  15
   Pro Gly Val Arg Thr His Gly Thr Leu Glu Ser Val Asn Gly Pro Lys
               20                  25                  30
   Ala Gly Ser Arg Gly Leu Thr Ser Leu Ala Asp Thr Phe Glu His Val
           35                  40                  45
   Ile Glu Glu Leu Leu Asp Glu Asp Gln Lys Val Arg Pro Asn Glu Glu
       50                  55                  60
   Asn Asn Lys Asp Ala Asp Leu Tyr Thr Ser Arg Val Met Leu Ser Ser
   65                  70                  75                  80
   Gln Val Pro Leu Glu Pro Pro Leu Leu Phe Leu Leu Glu Glu Tyr Lys
                   85                  90                  95
   Asn Tyr Leu Asp Ala Ala Asn Met Ser Met Arg Val Arg Arg His Ser
               100                 105                 110
   Asp Pro Ala Arg Arg Gly Glu Leu Ser Val Cys Asp Ser Ile Ser Glu
           115                 120                 125
   Trp Val Thr Ala Ala Asp Lys Lys Thr Ala Val Asp Met Ser Gly Gly
       130                 135                 140
   Thr Val Thr Val Leu Glu Lys Val Pro Val Ser Lys Gly Gln Leu Lys
   145                 150                 155                 160
   Gln Tyr Phe Tyr Glu Thr Lys Cys Asn Pro Met Gly Tyr Thr Lys Glu
                   165                 170                 175
   Gly Cys Arg Gly Ile Asp Lys Arg His Trp Asn Ser Gln Cys Arg Thr
               180                 185                 190
   Thr Gln Ser Tyr Val Arg Ala Leu Thr Met Asp Ser Lys Lys Arg Ile
           195                 200                 205
   Gly Trp Arg Phe Ile Arg Ile Asp Thr Ser Cys Val Cys Thr Leu Thr
       210                 215                 220
   Ile Lys Arg Gly Arg
   225
<210> 16
   <211> 690
   <212> DNA
   <213> Homo sapiens
<400> 16
   gcccccatga aagaagcaaa catccgagga caaggtggct tggcctaccc aggtgtgcgg 60
   acccatggga ctctggagag cgtgaatggg cccaaggcag gttcaagagg cttgacatca 120
   ttggctgaca ctttcgaaca cgtgatagaa gagctgttgg atgaggacca gaaagttcgg 180
   cccaatgaag aaaacaataa ggacgcagac ttgtacacgt ccagggtgat gctcagtagt 240
   caagtgcctt tggagcctcc tcttctcttt ctgctggagg aatacaaaaa ttacctagat 300
   gctgcaaaca tgtccatgag ggtccggcgc cactctgacc ctgcccgccg aggggagctg 360
   agcgtgtgtg acagtattag tgagtgggta acggcggcag acaaaaagac tgcagtggac 420
   atgtcgggcg ggacggtcac agtccttgaa aaggtccctg tatcaaaagg ccaactgaag 480
   caatacttct acgagaccaa gtgcaatccc atgggttaca caaaagaagg ctgcaggggc 540
   atagacaaaa ggcattggaa ctcccagtgc cgaactaccc agtcgtacgt gcgggccctt 600
   accatggata gcaaaaagag aattggctgg cgattcataa ggatagacac ttcttgtgta 660
   tgtacattga ccattaaaag gggaagatag 690
<210> 17
   <211> 229
   <212> PRT
   <213> Homo sapiens
<400> 17
   Ala Pro Met Lys Glu Ala Asn Ile Arg Gly Gln Gly Gly Leu Ala Tyr
   1               5                   10                  15
   Pro Gly Val Arg Thr His Gly Thr Leu Glu Ser Val Asn Gly Pro Lys
               20                  25                  30
   Ala Gly Ser Arg Gly Leu Thr Ser Leu Ala Asp Thr Phe Glu His Met
           35                  40                  45
   Ile Glu Glu Leu Leu Asp Glu Asp Gln Lys Val Arg Pro Asn Glu Glu
       50                  55                  60
   Asn Asn Lys Asp Ala Asp Leu Tyr Thr Ser Arg Val Met Leu Ser Ser
   65                  70                  75                  80
   Gln Val Pro Leu Glu Pro Pro Leu Leu Phe Leu Leu Glu Glu Tyr Lys
                   85                  90                  95
   Asn Tyr Leu Asp Ala Ala Asn Met Ser Met Arg Val Arg Arg His Ser
               100                 105                 110
   Asp Pro Ala Arg Arg Gly Glu Leu Ser Val Cys Asp Ser Ile Ser Glu
           115                 120                 125
   Trp Val Thr Ala Ala Asp Lys Lys Thr Ala Val Asp Met Ser Gly Gly
       130                 135                 140
   Thr Val Thr Val Leu Glu Lys Val Pro Val Ser Lys Gly Gln Leu Lys
   145                 150                 155                 160
   Gln Tyr Phe Tyr Glu Thr Lys Cys Asn Pro Met Gly Tyr Thr Lys Glu
                   165                 170                 175
   Gly Cys Arg Gly Ile Asp Lys Arg His Trp Asn Ser Gln Cys Arg Thr
               180                 185                 190
   Thr Gln Ser Tyr Val Arg Ala Leu Thr Met Asp Ser Lys Lys Arg Ile
           195                 200                 205
   Gly Trp Arg Phe Ile Arg Ile Asp Thr Ser Cys Val Cys Thr Leu Thr
       210                 215                 220
   Ile Lys Arg Gly Arg
   225
<210> 18
   <211> 119
   <212> PRT
   <213> Homo sapiens
<400> 18
   His Ser Asp Pro Ala Arg Arg Gly Glu Leu Ser Val Cys Asp Ser Ile
   1               5                   10                  15
   Ser Glu Trp Val Thr Ala Ala Asp Lys Lys Thr Ala Val Asp Met Ser
               20                  25                  30
   Gly Gly Thr Val Thr Val Leu Glu Lys Val Pro Val Ser Lys Gly Gln
           35                  40                  45
   Leu Lys Gln Tyr Phe Tyr Glu Thr Lys Cys Asn Pro Met Gly Tyr Thr
       50                  55                  60
   Lys Glu Gly Cys Arg Gly Ile Asp Lys Arg His Trp Asn Ser Gln Cys
   65                  70                  75                  80
   Arg Thr Thr Gln Ser Tyr Val Arg Ala Leu Thr Met Asp Ser Lys Lys
                   85                  90                  95
   Arg Ile Gly Trp Arg Phe Ile Arg Ile Asp Thr Ser Cys Val Cys Thr
               100                 105                 110
   Leu Thr Ile Lys Arg Gly Arg
           115
<210> 19
   <211> 744
   <212> DNA
   <213> Homo sapiens
<400> 19
   atgaccatcc ttttccttac tatggttatt tcatactttg gttgcatgaa ggctgccccc        60
   atgaaagaag caaacatccg aggacaaggt ggcttggcct acccaggtgt gcggacccat       120
   gggactctgg agagcgtgaa tgggcccaag gcaggttcaa gaggcttgac atcattggct       180
   gacactttcg aacacgtgat agaagagctg ttggatgagg accagaaagt tcggcccaat       240
   gaagaaaaca ataaggacgc agacttgtac acgtccaggg tgatgctcag tagtcaagtg       300
   cctttggagc ctcctcttct ctttctgctg gaggaataca aaaattacct agatgctgca       360
   aacatgtcca tgagggtccg gcgccactct gaccctgccc gccgagggga gctgagcgtg       420
   tgtgacagta ttagtgagtg ggtaacggcg gcagacaaaa agactgcagt ggacatgtcg       480
   ggcgggacgg tcacagtcct tgaaaaggtc cctgtatcaa aaggccaact gaagcaatac       540
   ttctacgaga ccaagtgcaa tcccatgggt tacacaaaag aaggctgcag gggcatagac        600
   aaaaggcatt ggaactccca gtgccgaact acccagtcgt acgtgcgggc ccttaccatg       660
   gatagcaaaa agagaattgg ctggcgattc ataaggatag acacttcttg tgtatgtaca       720
   ttgaccatta aaaggggaag atag                                              744
<210> 20
   <211> 18
   <212> PRT
   <213> Homo sapiens
<400> 20
   Met Thr Ile Leu Phe Leu Thr Met Val Ile Ser Tyr Phe Gly Cys Met
   1               5                   10                  15
   Lys Ala
<210> 21
   <211> 54
   <212> DNA
   <213> Homo sapiens
<400> 21
   atgaccatcc ttttccttac tatggttatt tcatacttcg gttgcatgaa ggcg 54
<210> 22
   <211> 26
   <212> PRT
   <213> Homo sapiens
<400> 22
   Met Phe His Gln Val Arg Arg Val Met Thr Ile Leu Phe Leu Thr Met
   1               5                   10                  15
   Val Ile Ser Tyr Phe Gly Cys Met Lys Ala
               20                  25
<210> 23
   <211> 78
   <212> DNA
   <213> Homo sapiens
<400> 23
   atgttccacc aggtgagaag agtgatgacc atccttttcc ttactatggt tatttcatac 60
   ttcggttgca tgaaggcg 78
<210> 24
   <211> 33
   <212> PRT
   <213> Homo sapiens
<400> 24
   Met Gln Ser Arg Glu Glu Glu Trp Phe His Gln Val Arg Arg Val Met
   1 5 10 15
   Thr Ile Leu Phe Leu Thr Met Val Ile Ser Tyr Phe Gly Cys Met Lys
   20 25 30
   Ala
<210> 25
   <211> 99
   <212> DNA
   <213> Homo sapiens
<400> 25
   atgcagagcc gggaagagga atggttccac caggtgagaa gagtgatgac catccttttc 60
   cttactatgg ttatttcata cttcggttgc atgaaggcg 99
<210> 26
   <211> 47
   <212> PRT
   <213> Homo sapiens
<400> 26
   Met Leu Cys Ala Ile Ser Leu Cys Ala Arg Val Arg Lys Leu Arg Ser
   1 5 10 15
   Ala Gly Arg Cys Gly Lys Phe His Gln Val Arg Arg Val Met Thr Ile
   20 25 30
   Leu Phe Leu Thr Met Val Ile Ser Tyr Phe Gly Cys Met Lys Ala
   35 40 45
<210> 27
   <211> 141
   <212> DNA
   <213> Homo sapiens
<400> 27
   atgctctgtg cgatttcatt gtgtgctcgc gttcgcaagc tccgtagtgc aggaaggtgc 60
   gggaagttcc accaggtgag aagagtgatg accatccttt tccttactat ggttatttca 120
   tacttcggtt gcatgaaggc g 141
<210> 28
   <211> 100
   <212> PRT
   <213> Homo sapiens
<400> 28 Cys Met Lys Ala 100
<210> 29
   <211> 300
   <212> DNA
   <213> Homo sapiens
<400> 29
   atgtgtggag ccaccagttt tctccatgag tgcacaaggt taatccttgt tactactcag      60
   aatgctgagt ttctacagaa agggttgcag gtccacacat gttttggcgt ctacccacac     120
   gcttctgtat ggcatgactg tgcatcccag aagaagggct gtgctgtgta cctccacgtt     180
   tcagtggaat ttaacaaact gatccctgaa aatggtttca taaagttcca ccaggtgaga     240
   agagtgatga ccatcctttt ccttactatg gttatttcat acttcggttg catgaaggcg     300
<210> 30
   <211> 20
   <212> PRT
   <213> Homo sapiens
<400> 30
   Met Tyr Arg Met Gln Leu Leu Ser Cys Ile Ala Leu Ser Leu Ala Leu
   1               5                   10                  15
   Val Thr Asn Ser
               20
<210> 31
   <211> 60
   <212> DNA
   <213> Homo sapiens
<400> 31
   atgtacagga tgcaactcct gtcttgcatt gcactaagtc ttgcacttgt cacaaacagt 60
<210> 32
   <211> 22
   <212> PRT
   <213> Homo sapiens
<400> 32
   Met Lys Arg Arg Val Met Ile Ile Leu Phe Leu Thr Met Val Ile Ser
   1               5                   10                  15
   Tyr Phe Gly Cys Met Lys
               20
<210> 33
   <211> 70
   <212> DNA
   <213> Homo sapiens
<400> 33
   atgaaaagaa gagtgatgat catccttttc cttactatgg ttatttcata cttcggttgc 60
   atgaagagcg 70
<210> 34
   <211> 20
   <212> PRT
   <213> Homo sapiens
<400> 34 Val Thr Asn Ser 20
<210> 35
   <211> 60
   <212> DNA
   <213> Homo sapiens
<400> 35
   atgaggagga tgcaactcct gctcctgatt gcactaagtc ttgcacttgt cacaaacagt 60
<210> 36
   <211> 21
   <212> PRT
   <213> Homo sapiens
<400> 36
   Met Arg Arg Met Gln Leu Leu Leu Leu Thr Met Val Ile Ser Tyr Phe
   1 5 10 15
   Gly Cys Met Lys Ala
   20
<210> 37
   <211> 63
   <212> DNA
   <213> Homo sapiens
<400> 37
   atgaggagga tgcaactcct gctcctgact atggttattt catacttcgg ttgcatgaag 60
   gcg 63
<210> 38
   <211> 18
   <212> PRT
   <213> Homo sapiens
<400> 38
   Met Arg Ile Leu Leu Leu Thr Met Val Ile Ser Tyr Phe Gly Cys Met
   1               5                   10                  15
   Lys Ala
<210> 39
   <211> 54
   <212> PRT
   <213> Homo sapiens
<400> 39
   Ala Thr Gly Ala Gly Ala Ala Thr Cys Cys Thr Thr Cys Thr Thr Cys
   1               5                   10                  15
   Thr Thr Ala Cys Thr Ala Thr Gly Gly Thr Thr Ala Thr Thr Thr Cys
               20                  25                  30
   Ala Thr Ala Cys Thr Thr Cys Gly Gly Thr Thr Gly Cys Ala Thr Gly
           35                  40                  45
   Ala Ala Gly Gly Cys Gly
       50
<210> 40
   <211> 19
   <212> PRT
   <213> Homo sapiens
<400> 40 Met Lys Ala
<210> 41
   <211> 57
   <212> DNA
   <213> Homo sapiens
<400> 41
   atgagaagaa tccttttcct tactatggtt atttcatact tcggttgcat gaaggcg 57
<210> 42
   <211> 18
   <212> PRT
   <213> Homo sapiens
<400> 42
   Met Arg Arg Phe Leu Phe Leu Leu Val Ile Ser Tyr Phe Gly Cys Met
   1               5                   10                  15
   Lys Ala
<210> 43
   <211> 54
   <212> DNA
   <213> Homo sapiens
<400> 43
   atgaggaggt tccttttcct tcttgttatt tcatacttcg gttgcatgaa ggcg 54
<210> 44
   <211> 15
   <212> PRT
   <213> Homo sapiens
<400> 44
   Met Arg Arg Phe Leu Phe Leu Leu Tyr Phe Gly Cys Met Lys Ala
   1               5                   10                  15
<210> 45
   <211> 45
   <212> DNA
   <213> Homo sapiens
<400> 45
   atgaggaggt tccttttcct tctttacttc ggttgcatga aggcg 45
<210> 46
   <211> 130
   <212> DNA
   <213> Homo sapiens
<400> 46
   cctgcaggca gctgcgcgct cgctcgctca ctgaggccgc ccgggcgtcg ggcgaccttt 60
   ggtcgcccgg cctcagtgag cgagcgagcg cgcagagagg gagtggccaa ctccatcact 120
   aggggttcct 130
<210> 47
   <211> 141
   <212> DNA
   <213> Homo sapiens
<400> 47
   aggaacccct agtgatggag ttggccactc cctctctgcg cgctcgctcg ctcactgagg        60
   ccgggcgacc aaaggtcgcc cgacgcccgg gctttgcccg ggcggcctca gtgagcgagc       120
   gagcgcgcag ctgcctgcag g                                                 141
<210> 48
   <211> 584
   <212> DNA
   <213> Homo sapiens
<400> 48
   gcgttacata acttacggta aatggcccgc ctggctgacc gcccaacgac ccccgcccat        60
   tgacgtcaat aatgacgtat gttcccatag taacgccaat agggactttc cattgacgtc       120
   aatgggtgga ctatttacgg taaactgccc acttggcagt acatcaagtg tatcatatgc       180
   caagtacgcc ccctattgac gtcaatgacg gtaaatggcc cgcctggcat tatgcccagt       240
   acatgacctt atgggacttt cctacttggc agtacatcta cgtattagtc atcgctatta       300
   ccatggtcga ggtgagcccc acgttctgct tcactctccc catctccccc ccctccccac       360
   ccccaatttt gtatttattt attttttaat tattttgtgc agcgatgggg gcgggggggg       420
   ggggggggcg cgcgccaggc ggggcggggc ggggcgaggg gcggggcggg gcgaggcgga       480
   gaggtgcggc ggcagccaat cagagcggcg cgctccgaaa gtttcctttt atggcgaggc       540
   ggcggcggcg gcggccctat aaaaagcgaa gcgcgcggcg ggcg                       584
<210> 49
   <211> 210
   <212> PRT
   <213> Homo sapiens
<400> 49
<210> 50
   <211> 630
   <212> DNA
   <213> Homo sapiens
<400> 50
   atgctccctc tcccctcatg ctccctcccc atcctcctcc ttttcctcct ccccagtgtg        60
   ccaattgagt cccaaccccc accctcaaca ttgccccctt ttctggcccc tgagtgggac       120
   cttctctccc cccgagtagt cctgtctagg ggtgcccctg ctgggccccc tctgctcttc       180
   ctgctggagg ctggggcctt tcgggagtca gcaggtgccc cggccaaccg cagccggcgt       240
   ggggtgagcg aaactgcacc agcgagtcgt cggggtgagc tggctgtgtg cgatgcagtc       300
   agtggctggg tgacagaccg ccggaccgct gtggacttgc gtgggcgcga ggtggaggtg       360
   ttgggcgagg tgcctgcagc tggcggcagt cccctccgcc agtacttctt tgaaacccgc       420
   tgcaaggctg ataacgctga ggaaggtggc ccgggggcag gtggaggggg ctgccgggga       480
   gtggacagga ggcactgggt atctgagtgc aaggccaagc agtcctatgt gcgggcattg       540
   accgctgatg cccagggccg tgtgggctgg cgatggattc gaattgacac tgcctgcgtc       600
   tgcacactcc tcagccggac tggccgggcc                                        630
<210> 51
   <211> 24
   <212> PRT
   <213> Homo sapiens
<400> 51
   Met Leu Pro Leu Pro Ser Cys Ser Leu Pro Ile Leu Leu Leu Phe Leu
   1               5                   10                  15
   Leu Pro Ser Val Pro Ile Glu Ser
               20
<210> 52
   <211> 72
   <212> DNA
   <213> Homo sapiens
<400> 52
   atgctccctc tcccctcatg ctccctcccc atcctcctcc ttttcctcct ccccagtgtg        60
   ccaattgagt cc                                                            72
<210> 53
   <211> 56
   <212> PRT
   <213> Homo sapiens
<400> 53
   Gln Pro Pro Pro Ser Thr Leu Pro Pro Phe Leu Ala Pro Glu Trp Asp
   1               5                   10                  15
   Leu Leu Ser Pro Arg Val Val Leu Ser Arg Gly Ala Pro Ala Gly Pro
               20                  25                  30
   Pro Leu Leu Phe Leu Leu Glu Ala Gly Ala Phe Arg Glu Ser Ala Gly
           35                  40                  45
   Ala Pro Ala Asn Arg Ser Arg Arg
       50                  55
<210> 54
   <211> 168
   <212> DNA
   <213> Homo sapiens
<400> 54
   caacccccac cctcaacatt gccccctttt ctggcccctg agtgggacct tctctccccc 60
   cgagtagtcc tgtctagggg tgcccctgct gggccccctc tgctcttcct gctggaggct 120
   ggggcctttc gggagtcagc aggtgccccg gccaaccgca gccggcgt 168
<210> 55
   <211> 130
   <212> PRT
   <213> Homo sapiens
<400> 55
   Gly Val Ser Glu Thr Ala Pro Ala Ser Arg Arg Gly Glu Leu Ala Val
   1               5                   10                  15
   Cys Asp Ala Val Ser Gly Trp Val Thr Asp Arg Arg Thr Ala Val Asp
               20                  25                  30
   Leu Arg Gly Arg Glu Val Glu Val Leu Gly Glu Val Pro Ala Ala Gly
           35                  40                  45
   Gly Ser Pro Leu Arg Gln Tyr Phe Phe Glu Thr Arg Cys Lys Ala Asp
       50                  55                  60
   Asn Ala Glu Glu Gly Gly Pro Gly Ala Gly Gly Gly Gly Cys Arg Gly
   65                  70                  75                  80
   Val Asp Arg Arg His Trp Val Ser Glu Cys Lys Ala Lys Gln Ser Tyr
                   85                  90                  95
   Val Arg Ala Leu Thr Ala Asp Ala Gln Gly Arg Val Gly Trp Arg Trp
               100                 105                 110
   Ile Arg Ile Asp Thr Ala Cys Val Cys Thr Leu Leu Ser Arg Thr Gly
           115                 120                 125
   Arg Ala
       130
<210> 56
   <211> 390
   <212> DNA
   <213> Homo sapiens
<400> 56
   ggggtgagcg aaactgcacc agcgagtcgt cggggtgagc tggctgtgtg cgatgcagtc      60
   agtggctggg tgacagaccg ccggaccgct gtggacttgc gtgggcgcga ggtggaggtg     120
   ttgggcgagg tgcctgcagc tggcggcagt cccctccgcc agtacttctt tgaaacccgc     180
   tgcaaggctg ataacgctga ggaaggtggc ccgggggcag gtggaggggg ctgccgggga     240
   gtggacagga ggcactgggt atctgagtgc aaggccaagc agtcctatgt gcgggcattg     300
   accgctgatg cccagggccg tgtgggctgg cgatggattc gaattgacac tgcctgcgtc     360
   tgcacactcc tcagccggac tggccgggcc                                      390
<210> 57
   <211> 592
   <212> DNA
   <213> Homo sapiens
<400> 57
   aatcaacctc tggattacaa aatttgtgaa agattgactg gtattcttaa ctatgttgct 60
   ccttttacgc tatgtggata cgctgcttta atgcctttgt atcatgctat tgcttcccgt 120
   atggctttca ttttctcctc cttgtataaa tcctggttgc tgtctcttta tgaggagttg 180
   tggcccgttg tcaggcaacg tggcgtggtg tgcactgtgt ttgctgacgc aacccccact 240
   ggttggggca ttgccaccac ctgtcagctc ctttccggga ctttcgcttt ccccctccct 300
   attgccacgg cggaactcat cgccgcctgc cttgcccgct gctggacagg ggctcggctg 360
   ttgggcactg acaattccgt ggtgttgtcg gggaagctga cgtcctttcc atggctgctc 420
   gcctgtgttg ccacctggat tctgcgcggg acgtccttct gctacgtccc ttcggccctc 480
   aatccagcgg accttccttc ccgcggcctg ctgccggctc tgcggcctct tccgcgtctt 540
   cgccttcgcc ctcagacgag tcggatctcc ctttgggccg cctccccgcc tg 592
<210> 58
   <211> 247
   <212> DNA
   <213> Homo sapiens
<400> 58
   aatcaacctc tggattacaa aatttgtgaa agattgactg gtattcttaa ctatgttgct 60
   ccttttacgc tatgtggata cgctgcttta atgcctttgt atcatgctat tgcttcccgt 120
   atggctttca ttttctcctc cttgtataaa tcctggttag ttcttgccac ggcggaactc 180
   atcgccgcct gccttgcccg ctgctggaca ggggctcggc tgttgggcac tgacaattcc 240
   gtggtgt 247
<210> 59
   <211> 224
   <212> DNA
   <213> Homo sapiens
<400> 59
   agcagacatg ataagataca ttgatgagtt tggacaaacc acaactagaa tgcagtgaaa 60
   aaaatgcttt atttgtgaaa tttgtgatgc tattgcttta tttgtaacca ttataagctg 120
   caataaacaa gttaacaaca acaattgcat tcattttatg tttcaggttc agggggaggt 180
   gtgggaggtt ttttaaagca agtaaaacct ctacaaatgt ggta 224
<210> 60
   <211> 18
   <212> PRT
   <213> Homo sapiens
<400> 60
   Met Thr Ile Leu Phe Leu Thr Met Val Ile Ser Tyr Phe Gly Cys Met
   1               5                   10                  15
   Lys Ala
<210> 61
   <211> 54
   <212> DNA
   <213> Homo sapiens
<400> 61
   atgaccatcc ttttccttac tatggttatt tcatacttcg gttgcatgaa ggcg 54
<210> 62
   <211> 2
   <212> PRT
   <213> Homo sapiens
<400> 62
   Met Arg
   1
<210> 63
   <211> 6
   <212> DNA
   <213> Homo sapiens
<400> 63
   atgaga 6
<210> 64
   <211> 3
   <212> PRT
   <213> Homo sapiens
<400> 64
   Met Arg Arg
   1
<210> 65
   <211> 9
   <212> DNA
   <213> Homo sapiens
<400> 65
   atgagaaga 9
<210> 66
   <211> 4
   <212> PRT
   <213> Homo sapiens
<400> 66
   Met Arg Arg Arg
   1
<210> 67
   <211> 12
   <212> DNA
   <213> Homo sapiens
<400> 67
   atgagaagaa ga 12
<210> 68
   <211> 2
   <212> PRT
   <213> Homo sapiens
<400> 68
   Met Lys
   1
<210> 69
   <211> 6
   <212> DNA
   <213> Homo sapiens
<400> 69
   atgaaa 6
<210> 70
   <211> 3
   <212> PRT
   <213> Homo sapiens
<400> 70
   Met Lys Lys
   1
<210> 71
   <211> 9
   <212> DNA
   <213> Homo sapiens
<400> 71
   atgaaaaka 9
<210> 72
   <211> 4
   <212> PRT
   <213> Homo sapiens
<400> 72
   Met Lys Lys Lys
   1
<210> 73
   <211> 12
   <212> DNA
   <213> Homo sapiens
<400> 73
   atgaaaaaaa aa 12
<210> 74
   <211> 4
   <212> PRT
   <213> Homo sapiens
<400> 74
   Met Lys Arg Arg
   1
<210> 75
   <211> 12
   <212> DNA
   <213> Homo sapiens
<400> 75
   atgaaaagaa ga 12
<210> 76
   <211> 4
   <212> PRT
   <213> Homo sapiens
<400> 76
   Met Arg Lys Arg
   1
<210> 77
   <211> 12
   <212> DNA
   <213> Homo sapiens
<400> 77
   atgagaaaaa ga 12
<210> 78
   <211> 4
   <212> PRT
   <213> Homo sapiens
<400> 78
   Met Arg Arg Lys
   1
<210> 79
   <211> 12
   <212> DNA
   <213> Homo sapiens
<400> 79
   atgagaagaa aa 12
<210> 80
   <211> 4
   <212> PRT
   <213> Homo sapiens
<400> 80
   Met Lys Lys Arg
   1
<210> 81
   <211> 12
   <212> DNA
   <213> Homo sapiens
<400> 81
   atgaaaaaaa ga 12
<210> 82
   <211> 4
   <212> PRT
   <213> Homo sapiens
<400> 82
   Phe Leu Phe Leu
   1
<210> 83
   <211> 12
   <212> DNA
   <213> Homo sapiens
<400> 83
   ttccttttcc tt 12
<210> 84
   <211> 4
   <212> PRT
   <213> Homo sapiens
<400> 84
   Phe Phe Phe Leu
   1
<210> 85
   <211> 12
   <212> DNA
   <213> Homo sapiens
<400> 85
   ttcttcttcc tt 12
<210> 86
   <211> 4
   <212> PRT
   <213> Homo sapiens
<400> 86
   Phe Ile Phe Leu
   1
<210> 87
   <211> 12
   <212> DNA
   <213> Homo sapiens
<400> 87
   ttcatcttcc tt 12
<210> 88
   <211> 4
   <212> PRT
   <213> Homo sapiens
<400> 88
   Phe Ile Phe Ile
   1
<210> 89
   <211> 12
   <212> DNA
   <213> Homo sapiens
<400> 89
   ttcatcttca tc 12
<210> 90
   <211> 4
   <212> PRT
   <213> Homo sapiens
<400> 90
   Phe Val Phe Ile
   1
<210> 91
   <211> 12
   <212> DNA
   <213> Homo sapiens
<400> 91
   ttcgttttca tc 12
<210> 92
   <211> 4
   <212> PRT
   <213> Homo sapiens
<400> 92
   Phe Val Phe Val
   1
<210> 93
   <211> 12
   <212> DNA
   <213> Homo sapiens
<400> 93
   ttcgttttcg tt 12
<210> 94
   <211> 4
   <212> PRT
   <213> Homo sapiens
<400> 94
   Phe Leu Phe Val
   1
<210> 95
   <211> 12
   <212> DNA
   <213> Homo sapiens
<400> 95
   ttccttttcg tt 12
<210> 96
   <211> 4
   <212> PRT
   <213> Homo sapiens
<400> 96
   Phe Ile Phe Val
   1
<210> 97
   <211> 12
   <212> DNA
   <213> Homo sapiens
<400> 97
   ttcatcttcg tt 12
<210> 98
   <211> 4
   <212> PRT
   <213> Homo sapiens
<400> 98
   Phe Phe Phe Ile
   1
<210> 99
   <211> 12
   <212> DNA
   <213> Homo sapiens
<400> 99
   ttcttcttca tc 12
<210> 100
   <211> 4
   <212> PRT
   <213> Homo sapiens
<400> 100
   Phe Phe Phe Val
   1
<210> 101
   <211> 12
   <212> DNA
   <213> Homo sapiens
<400> 101
   ttcttcttcg tt 12
<210> 102
   <211> 5
   <212> PRT
   <213> Homo sapiens
<400> 102
   Phe Ile Leu Phe Leu
   1 5
<210> 103
   <211> 15
   <212> DNA
   <213> Homo sapiens
<400> 103
   ttcatccttt tcctt 15
<210> 104
   <211> 1203
   <212> DNA
   <213> Homo sapiens
<400> 104
   atgactatcc tgtttctgac aatggttatt agctatttcg gttgcatgaa ggctcacagt 60
   gatcccgcac gccgcggaga acttagcgtg tgcgacagca tcagcgagtg ggtcaccgcc 120
   gccgataaga agaccgctgt ggatatgtcc ggcgggaccg tcactgtact cgaaaaagtt 180
   ccagtgagca aaggccaact gaaacaatat ttctatgaaa ctaagtgcaa ccccatgggg 240
   tacaccaagg agggctgccg gggaatcgac aagagacact ggaattccca gtgccggacc 300
   actcagagct acgtccgcgc cttgacgatg gattcaaaga agcgcatcgg atggcggttc 360
   ataagaatcg acaccagttg tgtgtgcacg ctgacgataa aacgggggcg ggcccccgtg 420
   aagcagaccc tgaactttga tttgctcaag ttggcggggg atgtggaaag caatcccggg 480
   ccaatggtga gcaagggcga ggagctgttc accggcgttg tgccaatact ggttgagttg 540
   gatggcgatg tcaacggaca caaatttagc gtaagcgggg agggagaggg cgacgccaca 600
   tatggcaagc tgaccctgaa gttcatttgc acgaccggca aattgcccgt cccttggccc 660
   acacttgtga cgaccctgac ttatggcgta cagtgcttca gcaggtaccc tgatcatatg 720
   aagcaacacg acttctttaa gagtgccatg ccagagggat acgtccagga aagaaccata 780
   ttcttcaaag atgatggaaa ttacaaaacc cgggcagagg tcaagtttga aggcgacacc 840
   ctggtgaaca ggatcgaact caaaggcatc gatttcaaag aggacggaaa catcctcgga 900
   cacaaactgg aatacaatta caacagccac aacgtctaca tcatggcaga taaacaaaag 960
   aacggtatta aagtgaactt caagatccgg cacaacatcg aagacggctc cgtccagctt 1020
   gccgaccact accagcaaaa taccccgatc ggcgacggcc ccgttctcct ccccgataat 1080
   cactacctga gtacacagtc agccttgagc aaagacccta atgaaaagcg ggaccacatg 1140
   gttttgctgg agttcgttac cgcagcgggt attacgctgg gtatggacga gctttacaag 1200
   taa 1203
<210> 105
   <211> 1203
   <212> DNA
   <213> Homo sapiens
<400> 105
   atgactatcc tgtttctgac aatggttatt agctatttcg gttgcatgaa ggctcacagt        60
   gatcccgcac gccgcggaga acttagcgtg tgcgacagca tcagcgagtg ggtcaccgcc       120
   gccgataaga agaccgctgt ggatatgtcc ggcgggaccg tcactgtact cgaaaaagtt       180
   ccagtgagca aaggccaact gaaacaatat ttctatgaaa ctaagtgcaa ccccatgggg       240
   tacaccaagg agggctgccg gggaatcgac aagagacact ggaattccca gtgccggacc       300
   actcagagct acgtccgcgc cttgacgatg gattcaaaga agcgcatcgg atggcggttc       360
   ataagaatcg acaccagttg tgtgtgcacg ctgacgataa aacgggggcg ggcccctgtc       420
   aaacaaaccc tcaattttga cttgctgaag cttgctgggg atgtcgagtc cgctgccgcg       480
   gctatggtga gcaagggcga ggagctgttc accggcgttg tgccaatact ggttgagttg       540
   gatggcgatg tcaacggaca caaatttagc gtaagcgggg agggagaggg cgacgccaca       600
   tatggcaagc tgaccctgaa gttcatttgc acgaccggca aattgcccgt cccttggccc       660
   acacttgtga cgaccctgac ttatggcgta cagtgcttca gcaggtaccc tgatcatatg       720
   aagcaacacg acttctttaa gagtgccatg ccagagggat acgtccagga aagaaccata       780
   ttcttcaaag atgatggaaa ttacaaaacc cgggcagagg tcaagtttga aggcgacacc       840
   ctggtgaaca ggatcgaact caaaggcatc gatttcaaag aggacggaaa catcctcgga       900
   cacaaactgg aatacaatta caacagccac aacgtctaca tcatggcaga taaacaaaag       960
   aacggtatta aagtgaactt caagatccgg cacaacatcg aagacggctc cgtccagctt      1020
   gccgaccact accagcaaaa taccccgatc ggcgacggcc ccgttctcct ccccgataat      1080
   cactacctga gtacacagtc agccttgagc aaagacccta atgaaaagcg ggaccacatg      1140
   gttttgctgg agttcgttac cgcagcgggt attacgctgg gtatggacga gctttacaag      1200
   taa                                                                    1203
<210> 106
   <211> 1203
   <212> DNA
   <213> Homo sapiens
<400> 106
   atggtgagca agggcgagga gctgttcacc ggggtggtgc ccatcctggt cgagctggac        60
   ggcgacgtaa acggccacaa gttcagcgtg tccggcgagg gcgagggcga tgccacctac       120
   ggcaagctga ccctgaagtt catctgcacc accggcaagc tgcccgtgcc ctggcccacc       180
   ctcgtgacca ccctgaccta cggcgtgcag tgcttcagcc gctaccccga ccacatgaag       240
   cagcacgact tcttcaagtc cgccatgccc gaaggctacg tccaggagcg caccatcttc       300
   ttcaaggacg acggcaacta caagacccgc gccgaggtga agttcgaggg cgacaccctg       360
   gtgaaccgca tcgagctgaa gggcatcgac ttcaaggagg acggcaacat cctggggcac       420
   aagctggagt acaactacaa cagccacaac gtctatatca tggccgacaa gcagaagaac       480
   ggcatcaagg tgaacttcaa gatccgccac aacatcgagg acggcagcgt gcagctcgcc       540
   gaccactacc agcagaacac ccccatcggc gacggccccg tgctgctgcc cgacaaccac       600
   tacctgagca cccagtccgc cctgagcaag gaccccaacg agaagcgcga tcacatggtc       660
   ctgctggagt tcgtgaccgc cgccgggatc actctcggca tggacgagct gtacaaggct       720
   cccgttaaac aaactctgaa cttcgacctg ctgaagctgg ctggagacgt ggagtccaac       780
   cctggaccta tgaccatcct tttccttact atggttattt catacttcgg ttgcatgaag       840
   gcgcactccg accctgcccg ccgtggggag ctgagcgtgt gtgacagtat tagcgagtgg       900
   gtcacagcgg cagataaaaa gactgcagtg gacatgtctg gcgggacggt cacagtccta       960
   gagaaagtcc cggtatccaa aggccaactg aagcagtatt tctacgagac caagtgtaat      1020
   cccatgggtt acaccaagga aggctgcagg ggcatagaca aaaggcactg gaactcgcaa      1080
   tgccgaacta cccaatcgta tgttcgggcc cttactatgg atagcaaaaa gagaattggc      1140
   tggcgattca taaggataga cacttcctgt gtatgtacac tgaccattaa aaggggaaga      1200
   tag                                                                    1203
<210> 107
   <211> 2940
   <212> DNA
   <213> Homo sapiens
<400> 107
   atgagcccat ggctgaagtg gcacggacca gcaatggcaa gactgtgggg cctgtgcctg 60
   ctggtgctgg gcttctggag agccagcctg gcctgtccaa cctcctgcaa gtgtagctcc 120
   gccaggatct ggtgcacaga gccttctcca ggcatcgtgg cctttccccg cctggagcct 180
   aacagcgtgg atcccgagaa tatcaccgag atcctgatcg ccaaccagaa gcggctggag 240
   atcatcaatg aggacgatgt ggaggcctac gtgggcctga gaaacctgac aatcgtggac 300
   tccggcctga agttcgtggc ctataaggcc tttctgaaga actctaatct gaggcacatc 360
   aacttcaccc gcaataagct gacatctctg agccggagac actttcggca cctggatctg 420
   tccgacctga tcctgaccgg caatccattc acatgctctt gtgacatcat gtggctgaag 480
   accctgcagg agacaaagtc tagccccgat acccaggacc tgtactgtct gaacgagtcc 540
   tctaagaata tgcctctggc caacctgcag atccctaatt gtggactgcc aagcgcccgg 600
   ctggccgcac ctaacctgac agtggaggag ggcaagtccg tgacactgtc ctgttctgtg 660
   ggcggcgatc ccctgcctac cctgtattgg gacgtgggca acctggtgtc taagcacatg 720
   aatgagacct cccacacaca gggctctctg agaatcacaa atatcagctc cgacgatagc 780
   ggcaagcaga tctcttgcgt ggcagagaac ctggtgggag aggatcagga cagcgtgaat 840
   ctgaccgtgc acttcgcccc caccatcaca tttctggagt ctcctaccag cgatcaccac 900
   tggtgcatcc ccttcacagt gcggggaaac ccaaagcccg ccctgcagtg gttttacaac 960
   ggcgccatcc tgaatgagtc caagtatatc tgtaccaaga tccacgtgac caaccacaca 1020
   gagtaccacg gctgcctgca gctggataat cccacccaca tgaacaatgg cgactacaca 1080
   ctgatggcca agaacgagta tggcaaggac gagaggcaga tcagcgccca cttcatgggc 1140
   cgccctggag tggattatga gaccaaccct aattacccag aggtgctgta tgaggactgg 1200
   accacaccta ccgatatcgg cgacaccaca aacaagtcta atgagatccc aagcacagat 1260
   gtggccgacc agtctaacag ggagcacctg agcgtgtacg cagtggtggt catcgcctcc 1320
   gtggtgggct tctgcctgct ggtcatgctg ctgctgctga agctggcccg ccactctaag 1380
   tttggcatga agggcccagc ctccgtgatc tctaatgacg atgacagcgc cagccccctg 1440
   caccacatca gcaacggctc caatacccct tctagctccg agggcggccc agatgccgtg 1500
   atcatcggca tgacaaagat ccccgtgatc gagaaccctc agtacttcgg catcaccaat 1560
   tcccagctga agcctgacac atttgtgcag cacatcaagc ggcacaacat cgtgctgaag 1620
   agggaactgg gagagggagc cttcggcaag gtgtttctgg ccgagtgcta taacctgtgc 1680
   ccagagcagg ataagatcct ggtggccgtg aagaccctga aggatgccag cgacaacgcc 1740
   cggaaggact tccacagaga ggccgagctg ctgacaaatc tgcagcacga gcacatcgtg 1800
   aagttttacg gcgtgtgcgt ggagggcgac cctctgatca tggtgttcga gtatatgaag 1860
   cacggcgatc tgaacaagtt tctgagagca cacggaccag atgccgtgct gatggcagag 1920
   ggaaatcccc ctaccgagct gacacagtct cagatgctgc acattgcaca gcagattgca 1980
   gcaggaatgg tgtacctggc cagccagcac ttcgtgcaca gggatctggc aaccagaaac 2040
   tgcctggtgg gagagaatct gctggtgaag atcggcgact ttggcatgtc ccgggacgtg 2100
   tactctaccg actactatag agtgggcggc cacacaatgc tgcccatcag gtggatgcca 2160
   cccgagagca tcatgtatcg caagttcacc acagagtctg acgtgtggag cctgggcgtg 2220
   gtgctgtggg agatctttac ctacggcaag cagccttggt atcagctgtc caacaatgaa 2280
   gtgatcgagt gtattacaca gggacgcgtg ctgcagaggc cacgcacatg cccccaggag 2340
   gtgtacgagc tgatgctggg ctgttggcag cgggagccac acaccagaaa gaacatcaag 2400
   agcatccaca cactgctgca gaatctggcc aaggcctccc ccgtgtatct ggacatcctg 2460
   ggcagcggag ctactaactt cagcctgctg aagcaggctg gagacgtgga ggagaaccct 2520
   ggacctatga gaatccttct tcttactatg gttatttcat acttcggttg catgaaggcg 2580
   cactccgacc ctgcccgccg tggggagctg agcgtgtgtg acagtattag cgagtgggtc 2640
   acagcggcag ataaaaagac tgcagtggac atgtctggcg ggacggtcac agtcctagag 2700
   aaagtcccgg tatccaaagg ccaactgaag cagtatttct acgagaccaa gtgtaatccc 2760
   atgggttaca ccaaggaagg ctgcaggggc atagacaaaa ggcactggaa ctcgcaatgc 2820
   cgaactaccc aatcgtatgt tcgggccctt actatggata gcaaaaagag aattggctgg 2880
   cgattcataa ggatagacac ttcctgtgta tgtacactga ccattaaaag gggaagatag 2940
<210> 108
   <211> 2943
   <212> DNA
   <213> Homo sapiens
<400> 108
   atgtcatctt ggatccgctg gcacgggcca gcgatggccc gattgtgggg cttctgctgg      60
   cttgttgtag gcttctggcg cgcggcgttc gcgtgtccga cctcttgcaa atgctcagca     120
   agccgaattt ggtgctcaga ccctagtcca ggaattgttg cattcccccg actggaacca     180
   aactccgtcg acccggagaa tataactgag atatttattg caaatcaaaa acgccttgaa     240
   atcattaacg aggatgacgt ggaggcctac gttggtttga gaaatcttac tattgtcgac     300
   tccggactta aatttgtagc tcataaagcc ttcctgaaga actctaatct gcagcacatt     360
   aatttcacga gaaataagct gaccagcttg tcccggaagc atttccgcca tctcgacctg     420
   agcgagctca tactggtcgg aaacccattt acgtgctcct gtgacatcat gtggatcaaa     480
   actctgcaag aggcgaaaag tagtccggat acccaagacc tttactgtct taatgaaagc     540
   tcaaaaaata tcccgctggc caacctgcag ataccgaact gcggacttcc tagtgcgaat 600
   ttggctgccc caaatcttac cgtcgaagaa ggcaaatcaa tcacgctttc ttgttctgta 660
   gctggagatc cagtgcctaa tatgtattgg gacgtgggta acctcgtctc aaaacatatg 720
   aacgaaacga gccacaccca gggctctttg cggataacaa acatctcctc tgatgattct 780
   ggaaagcaaa tcagttgcgt agctgaaaat ctggttggcg aagatcaaga ttcagtcaat 840
   ctgacagtcc atttcgcccc aacgatcacc tttctggaga gcccaactag cgatcaccac 900
   tggtgtattc cgtttacggt aaaaggaaat ccaaaacctg cactccaatg gttttataat 960
   ggagccatct tgaatgaaag caaatatatc tgtactaaaa tccatgtgac gaatcacacc 1020
   gagtatcacg ggtgtcttca attggataat ccaacccata tgaataatgg tgattatact 1080
   ttgatagcga agaacgaata cggcaaagac gaaaagcaaa tatccgcaca tttcatgggt 1140
   tggcctggca tcgacgacgg tgcgaacccg aactacccag atgttattta cgaggattat 1200
   gggactgcgg caaacgacat tggcgacacc acaaaccgaa gcaacgagat accaagtact 1260
   gacgtcactg acaaaacggg tcgagagcat ttgtctgttt acgccgttgt tgttatcgcc 1320
   tcagttgtcg gattttgcct gttggtcatg cttttcctcc tgaagctcgc gcgacattcc 1380
   aagtttggca tgaaggggcc agcaagtgtt atatccaatg atgatgatag cgcttctcca 1440
   ttgcaccaca taagtaacgg ctcaaacacg ccgtcatcta gtgaaggtgg accagacgcg 1500
   gtcattatag ggatgactaa aattcccgta atcgaaaacc ctcagtactt cggcataacc 1560
   aacagtcagc ttaaacccga tactttcgtg cagcacatca aaaggcacaa catagtcctc 1620
   aagcgcgaac tcggggaggg agccttcgga aaggtctttc ttgctgagtg ctataatttg 1680
   tgtcctgagc aggataaaat tcttgtggct gtaaaaactc tcaaagatgc ttccgacaac 1740
   gcacggaagg attttcatcg ggaggccgaa ctgttgacga atttgcagca cgagcatata 1800
   gtaaagttct acggggtatg tgttgagggg gacccgttga ttatggtctt cgagtatatg 1860
   aagcacgggg acctgaacaa atttttgcgc gcccatgggc ctgatgccgt ccttatggca 1920
   gaagggaacc ctccaacaga actcacccag agtcagatgt tgcacatagc gcaacagatc 1980
   gcggccggca tggtttacct ggccagtcaa cacttcgtgc atagagatct tgccactcgc 2040
   aactgtttgg tcggggagaa ccttctggtt aagattggtg actttggtat gtcacgagat 2100
   gtgtattcca ctgactatta cagagttggg ggtcatacaa tgcttcctat tcggtggatg 2160
   ccccccgaat ccatcatgta cagaaagttc acgacagaga gtgatgtttg gagtctcggc 2220
   gtggtgctct gggaaatttt cacatacgga aagcagccgt ggtatcaact tagcaacaat 2280
   gaggtgatag agtgtattac acagggtcgg gtgttgcagc gccctcgaac gtgcccacaa 2340
   gaagtatatg aacttatgct cgggtgctgg caaagagaac cacatatgag aaaaaatatc 2400
   aaggggatac atacattgct tcagaacttg gccaaggcat cacccgtcta cctcgatata     2460
   ctgggcagcg gagctactaa cttcagcctg ctgaagcagg ctggagacgt ggaggagaac     2520
   cctggaccta tgagaatcct tcttcttact atggttattt catacttcgg ttgcatgaag     2580
   gcgcactccg accctgcccg ccgtggggag ctgagcgtgt gtgacagtat tagcgagtgg     2640
   gtcacagcgg cagataaaaa gactgcagtg gacatgtctg gcgggacggt cacagtccta     2700
   gagaaagtcc cggtatccaa aggccaactg aagcagtatt tctacgagac caagtgtaat     2760
   cccatgggtt acaccaagga aggctgcagg ggcatagaca aaaggcactg gaactcgcaa     2820
   tgccgaacta cccaatcgta tgttcgggcc cttactatgg atagcaaaaa gagaattggc     2880
   tggcgattca taaggataga cacttcctgt gtatgtacac tgaccattaa aaggggaaga     2940
   tag                                                                   2943

## Description

The present invention relates to genetic constructs, and in particular to recombinant vectors comprising such constructs, and to the uses of the constructs and vectors in gene therapy methods for the treatment, prevention or amelioration of a neurodegenerative disorder, or for the treatment of stroke, or for promoting nerve regeneration and/or survival. The invention is defined in the claims. Neurodegenerative diseases are those that primarily affect neurons. The degenerative process can involve the progressive loss of neuronal structure, the progressive loss of neuronal function, or progressive neuron cell death. Many specific disorders are categorised as neurodegenerative diseases. Parkinson's disease is a long-term neurodegenerative disorder, and has been estimated to affect approximately seven million people. Huntington's disease is also a long-term neurodegenerative disorder, and so there is a need for improved treatments for Parkinson's disease and Huntington's disease, and the promotion of nerve regeneration or survival could be beneficial to such patients.

Motor neurone disease includes any disorder that has a neurodegenerative effect on motor neurons. This includes amyotrophic lateral sclerosis (ALS), primary lateral sclerosis (PLS), progressive muscular atrophy (PMA), progressive bulbar palsy (PBP), pseudobulbar palsy, or spinal muscular atrophies. Stroke occurs when blood flow to the brain is interrupted or reduced, and the poor blood flow can result in cell death. Alzheimer's disease accounts for about 60% of all dementias, and estimates are that over 26 million people worldwide are reported to have Alzheimer's disease [1]. Dementia involves a progressive decline in mental function, usually including deficits in memory, language and cognitive processes. Alzheimer's disease can not only affect patients themselves, but has a significant impact on the millions of carers, often unpaid, who are needed to look after them. Since the greatest risk factor of Alzheimer's disease is age, there is a dramatic increase in the prevalence as people survive longer in old-age [1]. Increasing numbers of Alzheimer patients is already having major impacts on global healthcare systems. Typical pathology associated with Alzheimer's disease involves gross atrophy of the brain, thinning of the grey matter in the cerebral cortex, enlarged ventricles indicative of neuronal loss, microscopic extracellular amyloid plaques comprising beta-amyloid peptide [Aβ], which aggregate into protein clumps, intracellular neurofibrillary tangles comprising aggregated Tau protein, and cerebrovascular amyloid, i.e. amyloid protein surrounding the blood vessels. In Alzheimer's disease, many areas in the brain have amyloid plaques caused by extracellular deposits of misfolded amyloid β-peptide, and neurofibrillary tangles composed of hyperphosphorylated Tau protein, especially the frontal, temporal and parietal cortices, the hippocampus, and the cholinergic nuclei of the basal forebrain. These brain regions represent key areas involved in the neuronal circuitry essential for short-term memory. Amyloid plaque deposition appears randomly throughout the brain, whereas the appearance of intracellular neurofibrillary tangles seems to follow a well-defined pattern [2] being detected first in the trans-entorhinal cortex. The neurofibrillary tangles are then observed to spread sequentially to the entorhinal cortex, to areas of the hippocampus and then outwards to the cerebral cortex. Numerous studies have indicated that one of the earliest changes in Alzheimer's disease involves the loss of synapses, which correlates with mental decline [3] eventually leading to marked cell loss throughout a number of brain areas. The symptoms of the disease therefore follow the slow progression of destruction throughout the brain, beginning with the inability to make new memories, a process which is dependent on the hippocampus.

Brain-derived neurotrophic factor (BDNF) along with nerve growth factor (NGF), neurotrophin-3 (NT-3), and neurotrophin-4/5 (NT-4/5) are members of the neurotrophin family of trophic factors [4-5]. The neurotrophins play essential roles in the development, survival and function of a wide range of neurons in both the peripheral and central nervous systems. Neurotrophins interact with two cell surface receptors, low affinity P7S^{NTR} receptors and the high affinity tyrosine receptor kinase (Trk) family [4-5]. Nerve growth factor (NGF) preferentially binds TrkA, Brain Derived Neurotrophic Factor (BDNF) and Neurotrophin-4/5 (NT4/5) bind to tropmyosin receptor kinase-B (TrkB), and Neurotrophin-3 (NT-3) binds TrkC (and TrkA to a lesser extent) [12-13].

Brain-derived neurotrophic factor (BDNF) is a protein which is highly expressed and widely distributed throughout the central nervous system, especially in the hippocampus and cerebral cortex [6-7]. It has been shown to be important in the survival and function of hippocampal, cortical, cholinergic and dopaminergic neurons [8]. BDNF is associated with a number of disorders of the brain, including Alzheimer's disease, Huntington's disease, depression, schizophrenia, and Rett syndrome. It has been hypothesised that early memory dysfunction seen in Alzheimer's disease may be related to the levels of BDNF in the hippocampus as there are reports of substantial reduced BDNF mRNA levels in Alzheimer's disease hippocampus [9] and parietal cortex [10] and decreased protein levels of BDNF in entorhinal cortex, hippocampus, temporal, frontal and parietal cortex [11-16]. Changes in BDNF levels, however, seem to be due to specific downregulation of certain BDNF transcripts. Meta-analysis also shows a significant decrease of neurotrophin levels in blood of Alzheimer patients compared to healthy subjects [17]. Moreover lower cerebrospinal fluid concentration of BDNF was shown to predict progression from mild cognitive impairment (MCI) to Alzheimer's disease [18].

A number of studies indicate that subjects exhibiting the Val66Met polymorphism (where the valine is substituted by methionine) of the pro-domain of BDNF is associated with increased progression to Alzheimer's disease [19] and other BDNF polymorphisms may also be implicated. Loss of proBDNF a larger precursor version of BDNF and of mature BDNF (mBDNF) occurs early in the disease (before plaque deposition) and correlates with memory deficits [20-21]. These data strongly suggest a link between reduced BDNF concentrations, synaptic loss and cell dysfunction which underlie Alzheimer's cognitive impairment. BDNF has also been shown to induce rapid Tau dephosphorylation in neuronal cells through interactions with the TrkB receptor and subsequent increase in phosphoinositol-3-kinase (PI3K) and protein kinase (Akt) signalling, [22-23]. Therefore, decreases in BDNF concentrations might also contribute to Tau hyperphosphorylation, a pathological hallmark of AD. There also appears to be converse effect with increased Tau causing a reduction in BDNF expression in mice [24]. Recent data has also demonstrated potential exacerbation in Aβ neurotoxicity in the presence of pro-domain of neurotrophins, including BDNF [25].

Changes in neurons expressing the mBDNF receptor TrkB, have also been found in post-mortem Alzheimer brains. For example, a 47% reduction in TrkB positive neurons has been reported in post-mortem brains from Alzheimer's sufferers [26]. This may be attributed either to a loss of neurons which normally express the receptor or to a biochemical down-regulation of TrkB expression. The decrease of TrkB could also be aggravated by the up-regulation of truncated receptor isoforms TrkB-Ti and TrkB-Shc in both frontal and temporal cortex in Alzheimer's disease which do not display kinase activity essential for neuronal survival [27]. Activation of the protease, calpain, by Aβ in neuronal cultures induces a decrease of TrkB [28] by cleavage near the receptor She docking site leading to the conversion of fully functional receptors into truncated isoform with defective kinase activity. The effect of conversion of functional TrkB receptors into truncated isoform may then act as a neurotrophin sink or dominant negative receptor. In a mouse model of Alzheimer's disease, knockout of the TrkB receptors was observed to exacerbate Alzheimer's disease-like signalling aberrations and memory deficits without affecting the deposition of Aβ [29]. These data suggest that loss in TrkB receptors and/or loss in activity through reduced BDNF production and secretion represent important elements in producing Alzheimer-like symptoms and pathophysiology.

Other important mechanisms contributing to the deficiency of BDNF/TrkB signalling in Alzheimer's brains includes suppression of mitogen activated protein kinase (MAPK/ERK) and PI3K/Akt pathways by sub-lethal concentrations of Aβ, without interference of TrkB-FL and phospholipase-γ (PLCγ) activation [30], and the disruption of BDNF-induced TrkB endocytosis. The exposure to Aβ oligomers can impair receptor endocytosis and downstream Akt activation through glycogen synthase kinase-3β (GSK3β)-mediated dynamin-1 phosphorylation [31]. In addition, the Aβ oligomers have been shown to interfere with BDNF-mediated TrkB retrograde trafficking [32] through disruption of the ubiquitin system [33] and altering calcium homeostasis [34].

The overall picture is for significant impairment of neurotrophic signalling in Alzheimer's disease, and in particular for the BDNF system. Supplementation or boosting BDNF signalling has been examined in several animal models of Alzheimer's disease. For example, injections of BDNF ameliorate learning deficits in a rat model of Alzheimer's disease induced by Aβ[1-42] [35]. Injections of a novel fusion peptide containing the active domain of BDNF with an HIV-encoded transactivator of transcription (TAT) that can penetrate the brain significantly improved spatial memory with activation of the TrkB/ERK1/2/Akt pathway and restoration of several memory-associated proteins in animal models [36]. In addition, expression of BDNF using lentiviral-based gene therapy was shown to have a neuroprotective effect in mouse transgenic models of Alzheimer's disease and in older primates which are showing cognitive decline [37].

BDNF may be produced in the brain and may be transported to the periphery, where it can support neurons and maintain their survival [38-44]. In certain conditions, such as during excitotoxic insults with glutamate receptor agonists, such as N-methyl-D-aspartate, BDNF can also be produced in peripheral neurons although at relatively low levels [45-46]. BDNF is normally produced as a prepro-polypeptide (i.e. preproBDNF) containing a short signal peptide sequence, which facilitates trafficking of the entire polypeptide to vesicles for release into the extracellular space. Cleavage and removal of the signal peptide converts preproBDNF into proBDNF. An N-terminal proBDNF sequence is then cleaved either intracellulary or extracellularly to create mature BDNF (mBDNF) [47]. Both pro-BDNF and mBDNF possess biological activity with pro-BDNF preferentially activating P7S^{NTR} receptors and the shorter mBDNF activating TrkB receptors [48-50]. Activation of P7S^{NTR} and TrkB receptors in the retina, for instance, show opposing effects on retinal ganglion cell (RGC) survival, the former being responsible for apoptosis through direct RGC-cell-body-P75^{NTR}-activation [48-51] or indirectly via P75^{NTR} activation on Müller cells, thereby stimulating release of Tumour Necrosis Factor-alpha (TNF-α) which further promotes RGC loss [52].

Animal models of glaucoma have demonstrated that following nerve crush, or raised IOP, there is a shift away from neurotrophic mBDNF/TrkB signalling towards pro-BDNF/p75^{NTR} pathways. Reduced levels of mBDNF and TrkB receptors in the retina have been demonstrated [50, 53-54] together with opposing elevations in the relative levels of pro-BDNF [28] and p75^{NTR} receptors [55]. Supplementation of mBDNF through ocular injections of recombinant protein to rats with experimentally elevated IOP increases the survival of RGCs compared with untreated eyes, thereby confirming a key neuroprotective role for this neurotrophin [42-44].

In view of the above, there is therefore a need for an improved gene therapy for the promotion of nerve regeneration or survival, for the treatment, prevention, or amelioration of a neurodegenerative disorder or stroke.

The inventors have constructed a novel genetic construct, which encodes the tyrosine kinase receptor B (TrkB), and an agonist of the TrkB receptor under the control of a single promoter. The promoter of the construct may be used to ensure that the agonist and the receptor are only expressed in appropriate nerve cells, and promote the survival of these cells.

Thus, there is provided a genetic construct comprising a promoter operably linked to a first coding sequence, which encodes the tyrosine kinase receptor B (TrkB), and a second coding sequence, which encodes an agonist of the TrkB receptor, for use in the treatment, prevention or amelioration of a neurodegenerative disorder or stroke.

The inventors have demonstrated in the Examples that it is possible to combine the genes which code for both the TrkB receptor and its agonist in a single genetic construct. This was especially challenging given their large sizes, and it could not have been predicted that it would have been possible to co-express them in physiologically useful concentrations. Advantageously, with the construct of the invention, there is no need to inject a recombinant protein, as described in the prior art [56]. Furthermore, in the prior art, it is still necessary to perform regular injections of protein, whereas the construct of the invention only requires a single gene therapy administration.

Preferably, in use, the TrkB receptor is activated by the agonist to thereby promote survival of nerve cells. The genetic construct of the invention is preferably used for the treatment, prevention or amelioration of a neurodegenerative disorder selected from a group consisting of: Alexander's disease, Alper's disease, Alzheimer's Disease, amyotrophic lateral sclerosis (ALS), ataxia telangiectasia, neuronal ceroid lipofuscinoses, Batten disease, bovine spongiform encephalopathy (BSE), Canavan disease, cerebral palsy, Cockayne syndrome, corticobasal degeneration, Creutzfeldt-Jakob disease, frontotemporal lobar degeneration, Gaucher's disease, Huntington's disease, HIV-associated dementia, Kennedy's disease, Krabbe's disease, Lewy body dementia, lysosomal storage disorders, neuroborreliosis, Machado-Joseph disease, motor neurone disease, multiple system atrophy, multiple sclerosis, multiple sulfatase deficiency, mucolipidoses, narcolepsy, Niemann-Pick type C, Niemann Pick disease, Parkinson's Disease, Pelizaeus-Merzbacher Disease, Pick's disease, Pompe disease, primary lateral sclerosis, prion diseases, progressive supranuclear palsy, Refsum's disease, Sandhoff disease, Schilder's disease, subacute combined degeneration of spinal cord secondary to pernicious anaemia, Spielmeyer-Vogt-Sjogren-Batten disease, spinocerebellar ataxia, spinal muscular atrophy, Steele-Richardson-Olszewski disease, Tabes dorsalis, and Tay-Sachs disease.

In a preferred embodiment, the genetic construct is used for the treatment, prevention or amelioration of Alzheimer's disease.

In a preferred embodiment, the genetic construct is for the treatment, prevention or amelioration of Huntington's disease.

In a preferred embodiment, the genetic construct is for the treatment, prevention or amelioration of Parkinson's disease.

In a preferred embodiment, the genetic construct is for the treatment, prevention or amelioration of motor neurone disease.

In a preferred embodiment, the genetic construct is for the treatment, prevention or amelioration of stroke.

The gene therapy construct may have several beneficial therapeutic effects for treating neurodegenerative disorders, such as Alzheimer's disease, or stroke. Benefits include therapeutically supplementing the depleted brain mBDNF concentrations, or supplementing with other trophic factors from the neurotrophin family. Other benefits include restoring TrkB receptor density levels in normal brain tissue. The potential to include an agonist in the genetic construct that has an absence of coding for the pro-sequence, for instance the absence of coding for proBDNF, also has the capability of restoring the balance in favour of mBDNF/TrkB type signalling and away from pro-BDNF/p75NTR type effects. Furthermore, as the gene therapy may be used to produce a mature form of the agonist, such as mBDNF, without generating pro-domain neurotrophin there will be a significantly lower risk of exacerbating the Aβ neurotoxicity, which could occur if the construct produced and released a pro-form of the agonist, such as proBDNF. Preferably, the construct of the invention is configured to reduce Tau phosphorylation in neurones (which is one of the pathophysiological features associated with Alzheimer brains).

Advantageously, the construct of the invention may therefore be used to target nerve cells in order to maintain or enhance TrkB-signalling in these cells. Thus, the construct may be used to maximise protection against pathophysiological stressors, and to promote nerve regeneration and/or survival. Furthermore, the construct may be used to provide long-term treatment of neurodegenerative disorders or strokes due to the expression of the TrkB receptor and an agonist of the receptor under the control of one or more promoter. Consequently, the construct has overcome the need to use multiple alternative treatments, which, even in combination, provide a transient therapeutic effect. Moreover, the construct of the invention is advantageous because it may be used to significantly enhance nerve cell sensitivity to TrkB receptor agonists due to a localised increase in both the TrkB receptor and the agonist of the receptor.

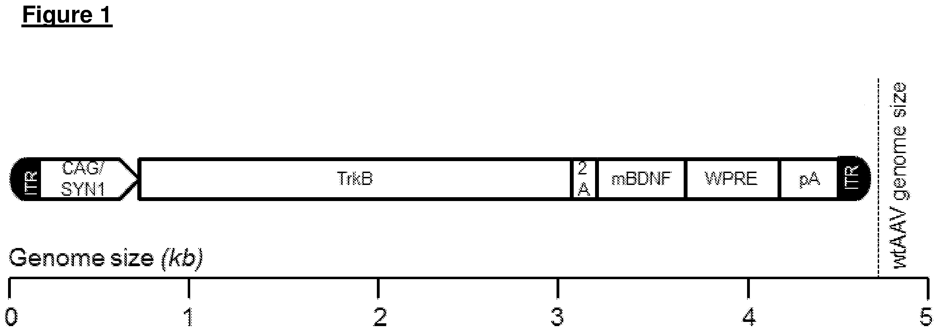

Preferably, the genetic construct of the invention comprises an expression cassette, one embodiment of which is shown in Figure 1. As can be seen in Figure 1, the construct comprises the promoter, the first nucleotide sequence encoding the TrkB receptor, and the second nucleotide sequence encoding mature brain derived neurotrophic (mBDNF), which acts as a preferred agonist of the TrkB receptor. It will be appreciated, however, that other agonist may be used, as discussed herein. Also as shown in Figure 1, the expression cassette also includes a 2A spacer sequence, a sequence encoding Hepatitis Virus Post-transciptional Regulatory Element (WHPE), a sequence encoding a polyA tail, and left and right hand Inverted Terminal Repeat sequences (ITRs).

The genetic construct comprises a spacer sequence disposed between the first and second coding sequences, which spacer sequence encodes a peptide spacer that is configured to be digested or cut to thereby produce the TrkB receptor and the agonist as separate molecules. In the embodiment illustrated in Figure 1, the coding sequence for the TrkB receptor is disposed 5' of the coding sequence for the receptor agonist (BDNF) with the spacer sequence therebetween. However, in another embodiment, the coding sequence for the receptor agonist may be disposed 5' of the coding sequence for the receptor with the spacer sequence therebetween.

Preferably, the genetic construct comprises a nucleotide sequence encoding Woodchuck Hepatitis Virus Post-transcriptional Regulatory Element (WHPE), which enhances the expression of the two transgenes, i.e. the TrkB receptor and its agonist, which is preferably BDNF. Preferably, the WHPE coding sequence is disposed 3' of the transgene coding sequence.

One embodiment of the Woodchuck Hepatitis Virus Post-transcriptional Regulatory Element (WHPE) is 592bp long, including gamma-alpha-beta elements, and is referred to herein as SEQ ID No: 57, as follows:

Preferably, the WHPE comprises a nucleic acid sequence substantially as set out in SEQ ID No: 57, or a fragment or variant thereof.

However, in a preferred embodiment, a truncated WHPE is used, which is 247bp long due to deletion of the beta element, and which is referred to herein as SEQ ID No: 58, as follows:

Advantageously, the truncated WHPE sequence used in the construct saved about 300bp in total without negatively impacting on transgene expression. Preferably, the WHPE comprises a nucleic acid sequence substantially as set out in SEQ ID No: 58, or a fragment or variant thereof.

Preferably, the genetic construct comprises a nucleotide sequence encoding a polyA tail. Preferably, the polyA tail coding sequence is disposed 3' of the transgene coding sequence, and preferably 3' of the WHPE coding sequence.

Preferably, the polyA tail comprises the simian virus 40 poly-A 224 bp sequence. One embodiment of the polyA tail is referred to herein as SEQ ID No: 59, as follows:

Preferably, the polyA tail comprises a nucleic acid sequence substantially as set out in SEQ ID No: 59, or a fragment or variant thereof.

Preferably, the genetic construct comprises left and/or right Inverted Terminal Repeat sequences (ITRs). Preferably, each ITR is disposed at the 5' and/or 3' end of the construct.

The promoter in the genetic construct of the first aspect may be any nucleotide sequence that is capable of inducing RNA polymerase to bind to and transcribe the first and second coding sequences. In one embodiment, the promoter in the genetic construct of the first aspect may be the cytomelalovirus (CMV) constitutive promoter. This is believes to be non-selective for both neuronal and glial cells.

In one preferred embodiment, the promoter is the human synapsin I (SYN I) promoter, which has been shown to work in human brain. One embodiment of the 469 nucleotide sequence encoding the human synapsin I (SYN I) promoter is referred to herein as SEQ ID NO.1, as follows:

Preferably, therefore, the promoter may comprise a nucleotide acid sequence substantially as set out in SEQ ID No: 1, or a fragment or variant thereof.

In another preferred embodiment, the promoter is the CAG promoter, which has also been shown to work in human brain. The CAG promoter preferably comprises the cytomegalovirus early enhancer element, the first exon and the first intron of chicken beta-actin gene and the splice acceptor of the rabbit beta-globin gene. One embodiment of the 1733 nucleotide sequence encoding the CAG promoter is referred to herein as SEQ ID NO.2, as follows:

In another preferred embodiment, the promoter is a truncated form of the CAG promoter, such as a 664 nucleotide form of the promoter referred to herein as SEQ ID NO.3, as follows:

In yet a further preferred embodiment, the promoter is a truncated form of the CAG promoter, such as a 584 nucleotide form of the promoter referred to herein as SEQ ID NO. 48, as follows:

Therefore, preferably the promoter comprises a nucleotide acid sequence substantially as set out in SEQ ID No: 2, 3 or 48, or a fragment or variant thereof.

Many bicistronic gene constructs presented in the scientific literature have either (i) incorporated dual promoters to separately drive expression of two genes, or (ii) use the internal ribosome entry site (IRES) of the encepahlomyocarditis virus (EMCV) to link two genes transcribed from a single promoter within recombinant viral vectors [45-46]. However, the efficiency of IRES-dependent translation may vary in different cells and tissues and IRES-dependent second gene expression can be significantly lower than cap-dependent first gene expression in bicistronic vectors [47]. Moreover, the size limitation of rAAV vectors (generally <5kb) will prevent the incorporation of large gene constructs, such as the TrkB receptor together with BDNF using dual promoters or IRES linkers.

Accordingly, the genetic construct comprises a spacer sequence disposed between the first and second coding sequences, which spacer sequence encodes a peptide spacer that is configured to be digested to thereby produce the TrkB receptor and agonist as separate molecules. Preferably, the spacer sequence comprises and encodes a viral peptide spacer sequence, more preferably a viral 2A peptide spacer sequence [47]. Preferably, the 2A peptide sequence connects the first coding sequence to the second coding sequence. This enables the construct to overcome the size restrictions that occur with expression in various vectors and enables expression of all of the peptides encoded by the construct of the first aspect to occur under control of a single promoter, as a single protein.

Thus, following the translation of the single protein containing the sequences of TrkB, the 2A peptide, and the agonist (preferably BDNF), cleavage occurs in the viral 2A peptide sequence at the terminal glycine-proline link, thereby liberating two proteins, i.e. TrkB and agonist (e.g. mBDNF). The genetic construct is designed such that the remaining short N-terminal amino acid sequence of the viral 2A peptide remain attached to the intracellular portion of the TrkB receptor, thereby removing immunogenicity risks and not interfering with the intracellular signalling capability of the mature receptor. The residual proline amino acid from the C-terminal viral 2A sequence remains attached to the N-terminal agonist signal peptide and is ultimately removed from the agonist protein following cleavage of the signal sequence from the mature protein.

The inventors have generated two embodiments of the spacer sequence. One important section of the peptide spacer sequence, which is common to both embodiments described herein, is the C-terminus. Accordingly, preferably the peptide spacer sequence comprises an amino acid sequence referred to herein as SEQ ID NO.4, or a fragment or variant thereof, as follows:
QAGDVEENPGP
[SEQ ID No: 4]

Preferably, the digestion or cut site of the peptide spacer sequence is disposed between the terminal glycine and end proline in SEQ ID No:4.

In a first preferred embodiment, the spacer sequence comprises a nucleotide sequence referred to herein as SEQ ID NO.5, or a fragment or variant thereof, as follows:
GGAAGCGGAGCTACTAACTTCAGCCTGCTGAAGGCTGGAGACGTGGAGGAGAACCCTGGACCT
[SEQ ID No: 5]

In this first embodiment, the peptide spacer sequence comprises an amino acid sequence referred to herein as SEQ ID NO.6, or a fragment or variant thereof, as follows:
GSGATNFSLLQAGDVEENPGP
[SEQ ID No: 6]

In a second preferred embodiment, the spacer sequence comprises a nucleotide sequence referred to herein as SEQ ID NO.7, or a fragment or variant thereof, as follows:
AGCGGAGCTACTAACTTCAGCCTGCTGAAGCAGGCTGGAGACGTGGAGGAGAACCCTGGACCT
[SEQ ID No: 7]

In this second embodiment, the peptide spacer sequence comprises an amino acid sequence referred to herein as SEQ ID NO.8, or a fragment or variant thereof, as follows:
SGATNFSLLKQAGDVEENPGP
[SEQ ID No: 8]

The inventors have carefully considered the sequences of the TrkB receptor, and have produced several preferred embodiments of the receptor that is encoded by the first coding sequence in the genetic construct of the first aspect.

In one preferred embodiment, the first coding sequence comprises a nucleotide sequence encoding the human canonical isoform of TrkB. Preferably, the canonical isoform of TrkB comprises an amino acid sequence (822 residues) referred to herein as SEQ ID NO.9, or a fragment or variant thereof, as set out below:

Preferably, in this embodiment, the first coding sequence comprises a nucleotide sequence referred to herein as SEQ ID NO. 10, or a fragment or variant thereof, as set out below:

In another preferred embodiment, the first coding sequence comprises a nucleotide sequence which encodes isoform 4 of TrkB. Preferably, isoform 4 of TrkB comprises an amino acid sequence referred to herein as SEQ ID NO. 11, or a fragment or variant thereof, as set out below:

Preferably, this embodiment of the first coding sequence comprises a nucleotide sequence referred to herein as SEQ ID NO. 12, or a fragment or variant thereof, as set out below:

The inventors have spent considerable inventive endeavour in studying the sequence of the TrkB receptor and have realised that TrkB comprises five tyrosine residues (at position 516, 701, 705, 706 and 816 of SEQ ID No: 9), which are normally phosphorylated following dimerization and autophosphorylation in the presence of a BDNF dimer. A problem with phosphorylation of these five tyrosine residues is that the receptor can be readily deactivated by a phosphatase, such as the Shp-2 phosphatase. Accordingly, in order to prevent phosphorylation and resultant deactivation of the receptor *in vivo,* preferably one or more of these key tyrosines is mutated (more preferably, to glutamic acid) in order to mimic the resultant phosphotyrosine and produce a receptor which remains active in the presence of BDNF, and which cannot be deactivated by a phosphatise, such as the Shp-2 phosphatase. Such mutant forms of TrkB are aimed at producing TrkB receptor activity which remains active for longer periods, or until the receptor is internalised.

The DNA and amino acid sequences provided below illustrate the positions of these five tyrosine (Y) residues which have been mutated into five glutamic acid (E) residues. It will be appreciated that 1, 2, 3, 4 or 5 of these residues may be mutated to glutamic acid in embodiments of the invention. Various combinations of these mutations is also envisaged, e.g. positions 516 and 701 only, or positions 705, 706 and 816 only, and so on.

Accordingly, in another preferred embodiment, the first coding sequence comprises a nucleotide sequence encoding a mutant form of TrkB receptor, wherein one or more tyrosine residue at position 516, 701, 705, 706 and/or 816 of SEQ ID No: 9 is modified or mutated. Preferably, at least two, three or four tyrosine residues at position 516, 701, 705, 706 and/or 816 of SEQ ID No: 9 are modified. Most preferably, all five tyrosine residues at position 516, 701, 705, 706 and/or 816 of SEQ ID No: 9 are modified.

Preferably, the or each tyrosine residue is modified to a different amino acid residue, more preferably a glutamic acid. Thus, preferably the mutant form of the TrkB receptor comprises Y516E, Y701E, Y705E, Y706E and/or Y816E.

Preferably, the modified form of the TrkB receptor comprises an amino acid sequence referred to herein as SEQ ID NO. 13, or a fragment or variant thereof, as set out below:

Preferably, in this embodiment, the first coding sequence comprises a nucleotide sequence referred to herein as SEQ ID NO. 14, or a fragment or variant thereof, as set out below:

It will be appreciated that the second coding sequence encodes an agonist of the TrkB receptor, which is preferably a member of the neurotrophin family of trophic factors. The agonist of the TrkB receptor may be a member of the neurotrophin family of trophic factors lacking the pro-sequence. The agonist of the TrkB receptor may be a member of the neurotrophin family of trophic factors in the mature form. Preferred agonists of the TrkB receptor may therefore be selected from a group of agonists consisting of: Brain-derived neurotrophic factor (BDNF); nerve growth factor (NGF); neurotrophin-3 (NT-3); neurotrophin-4 (NT-4); and neurotrophin-5 (NT-5); or fragments thereof. Preferred agonists of the TrkB receptor may be selected from a group of agonists consisting of: Brain-derived neurotrophic factor (BDNF) lacking the pro-sequence; nerve growth factor (NGF) lacking the pro-sequence; neurotrophin-3 (NT-3) lacking the pro-sequence; neurotrophin-4 (NT-4) lacking the pro-sequence; and neurotrophin-5 (NT-5) lacking the pro-sequence; or fragments thereof. Preferred agonists of the TrkB receptor may be selected from a group of agonists consisting of: mature Brain-derived neurotrophic factor (BDNF); mature nerve growth factor (NGF); mature neurotrophin-3 (NT-3); mature neurotrophin-4 (NT-4); and mature neurotrophin-5 (NT-5); or fragments thereof.

The nucleotide and amino acid sequences of each of these agonists will be known to the skilled person. However, by way of example, the amino acid sequence of one embodiment of Neurotrophin-4 (NT-4) is substantially as set out in SEQ ID NO. 49, as follows:

The nucleic acid coding sequence of this embodiment of Neurotrophin-4 (NT-4) is substantially as set out in SEQ ID NO. 50, as follows:

The amino acid sequence of the signal peptide for the NT-4 sequence is substantially as set out in SEQ ID NO. 51, as follows:
MLPLPSCSLPILLLFLLPSVPIES
[SEQ ID No: 51]

The nucleic acid sequence of this signal peptide is substantially as set out in SEQ ID NO. 52, as follows:

The amino acid sequence of the propeptide for this NT-4 sequence is substantially as set out in SEQ ID NO. 53, as follows:
QPPPSTLPPFLAPEWDLLSPRVVLSRGAPAGPPLLFLLEAGAFRESAGAPANRSRR
[SEQ ID No: 53]

The nucleic acid sequence of this propeptide is substantially as set out in SEQ ID NO. 54, as follows:

The amino acid sequence of the mature protein sequence for this NT-4 sequence is substantially as set out in SEQ ID NO. 55, as follows:

The nucleic acid coding sequence of this mature NT-4 protein is substantially as set out in SEQ ID NO. 56, as follows:

Accordingly, in one preferred embodiment, the second coding sequence encodes neurotrophin-4 (NT-4), which may comprise an amino acid sequence substantially as set out in SEQ ID NO: 49 or 55, or fragment or variant thereof. Thus, the second coding sequence may comprise a nucleotide sequence substantially as set out in SEQ ID No: 50 or 56, or a fragment or variant thereof.

Most preferred agonists of the TrkB receptor, however, include prepro-brain derived neurotrophic factor (pre-pro-BDNF), pro-BDNF or mature BDNF (mBDNF). BDNF is initially synthesised as the precursor protein, preproBDNF, by ribosomes found on endoplasmic reticulum. There are at least 17 known splice variants encoded by the human preproBDNF gene (ENSG00000176697). Once preproBDNF has entered into the rough endoplasmic reticulum, preproBDNF is converted into proBDNF by cleavage of the signal peptide (i.e. the "pre" sequence). proBDNF is converted into mBDNF by cleavage of an additional N-terminal peptide sequence that is present on proBDNF.

Both proBDNF and mBDNF are then secreted into the extracellular space, where they bind to and activate receptors on various cells.

proBDNF preferentially binds to and activates the receptor, p75^{NTR}, which, when activated, can induce apoptosis in some cell types. Thus, in one preferred embodiment, proBDNF is an agonist of the p75^{NTR} receptor. In one embodiment, the proBDNF is canonical proBDNF. Preferably, canonical proBDNF comprises an amino acid sequence referred to herein as SEQ ID NO. 15, or a fragment or variant thereof, as set out below:

Preferably, in this embodiment, the second coding sequence comprises a nucleotide sequence referred to herein as SEQ ID NO. 16, or a fragment or variant thereof, as set out below:

In another embodiment, the proBDNF is isoform 2 of proBDNF, which preferably comprises a Valine to Methionione mutation (amino acid underlined). Preferably, isoform 2 of proBDNF comprises an amino acid sequence referred to herein as SEQ ID NO. 17, or a fragment or variant thereof, as set out below:

In one embodiment, however, the agonist is not proBDNF, or a fragment or variant thereof, but instead the second coding sequence preferably comprises a nucleotide sequence which encodes mature BDNF. Mature BDNF (mBDNF) preferentially binds to, and activates, TrkB, which, when activated, promotes survival of nerve cells. Thus, mature BDNF is a most preferred agonist of TrkB. The construct according to the first aspect is advantageous because, unlike other known genetic constructs, the construct is capable of producing mature BDNF protein, which has not been mis-folded.

Thus, in one preferred embodiment, the second coding sequence comprises a nucleotide sequence which encodes mature BDNF. mBDNF is common to all 17 isoforms encoded by the gene. There 7 protein different sequences, five of which have extended signal sequences to the canonical form, and one has a canonical signal sequence, but a Valine to Methionine mutation (which is common to isoforms 2, 4, 7, 8, 9, 10, 11, 12, 13, 14 and 16). It is believed that the valine to methionine mutation reduces release of BDNF from the cell.

Preferably, mature BDNF comprises an amino acid sequence referred to herein as SEQ ID NO. 18, or a fragment or variant thereof, as set out below:

Preferably, this embodiment of the second coding sequence comprises a nucleotide sequence referred to herein as SEQ ID NO. 19, or a fragment or variant thereof, as set out below:

In another embodiment, the agonist is member of the neurotrophin family of trophic factors lacking the pro-sequence but with a signal peptide conjugated to the N-terminus. The agonist may be any member of the neurotrophin family of trophic factors in the mature form and with a signal peptide conjugated to the N-terminus. The signal peptide may be any signal peptide that promotes the proper folding or production of the agonist. In preferred embodiments, the signal peptide may be any signal peptide disclosed herein.

In yet another preferred embodiment, the agonist is mBDNF with a signal peptide conjugated to its N-terminus. As discussed below, the signal peptide may be canonical signal peptide of preproBDNF, or the signal peptide of IL-2, or a de novo novel signal sequence created by the inventors.

Preferably, the second coding sequence comprises a nucleotide sequence encoding a signal peptide for the agonist of the TrkB receptor, most preferably a signal peptide for BDNF. In one preferred embodiment, the nucleotide sequence encodes the canonical signal peptide for BDNF. Preferably, this embodiment of the second coding sequence comprises a nucleotide sequence which encodes a signal peptide comprising an amino acid sequence referred to herein as SEQ ID NO. 20, or a fragment or variant thereof, as set out below:
MTILFLTMVISYFGCMKA
[SEQ ID No: 20]

Preferably, this embodiment of the second coding sequence comprises a nucleotide sequence referred to herein as SEQ ID NO. 21, or a fragment or variant thereof, as set out below:
ATGACCATCCTTTTCCTTACTATGGTTATTTCATACTTCGGTTGCATGAAGGCG
[SEQ ID No: 21]

The inventors have created a series of extended signal peptides. In preferred embodiments, the nucleotide sequence encoding an isoform signal peptide for BDNF is selected from the group consisting of: isoform 2, 3, 6, 5 and 4. The nucleic acid and amino acid sequences for each of these extended signal peptides are set out below.
**Isoform 2**
   MFHQVRRVMTILFLTMVISYFGCMKA
   [SEQ ID No: 22]
**Isoform 3 and 6**
   MQSREEEWFHQVRRVMTILFLTMVISYFGCMKA
   [SEQ ID No: 24]
**Isoform 5**
   MLCAISLCARVRKLRSAGRCGKFHQVRRVMTILFLTMVISYFGCMKA
   [SEQ ID No: 26]
**Isoform 4**

Accordingly, in preferred embodiments, the second coding sequence comprises a nucleotide sequence encoding a signal sequence peptide referred to herein as any one of SEQ ID NO. 23, 25, 27 or 29. Preferably, the signal peptide comprises an amino acid sequence referred to herein as any one of SEQ ID NO. 22, 24, 26 or 28.

The inventors have also created various embodiments of novel signal peptides for the agonist, preferably BDNF. These signal peptides increase the level of basicity of the N-terminal section (with added lysine (K) and arginine (R) residues) and the proceeding hydrophobic region (with additions of leucine (L) residues), which increase secretion of BDNF compared to levels observed with the wild-type canonical signal sequence.
**a) QTA003P (IL-2 signal)**
   MYRMQLLSCIALSLALVTNS
      [SEQ ID No: 30]
   ATGTACAGGATGCAACTCCTGTCTTGCATTGCACTAAGTCTTGCACTTGTCACAAACAGT
      [SEQ ID No: 31]
**b) QTA004P**
   MKRRVMIILFLTMVISYFGCMK
      [SEQ ID No: 32]
   ATGAAAAGAAGAGTGATGATCATCCTTTTCCTTACTATGGTTATTTCATACTTCGGTTGCATGAAGAGCG
      [SEQ ID No: 33]
**c) QTA009P (modified IL-2)**
   MRRMQLLLLIALSLALVTNS
      [SEQ ID No: 34]
   ATGAGGAGGATGCAACTCCTGCTCCTGATTGCACTAAGTCTTGCACTTGTCACAAACAGT
      [SEQ ID No: 35]
**d) QTA010P**
   MRRMQLLLLTMVISYFGCMKA
      [SEQ ID No: 36]
   ATGAGGAGGATGCAACTCCTGCTCCTGACTATGGTTATTTCATACTTCGGTTGCATGAAGGCG
      [SEQ ID No: 37]
**e) QTA0012P**
   MRILLLTMVISYFGCMKA
      [SEQ ID No: 38]
   ATGAGAATCCTTCTTCTTACTATGGTTATTTCATACTTCGGTTGCATGAAGGCG
      [SEQ ID No: 39]
**f) QTA0013P**
   MRRILFLTMVISYFGCMKA
      [SEQ ID No: 40]
   ATGAGAAGAATCCTTTTCCTTACTATGGTTATTTCATACTTCGGTTGCATGAAGGCG
      [SEQ ID No: 41]
**g) QTA0014P**
   MRRFLFLLVISYFGCMKA
      [SEQ ID No: 42]
   ATGAGGAGGTTCCTTTTCCTTCTTGTTATTTCATACTTCGGTTGCATGAAGGCG
      [SEQ ID No: 43]
**i) QTA0015P**
   MRRFLFLLYFGCMKA
      [SEQ ID No: 44]
   ATGAGGAGGTTCCTTTTCCTTCTTTACTTCGGTTGCATGAAGGCG
      [SEQ ID No: 45]

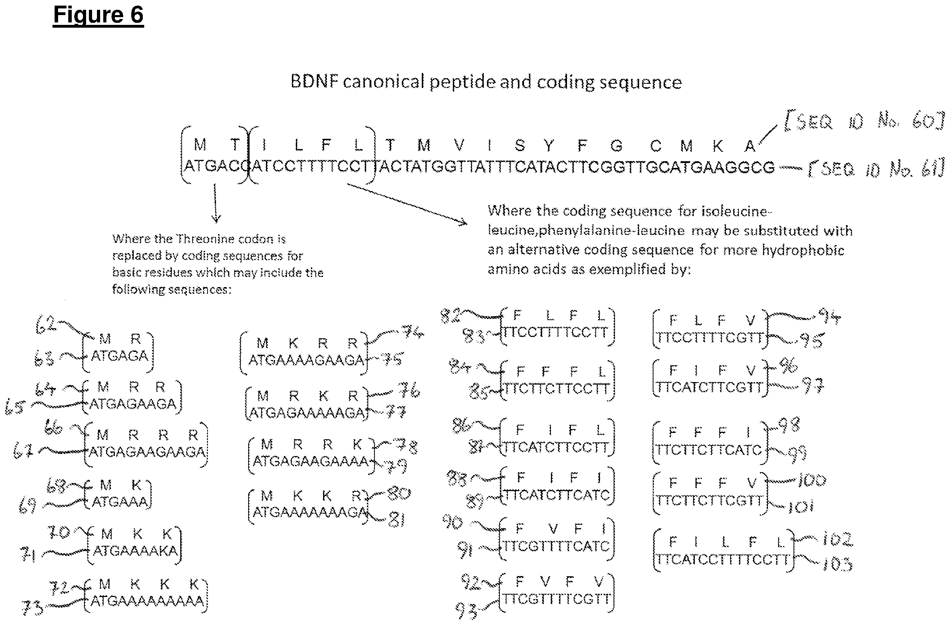

Figure 6 shows nucleotide and amino acid sequences for further preferred embodiments of signal peptide used in the construct of the invention to boost secretion of the agonist, preferably BDNF. The second residue in the signal peptide is threonine (T) which is preferably replaced by one or more basic residue, such as lysine (K) or arginine (R). The next stretch of residues in the signal peptide including isoleucine (I), leucine (L), phenylalanine (F) and Leucine (L) is preferably replaced by one or more hydrophobic residues.

Accordingly, in preferred embodiments, the second coding sequence comprises a nucleotide sequence encoding a signal sequence peptide referred to herein as any one of SEQ ID NO. 31, 33, 35, 37, 39, 41, 43, 45, 61, 63, 65, 67, 69, 71, 73, 75, 77, 79, 81, 83, 85, 87, 89, 91, 93, 95, 97, 99, 101 or 103. Preferably, the signal peptide comprises an amino acid sequence referred to herein as any one of SEQ ID NO. 30, 32, 34, 36, 38, 40, 42, 44, 60, 62, 64, 66, 68, 70, 72, 74, 76, 78, 80, 82, 84, 86, 88, 90, 92, 94, 96, 98, 100 or 102.

Accordingly, it will be appreciated that the inventors have modified the BDNF gene sequence by removal of the pro-sequence, which also has never been achieved before, with the result of generated properly folded mature BDNF, combined with the introduction of completely novel signal peptides, which significantly boost BDNF production and release above that ever achieved with the endogenous sequence.

Preferably, the genetic construct comprises left and/or right Inverted Terminal Repeat sequences (ITRs). Preferably, each ITR is disposed at the 5' and/or 3' end of the construct. An ITR can be specific to a virus (e.g. AAV or lentivirus) serotype, and can be any sequence, so long as it forms a hairpin loop in its secondary structure.

The DNA sequence of one embodiment (left ITR from a commercially available AAV plasmid) of the ITR is represented herein as SEQ ID No: 46, as follows:

The DNA sequence of another embodiment (right ITR from a commercially available AAV plasmid) of the ITR is represented herein as SEQ ID No: 47, as follows:

From the foregoing, the skilled person will appreciate the nucleotide sequence of an embodiment of the construct of the first aspect, as well as the amino acid sequence of the encoded transgene. However, for the avoidance of doubt, the coding sequence of codon optimised 2940 bp sequence for murine TrkB receptor-viral-2A peptide-mBDNF contained within the plasmid QTA020P (and the vector QTA020V), is referred to here as SEQ ID No: 107, as follows:

The coding sequence of codon optimised 2943 bp sequence for human TrkB receptor-viral-2A peptide-mBDNF contained within the plasmid QTA029P (and the vector QTA029V), is referred to here as SEQ ID No: 108, as follows:

Hence, in a most preferred embodiment, the construct comprises a nucleotide sequence substantially as set out in SEQ ID No: 107 or 108, or a fragment or variant thereof.

The inventors have created a series of recombinant expression vectors comprising the construct of the invention.

Thus, according to a second aspect, there is provided a recombinant vector comprising the genetic construct according to the first aspect, for use in the treatment, prevention or amelioration of a neurodegenerative disorder or stroke.

The constructs and expression vectors described herein can be used to promote nerve regeneration and survival. In some embodiments, the recombinant vector is for the treatment, prevention or amelioration of Alzheimer's disease, Huntington's disease, Parkinson's disease, motor neurone disease, or stroke. The recombination vectors described herein may be for any treatment or use as described herein.

The recombinant vector may be a recombinant AAV (rAAV) vector. The rAAV may be a naturally occurring vector or a vector with a hybrid AAV serotype. The rAAV may be AAV-1, AAV-2, AAV-3A, AAV-3B, AAV-4, AAV-5, AAV-6, AAV-7, AAV-8, AAV-9, AAV-10, and AAV-11. Preferably, the rAAV is rAAV serotype-2.

Advantageously, recombinant AAV2 evokes a minimal immune response in host organisms and mediates long-term transgene expression that can persist for at least one year after vector administration.

The term "recombinant AAV (rAAV) vector" as used herein means a recombinant AAV-derived nucleic acid containing at least one terminal repeat sequence.

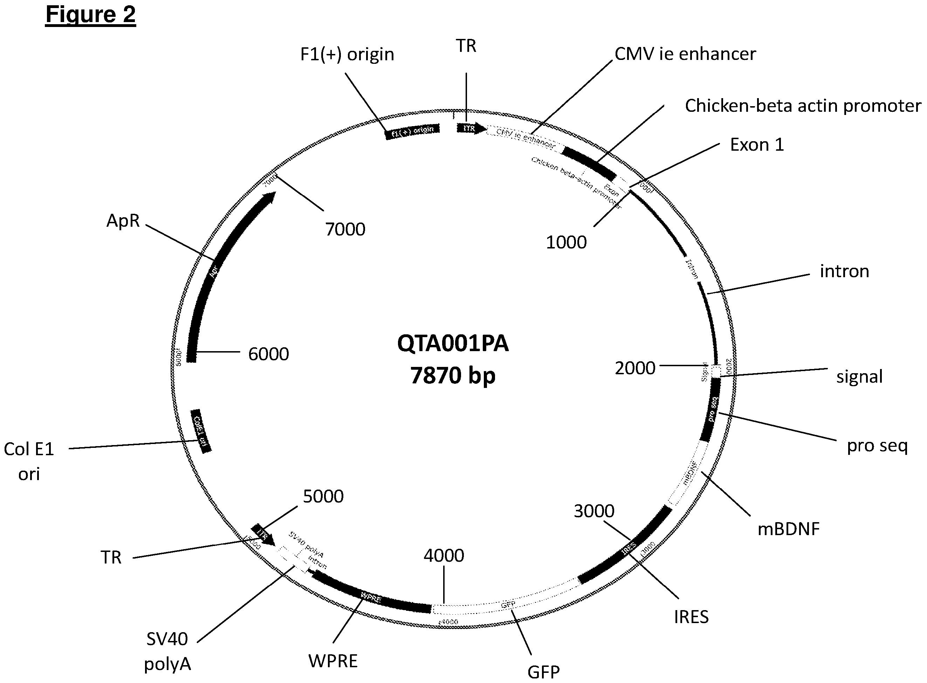

Preferred embodiments of the vector are shown in Figures 2-5.

There is provided a method of treating, preventing or ameliorating a neurodegenerative disorder or stroke in a subject, or for promoting nerve regeneration and/or survival in a subject, the method comprising administering, to a subject in need of such treatment, a therapeutically effective amount of the genetic construct according to the first aspect, or the recombinant vector according to the second aspect.

In some embodiments, the method may be for the treatment, prevention, or amelioration of Alzheimer's disease, Parkinson's disease, motor neurone disease, Huntington's disease, or any other neurodegenerative disclosed herein.

Preferably, the genetic construct or the recombinant vector according to invention are used in a gene therapy technique. The agonist encoded by the construct or vector activate the TrkB also encoded by the construct/vector to thereby promote survival of neuronal cells.

In another embodiment, the constructs and vectors may be used to promote nerve regeneration and/or survival.

It will be appreciated that the genetic construct according to the first aspect, or the recombinant vector according to the second aspect may be used in a medicament, which may be used as a monotherapy (i.e. use of the genetic construct according to the first aspect or the vector according to the second aspect of the invention), for treating, ameliorating, or preventing a neurodegenerative disorder or stroke, or for promoting nerve regeneration and/or survival. Alternatively, the genetic construct or the recombinant vector according to the invention may be used as an adjunct to, or in combination with, known therapies for treating, ameliorating, or preventing a neurodegenerative disorder or stroke, or for promoting nerve regeneration and/or survival.

The genetic construct according or the recombinant vector according to the invention may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

The genetic construct or the recombinant vector according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with the genetic construct or the recombinant vector is required and which would normally require frequent administration (e.g. at least daily injection).

In a preferred embodiment, medicaments according to the invention may be administered to a subject by injection into the blood stream, a nerve or directly into a site requiring treatment. For example, the medicament is configured to cross the blood-brain-barrier. Injections may be intravenous (bolus or infusion) or subcutaneous (bolus or infusion), or intradermal (bolus or infusion).

It will be appreciated that the amount of the genetic construct or the recombinant vector that is required is determined by its biological activity and bioavailability, which in turn depends on the mode of administration, the physiochemical properties of the genetic construct or the recombinant vector and whether it is being used as a monotherapy or in a combined therapy. The frequency of administration will also be influenced by the half-life of the cyclic polypeptide within the subject being treated. Optimal dosages to be administered may be determined by those skilled in the art, and will vary with the particular genetic construct or the recombinant vector in use, the strength of the pharmaceutical composition, the mode of administration, and the advancement or stage of the disorder. Additional factors depending on the particular subject being treated will result in a need to adjust dosages, including subject age, weight, gender, diet, and time of administration.

Generally, a daily dose of between 0.001µg/kg of body weight and 10mg/kg of body weight, or between 0.01µg/kg of body weight and 1mg/kg of body weight, of the construct or vector according to the invention may be used for treating, ameliorating, or preventing a neurodegenerative disorder, Alzheimer's disease, Parkinson's disease, Huntington's disease, motor neurone disease, or stroke, depending upon the genetic construct or recombinant vector used.

The genetic construct or the recombinant vector may be administered before, during or after onset of the disorder. Daily doses may be given as a single administration (e.g. a single daily injection or inhalation of a nasal spray). Alternatively, the genetic construct or the recombinant vector may require administration twice or more times during a day. As an example, the genetic construct or the recombinant vector may be administered as two (or more depending upon the severity of the disorder being treated) daily doses of between 0.07 µg and 700 mg (i.e. assuming a body weight of 70 kg). A patient receiving treatment may take a first dose upon waking and then a second dose in the evening (if on a two dose regime) or at 3- or 4-hourly intervals thereafter. Alternatively, a slow release device may be used to provide optimal doses of the genetic construct or the recombinant vector according to the invention to a patient without the need to administer repeated doses.

Known procedures, such as those conventionally employed by the pharmaceutical industry (e.g. *in vivo* experimentation, clinical trials, etc.), may be used to form specific formulations of the genetic construct or the recombinant vector according to the invention and precise therapeutic regimes (such as daily doses of the agents and the frequency of administration). The inventors believe that they are the first to suggest a genetic construct encoding promoter operably linked to coding sequences of a TrkB receptor and a TrkB receptor agonist.

There is provided a pharmaceutical composition comprising the genetic construct or the recombinant vector and a pharmaceutically acceptable vehicle.

There is provided a method of preparing the pharmaceutical composition the method comprising contacting the genetic construct or the recombinant with a pharmaceutically acceptable vehicle.

A "subject" maybe a vertebrate, mammal, or domestic animal. Hence, compositions and medicaments according to the invention may be used to treat any mammal, for example livestock (e.g. a horse), pets, or may be used in other veterinary applications. Most preferably, however, the subject is a human being.

A "therapeutically effective amount" of the genetic construct, the recombinant vector or the pharmaceutical composition is any amount which, when administered to a subject, is the amount of the aforementioned that is needed to treat a neurodegenerative disorder, Alzheimer's disease, Parkinson's disease, Huntington's disease, motor neurone disease, stroke, or produce the desired effect, such as promoting nerve regeneration and/or survival.

For example, the therapeutically effective amount of the genetic construct, the recombinant vector or the pharmaceutical composition used may be from about 0.01 mg to about 800 mg, and preferably from about 0.01 mg to about 500 mg. It is preferred that the amount of the genetic construct, the recombinant vector or the pharmaceutical composition is an amount from about 0.1 mg to about 250 mg, and most preferably from about 0.1 mg to about 20 mg.

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents according to the invention. In tablets, the active agent (e.g. the genetic construct or recombinant vector according to the invention) may be mixed with a vehicle having the necessary compression properties in suitable proportions and compacted in the shape and size desired. The powders and tablets preferably contain up to 99% of the active agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The genetic construct or the recombinant vector according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The genetic construct or the recombinant vector may be prepared as a sterile solid composition that may be dissolved or suspended at the time of administration using sterile water, saline, or other appropriate sterile injectable medium.

The genetic construct, the recombinant vector and the pharmaceutical composition of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The genetic construct, the recombinant vector or the pharmaceutical composition according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

According to a further aspect, there is provided the genetic construct according to the first aspect, or the recombinant vector according to the second aspect, for use in treating, preventing or ameliorating an optic nerve disorder or a cochlear disorder, or for promoting nerve regeneration and/or survival; wherein the second coding sequence comprises the mature form of a trophic factor from the neurotrophin family. The second coding sequence may comprise a signal peptide. The construct or vector may be such that the agonist lacks the pro-sequence but has a signal peptide. The signal peptide may be attached to the N-terminus and may boost secretion, expression, or folding of the agonist. The second coding sequence may comprise any of: mature nerve growth factor (NGF), mature neurotrophin-3 (NT-3), mature neurotrophin-5 (NT-5), or fragments or variants thereof.

It will be appreciated that the invention extends to any nucleic acid or peptide or variant, derivative or analogue thereof, which comprises substantially the amino acid or nucleic acid sequences of any of the sequences referred to herein, including variants or fragments thereof. The terms "substantially the amino acid/nucleotide/peptide sequence", "variant" and "fragment", can be a sequence that has at least 40% sequence identity with the amino acid/nucleotide/peptide sequences of any one of the sequences referred to herein, for example 40% identity with the sequence identified as SEQ ID No: 1-108, and so on.

Amino acid/polynucleotide/polypeptide sequences with a sequence identity which is greater than 65%, more preferably greater than 70%, even more preferably greater than 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90% identity, even more preferably at least 92% identity, even more preferably at least 95% identity, even more preferably at least 97% identity, even more preferably at least 98% identity and, most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (v) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al., 1994, Nucleic Acids Research, 22,4673-4680; Thompson et al., 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)^{∗}100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula:-Sequence Identity = (N/T)^{∗}100.

Alternative methods for identifying similar sequences will be known to those skilled in the art. For example, a substantially similar nucleotide sequence will be encoded by a sequence which hybridizes to DNA sequences or their complements under stringent conditions. By stringent conditions, we mean the nucleotide hybridises to filter-bound DNA or RNA in 3x sodium chloride/sodium citrate (SSC) at approximately 45°C followed by at least one wash in 0.2x SSC/0.1% SDS at approximately 20-65°C. Alternatively, a substantially similar polypeptide may differ by at least 1, but less than 5, 10, 20, 50 or 100 amino acids from the sequences shown in, for example, SEQ ID Nos: 3 and 5.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figure, in which:-
**Figure 1** is schematic of one embodiment of a genetic construct according to the invention;
**Figure 2** is a schematic drawing of a first embodiment of a recombinant vector according to the invention known as "Plasmid QTA001PA" containing canonical signal sequence (blue) plus proBDNF (red) and mBDNF (black). It also includes an -IRES-GFP- sequence (cyan and purple);
**Figure 3** is a schematic drawing of a second embodiment of the recombinant vector according to the invention known as "Plasmid QTA002P" with no proBDNF (but produces only mBDNF) and same signal sequence (blue) as QTA001PA. It also includes an -IRES-GFP- sequence (cyan and purple);
**Figure 4** is a schematic drawing of a third embodiment of the recombinant vector according to the invention known as of "Plasmid QTA003P" with no proBDNF (but produces only mBDNF) and IL-2 signal sequence (blue). It also includes an -IRES-GFP- sequence (cyan and purple);
**Figure 5** is a schematic drawing of a fourth embodiment of a recombinant vector according to the invention known as "Plasmid QTA004P" with no proBDNF (but produces only mBDNF) and a novel signal sequence (blue). It also includes an -IRES-GFP- sequence (cyan and purple);
**Figure** 6 shows nucleotide and amino acid sequences for different embodiments of signal peptide used in the construct of the invention. The second residue is threonine (t) which can be replaced by one or more basic residue, such as lysine (K) or arginine (R). The next stretch of residues including isoleucine (I), leucine (L), phenylalanine (F) and Leucine (L) can be replaced by one or more hydrophobic residues;
**Figure 7** shows release of BDNF from HEK293 cells using a specific ELISA at 24 hours following transduction of a plasmid (4 µg DNA/well) containing genes coding for mBDNF with differing signal peptide sequences and without the coding sequence for the extended proBDNF component (Data shown as mean ± SEM for n = 4);
**Figure 8** shows Western blotting results of cellular concentrations of BDNF-immunoreactive material (arbitrary units) in HEK293 cell lysates 24 hours after plasmid transduction (Data shown as mean ± SEM for n = 4);
**Figure 9** shows BDNF-immunoreactivity in Western blots of cell lysates showing two molecular weight bands (32kDa and 14kDa) when cells were transduced with QTA001PA, versus only a single 14kDa band with QTA002P, QTA003P and QTA004P transduction;
**Figure 10** shows proBDNF concentrations in the HEK293 incubation medium as measured using a specific ELISA 24 hours after plasmid transduction using a selective proBDNF ELISA (Data shown as mean ± SEM for n = 4);
**Figure 11** shows BDNF expression in HEK293 cell lysate by plasmids QTA002P (endogenous canonical signal peptide sequence), and QTA009P to QTA013P. Data is shown as mean + S.E.M. ** P<0.01 as compared to QTA002P;
**Figure 12** shows BDNF expression in HEK293 cell incubation medium by plasmids QTA002P (endogenous canonical signal peptide sequence), and QTA009P to QTA013P.
Data is shown as mean + S.E.M. ** P<0.01 as compared to QTA002P;

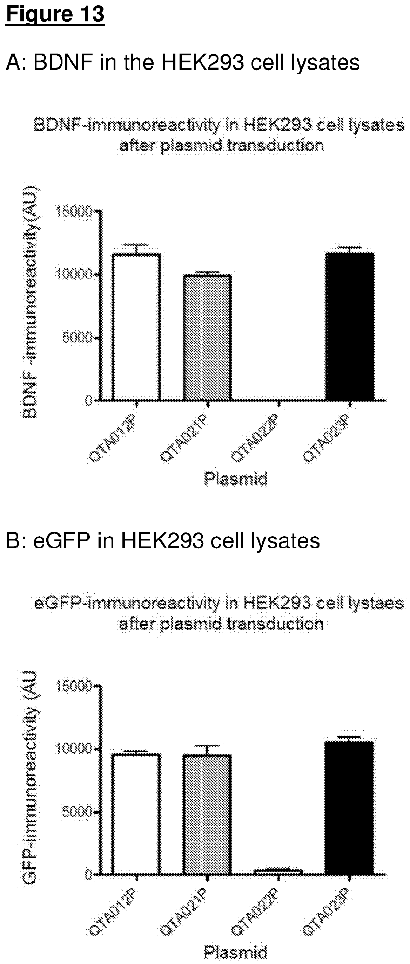

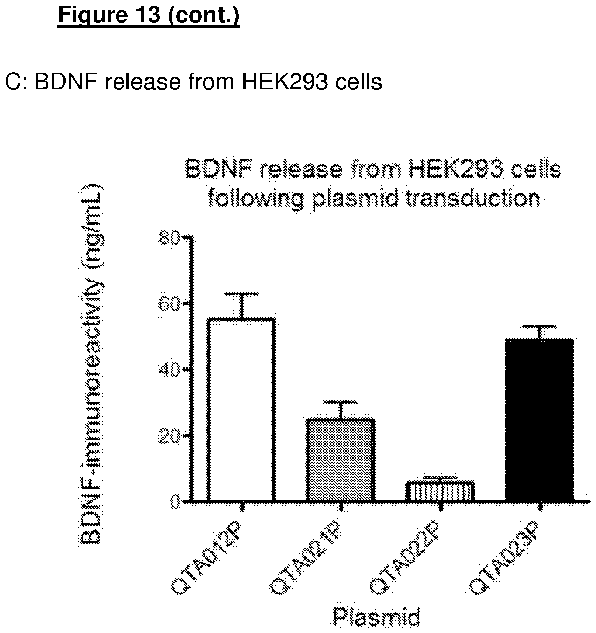

**Figure 13** shows Western Blots from HEK293 cells 24 hours after they were transduced with plasmids QTA015P (expressing BDNF and eGFP separated by an IRES spacer), QTA021P (expressing BDNF followed by eGFP separated by a functional viral-2A peptide sequence), QTA022P (expressing BDNF followed by eGFP separated by a non-functional viral-2A peptide sequence) and QTA023P (expressing eGFP followed by coding for BDNF separated by a functional viral-2A peptide sequence). Data shown as BDNF-immunoreactivity (A), eGFP-immunoreactivity (B) and the amount of BDNF released from the HEK293 cells into the incubation medium (C). Data is shown as mean + S.E.M of the density in the bands;
**Figure 14A** shows Western blot of HEK293 cell homogenates 48 hours after transfection with the QTA020V vector and showing efficient processing of the large precursor coding region which includes the TrkB receptor and BDNF separated by the viral-2A peptide sequence. **Figures 14B and 14C** show that the transgene proteins produced after vial-2A peptide cleavage have been transported to the correct intracellular compartments in HEK293 cells after processing (TrkB receptors to the cell surface and BDNF to storage vesicles prior to release);
**Figure 15A** shows TrkB receptor expression and **Figure 15B** shows BDNF expression in mouse retinal homogenate for the rAAV2 vector, QTA020V. Data is shown as mean + S.E.M of the density in the Western blot of mouse retina homogenates. ** P<0.01 as compared to naive (un-injected animals);
**Figure 16** shows expression of TrkB (A) and BDNF (B) transgenes in mouse retinal ganglion cell layer as shown by immunocytochemistry following injection of QTA020V, a rAAV2 vector containing the coding for the TrkB receptor and BDNF, separated by the viral-2A peptide sequence;
**Figure 17** shows retinal ganglion cell (RGC) survival following optic nerve crush (ONC) in the mouse versus control animals treated with rAAV2-CAG-eGFP vector. Data shown as mean + S.E.M. for average numbers of retinal ganglion cells throughout the retina per animal as counted by Brn3A-positive cells in retinal flat-mounts. ***P<0.001, *P<0.05 as compared to controls;

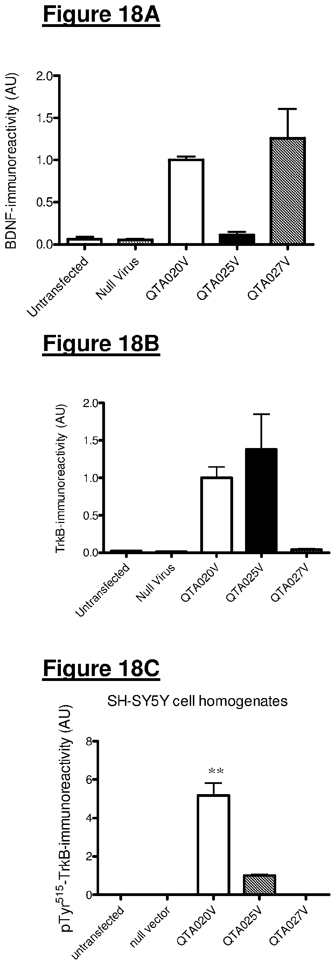

**Figure 18** shows expression of BDNF **(****Figure 18A****)** and TrkB **(****Figure 18B****)** transgenes in undifferentiated human SH-SY5Y neuroblastoma cell homogenates by Western blotting following transfection with rAAV2 viral vectors which express no transgenes (Null virus), BDNF only (QTA027V), TrkB only (QTA025V) and both BDNF and TrkB (QTA020V). **Figure 18C** shows the level of activated phosphorylated TrkB receptors in the SH-SY5Y cells in Western blots following transfection with the viral vectors Null, QTA020V, QTA025V or QTA027V. Only QTA020V vector which expresses both BDNF and TrkB was found to significantly increase the activation of TrkB receptors, as compared to untransfected cells (**P<0.01; ANOVA followed by Bonferroni modified t-tests for multiple comparisons). Data shown as mean + S.E.M. for n = 4 experiments;
**Figure 19** shows the level of apoptotic cell death of undifferentiated SH-SY5Y cells in culture following exposure to oxidative stress produced by addition of hydrogen peroxide (H₂O₂ at either 0.1mM or 1.0 mM) by TUNEL staining. Cells transfected with the rAAV2 vector QTA020V, which expresses both BDNF and TrkB receptors, prior to addition of the hydrogen peroxide were found to be significantly protected against apoptosis versus untreated cells (**P<0.01; ANOVA followed by Bonferroni modified t-tests for multiple comparisons). Data shown as mean + S.E.M. for n = 6-10; and
**Figure 20** shows representative immunocytochemical images of optic nerves obtained from P301S mutant human Tau transgenic mice and stained with antibodies which recognise phosphorylated Tau at positions serine 396/serine 404 (PHF-1) or serine 202/serine 205 (AT8). Mice were injected intravitreally with the rAAV2 vector QTA020V (which expresses both mBDNF and TrkB receptors) at 3 months old and terminated three weeks later prior to removal of optic nerves for immunocytochemistry.

### Examples

The following Examples demonstrate the use of embodiments of the present invention to promote nerve regeneration and/or survival. The teaching derivable from the uses, methods, and treatments disclosed by the Examples is also applicable to the treatment of the neurodegenerative disorders and stroke, as disclosed herein.

### Methods and Materials

### Molecular cloning and plasmid constructs

Codon optimisation of DNA sequences was performed using the on-line tool (http://www.idtdna.com/CodonOpt) and DNA blocks were synthesised by Integrated DNA technologies, Inc. (IDT; 9180 N. McCormick Boulevard, Skokie, IL 60076-2920, USA) or GenScript (860 Centennial Ave, Piscataway, NJ 08854, USA). Cloning to make the master plasmid QTA001PA and subsequent plasmids were performed using standard molecular biology and cloning techniques.

### Plasmid scale up and purification

DNA Plasmids were scaled up in SURE competent cells (Agilent Technologies; cat. #200238) overnight to provide 2.29µg/µl plasmid following maxi-prep purification. The remaining plasmids were scaled up to 500µg scale and transduction quality with minimal endotoxin presence.

### HEK293 culture and cell transduction with plasmid DNA

HEK293 cells (400,000 cells) were cultured in poly-L-lysine (10ug/mL, Sigma-Aldrich; cat. #P1274) coated 6 well plates in 1.5mL Dulbecco's minimum essential medium (DMEM) containing 10% foetal bovine serum (FBS), 1% penicillin and 1% streptomycin (1% Pen/Strep) until 80% confluent. The medium was then exchanged for 2mL DMEM (no additives). Two to three hours later, an additional 0.5ml transfection medium containing 4µg plasmid DNA plus 10µL lipofectamine (4µL/mL; Thermo Fisher Scientific; cat. #12566014) was added to each well resulting in an overall volume of 2.5ml throughout the transfection period and for supernatant collection.

### SH-SY5Y culture and cell transfection with rAAV2 viral vectors

SH-SY5Y cells were cultured in 6 well plates (300,000 cells), 96 well plates (10,000 cells) or on 13 mm glass coverslips (100,000 cells) coated with poly-L-lysine (10µg/mL, Sigma product #P1274). Dulbecco's minimum essential medium (DMEM) containing 10% foetal bovine serum (FBS), 1% penicillin and 1% streptomycin (1% Pen/Strep) was used to culture cells to 80% confluent at 37°C prior to exchange to DMEM with no additives prior to transfection. DMEM volumes used were 6 well plates (2mL), 96 well plate (100µL), coverslips (500µL). Vectors, diluted in PBS, were added directly to the culture medium at a final concentration of 1.0 x10¹⁰(VP)/mL and incubated for 48 hours at 37°C.

### Hydrogen peroxide-induced SH-SY5Y cell death and TUNEL staining

48 hours after SH-SY5Y cell transfection, medium was exchanged for fresh DMEM (no additives). Hydrogen peroxide (H₂O₂) (Thermo Fisher Scientific; product #BP2633500, lot #1378087) was diluted in filtered water (to a concentration of 0.1 or 1.0mM) and added at an equal volume to wells or plates for an additional 24 hours. Filtered water served as a vehicle control. Coverslips were washed twice in PBS and fixed for 30 min in 4% paraformaldehyde in 1M phosphate buffered saline (PBS) at room temperature.

After three more washes in PBS, cells were blocked and permeabilized by incubation in 5% normal goat serum (NGS), 3% bovine serum albumin (BSA) and 0.3% Triton X-100 in PBS for 60 minutes at room temperature. Cells were then incubated overnight at 4°C with commercial rabbit polyclonal antibodies for TrkB (Abcam; product # ab33655, lot # GR232306-1 diluted 1:500), rabbit polyclonal anti-BDNF antibodies (Santa Cruz Biotechnology Inc; product# sc-546; lot# CO915 at 1:300 dilution) or p-Tyr⁵¹⁵-TrkB (Abeam product# ab109684 lot# GR92849-4 1:750) diluted in blocking solution. Staining was revealed using secondary anti-rabbit antibodies conjugated to alexa fluor 488 (Life Technologies; product # A11034 at 1:1000) for 2 hours at room temperature. For TUNEL staining (Promega; product #G3250; lot #0000215719), cells were washed three times in PBS and immersed in TUNEL equilibration buffer for 10 minutes. The TUNEL reaction mixture was made per the manufacturers protocol and 100µL/coverslip added to cells for 1 hour at 37°C. The reaction was stopped by incubating in 1X standard citrate solution (SCS) for 15 minutes. Cell nuclei were counterstained with 1µg/mL DAPI (Thermo Scientific; product #D1306 at 1:8000). Cells were further washed three times before being mounted with fluorSave™ reagent (Calbiochem/EMD Chemicals Inc., Gibbstown, NJ, USA) prior to imaging. Imaging was carried out using a 20X objective and a Leica DM6000 epifluorescence microscope (Leica Microsystems, Wetzlar, Germany).

### BDNF measurement by ELISA

The amount of BDNF secreted from HEK293 cells was measured in cell culture medium 24 hours after transfection. Medium was centrifuged, to remove debris, and measured using a commercial Human BDNF ELISA kit (Sigma-Aldrich, product# RAB0026). BDNF concentration was determined by comparing samples to freshly made BDNF standards.

### Western blotting for BDNF and TrkB receptors

The amount of BDNF and TrkB-immunoreactivity within the HEK293 cells was measured by removing the DMEM incubation medium, washing the cells in cold phosphate buffered saline and the addition of 350µL freshly prepared lysis buffer to the wells (10ml Lysis-M reagent + 1 tablet of complete Mini Protease Inhibitor Cocktail, Roche; cat. #04719964001, + 100/µl Halt phosphatase inhibitor cocktail (100X), Thermo Scientific; cat. #78428). After cell homogenisation, the protein suspension was quantified using the BCA assay (Pierce BCA protein assay kit, Thermo Scientific; cat. #23227). Between 6µg and 15µg HEK293 cell lysate protein/lane were run down a Bis-Tris gel (12% NuPAGE Novex; cat. #NP0342BOX, Thermo Scientific) and examined by Western blotting using the primary rabbit polyclonal anti-BDNF antibodies (Santa Cruz Biotechnology Inc; product# sc-546; at 1:500 dilution), rabbit polyclonal anti-TrkB antibodies (Abcam; cat. #ab33655, used at 1:2000 dilution) or eGFP antibodies (Abeam product #ab-290 used at 1:500) which were incubated overnight. Primary antibodies were visualised with HRP conjugated anti-rabbit antibodies (Vector Laboratories; cat. #PI-1000, at 1:8000) and signal detection using ECL Prime (Amersham, GE Healthcare, UK) and an Alliance Western blot imaging system (UVItec Ltd, Cambridge, UK). For Western blots of mouse retina, eyes from vector-treated animals were homogenized in 500µL freshly prepared lysis buffer (10ml Lysis-M reagent + 1 tablet of cOmplete Mini Protease Inhibitor Cocktail, Roche product# 04719964001 + 100µl Halt phosphatase inhibitor cocktail (100X), Thermo Scientific product# 78428). Tissue was disrupted for 1 minute (Qiagen, TissueRuptor product# 9001273) and then kept on ice for an additional 15 minutes. The protein was then analysed by Western blotting as described above.

### Immunocytochemistry

HEK293 cells (70,000) were seeded on 13mm, poly-L-lysine coated coverslips within 4 well plates and incubated in DMEM containing 10% FBS and 1% Pen/Strep in 0.5ml medium. Once the cells had grown to 80% confluence, the medium was exchanged for 0.4ml DMEM (no additives) for 2-3 hours then an additional o.imL transfection medium (0.8µg plasmid DNA + 2µl lipofectamine) was added so that the final volume reached 0.5ml. Coverslips were washed twice in PBS and fixed for 30 min in 4% paraformaldehyde in 1M phosphate buffered saline (PBS) at room temperature. After three more washes in PBS, cells were blocked and permeabilized by incubation in 5% normal goat serum (NGS), 3% bovine serum albumin (BSA) and 0.3% Triton X-100 in PBS for 60 minutes at room temperature. Cells were then incubated overnight at 4°C with commercial rabbit polyclonal antibodies for BDNF (Santa Cruz Biotechnology Inc; product# sc-546; at 1:300 dilution) or TrkB (Abeam product# ab33655, diluted 1:500) diluted in blocking solution. Staining was revealed using secondary anti-rabbit antibodies conjugated to alexa fluor 647 (Invitrogen, product# A21248 at 1:1000) for 2 hours at room temperature. Cell nuclei were also counterstained with 1µg/ml DAPI (Thermo Scientific, product# D1306 at 1:8000). Cells were further washed three times before being mounted with fluorSave™ reagent (Calbiochem/EMD Chemicals Inc., Gibbstown, NJ, USA) prior to imaging. Imaging was carried out using a 20X objective and a Leica DM6000 epifluorescence microscope (Leica Microsystems, Wetzlar, Germany) or a Leica SP5 confocal microscope (Leica Microsystems, Wetzlar, Germany) equipped with a 63X oil objective using a 3X digital zoom and 0.5-0.8 sequential scanning z-step interval.

For immunocytochemistry of retinal structures and optic nerves from control or vector treated animals (at between 3 or 4 weeks following injection), carefully dissected eyes were fixed in 4% paraformaldehyde/0.1% PBS (pH 7.4) overnight and dehydrated in 30% sucrose/0.1% PBS at 4°C (24 hours). Eyes were then embedded in silicon moulds containing optimal cutting temperature compound (OCT) (Sakura Finetek, Zoeterwoude, Netherlands) and frozen on dry ice. Thirteen µm sections through the dorsal-ventral/superior-inferior axis of the retina or longitudinal sections through the optic nerve of P301S mice were collected onto superfrost plus slides (VWR product#631-0108), using a Bright OTF 5000 cryostat (Bright Instruments, Huntingdon, UK). Slides were washed three times in PBS, and permeabilized in 5% normal goat serum (NGS), 3% bovine serum albumin (BSA) and 0.3% Triton X-100 in PBS for 60 minutes at room temperature. Slides were then incubated overnight at 4°C with commercial rabbit polyclonal antibodies for BDNF (Santa Cruz Biotechnology Inc; product# sc-546 1:300), TrkB (Abcam; product# ab33655 1:500), Tau Ser^{396/404} (PHF-1; generated in Cambridge 1:500) or Tau Ser^{202/205} (AT8; Invitrogen product#MN1020 1:500) diluted in blocking solution. Staining was revealed using secondary anti-rabbit antibodies conjugated to alexa fluor 647 (Invitrogen, product# A21248 at 1:1000) for 2 hours at room temperature. Retinal cell nuclei were also counterstained with 1µg/mL DAPI (Thermo Scientific, product# D1306 at 1:8000). Slides were further washed three times before being mounted with fluorSave™ reagent (Calbiochem/EMD Chemicals Inc., Gibbstown, NJ, USA) prior to imaging. Imaging was carried out using a 20X objective and a Leica DM6000 epifluorescence microscope (Leica Microsystems, Wetzlar, Germany) or a Leica SP5 confocal microscope (Leica Microsystems, Wetzlar, Germany) equipped with a 63X oil objective using a 3X digital zoom and 0.5-0.8 sequential scanning z-step interval.

### Intravitreal injections

Following a 7-10 day acclimatisation period, 12 week old C57/BL.6 or 16 week old P301S (Harlan labs, Bicester, U.K.) mice were randomised into various study groups. They were then anaesthetized with intraperitoneal injection of ketamine (50mg/kg) and xylazine (5g/kg). Topical 1% tetracaine eye drops were administered on Day 1 of the study. Pupillary dilation was achieved using 1% tropicamide eye drops. Using an operating microscope, a partial-thickness scleral pilot hole was made with a 30-gauge needle to facilitate penetration of the underlying sclera, choroid, and retina by a fine metal micropipette with a tip diameter of 30µm and a tip length of 2.5mm. The micropipette was then connected to a 10µL glass syringe (Hamilton Co., Reno, NV) prior drawing up 2µL of vector suspensions into the pipette depending on the group. Care was taken to avoid penetration of the lens or damage to the vortex veins during intravitreal injection. The injection site was aimed approximately 3mm posterior to the supero-temporal limbus. Injections were given slowly over 1 minute to allow diffusion of vector suspension. The right eye was left untouched and served as an internal contralateral control.

### Optic nerve crush (ONC)

Three weeks (21 days) after vector administration, the mice were subject to the ONC procedure, left untreated or sham-crushed. Under a binocular operating scope, a small incision was made with spring scissors in the conjunctiva beginning inferior to the globe and around the eye temporally. This exposed the posterior aspect of the globe, allowing visualization of the optic nerve. The exposed optic nerve was grasped approximately 1-3 mm from the globe with cross-action forceps (Dumont #N7 cat. #RS-5027; Roboz) for 10 s, with the only pressure from the self-clamping action to press on the nerve. After 10 s the optic nerve was released, the forceps are removed and the eye rotates back into place. 7 days after ONC, animals were culled. Both eyes from each group were fixed by placing the organ in 4% paraformaldehyde/0.1% PBS (pH 7.4) overnight. Retinal flat-mounts were then prepared following dissection of the posterior eye structure from the cornea and removal of the lens. The retinal flat-mounts were post fixed for 30 minutes in 4% paraformaldehyde/0.1% PBS and washed in 0.5% Triton X-100 in PBS. Retinas were frozen at -80°C for 10 minutes to permeate the nuclear membrane and improve antibody permeation before blocking in 10% normal donkey serum (NDS), 2% bovine serum albumin (BSA) and 2% Triton X-100 in PBS for 60 minutes at room temperature. RGCs were counterstained with antibodies against Brn3A (1:200 Santa Cruz, #SC-31984) and visualised under fluorescence microscopy using a 20X objective and a Leica DM6000 epifluorescence microscope (Leica Microsystems, Wetzlar, Germany). Higher resolution images were be obtained using a Leica SP5 confocal microscope (Leica Microsystems) equipped with a 40X oil objective using a 1.5X digital zoom and 0.5-0.8 sequential scanning z-step interval. RGC cell counts were measured by ImageJ using the image-based tool for counting nuclei plugin (ITCN) and expressed as density of RGCs/mm².

### Constructs and vectors

The inventors have generated a genetic construct, as shown in Figure 1, which may be used to treat a subject afflicted with an optic nerve pathology, such as glaucoma, or a cochlear pathology, or for promoting nerve regeneration and/or survival. The construct has been designed to maintain or increase the density of TrkB receptors on the cell surface of RGCs and maintain or increase signaling through the TrkB receptor pathway by concomitant production and local release of mBDNF.

The construct comprises transgenes encoding the TrkB receptor and its agonist, mature brain-derived neurotrophic factor. These transgenes are operably-linked to a single promoter, which is either the human synapsin I (SYN I) promoter or the CAG promoter. Advantageously, the construct of Figure 1 can be placed in a rAAV2 vector without being hindered by the size of the transgenes that it encodes. This is because the construct is orientated such that the first transgene, TrkB, is linked to the viral 2A peptide sequence followed by the BDNF signal peptide and then the mature protein. This orientation also minimises immunogenicity risks because the short N-terminal amino acid sequence of the viral 2A peptide remains attached to the intracellular portion of the TrkB receptor and the residual proline amino acid from the C-terminal viral 2A sequence remains attached to the N-terminal BDNF signal peptide and is ultimately removed from the mBDNF protein following cleavage. The vector may be placed in a pharmacologically acceptable buffered solution, which may be administered to a subject.

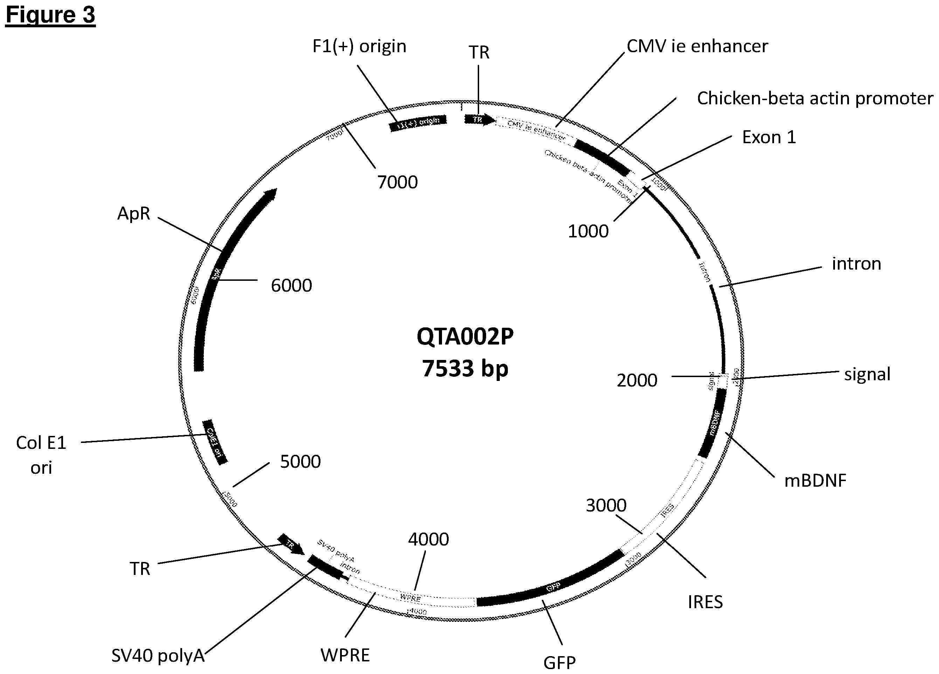

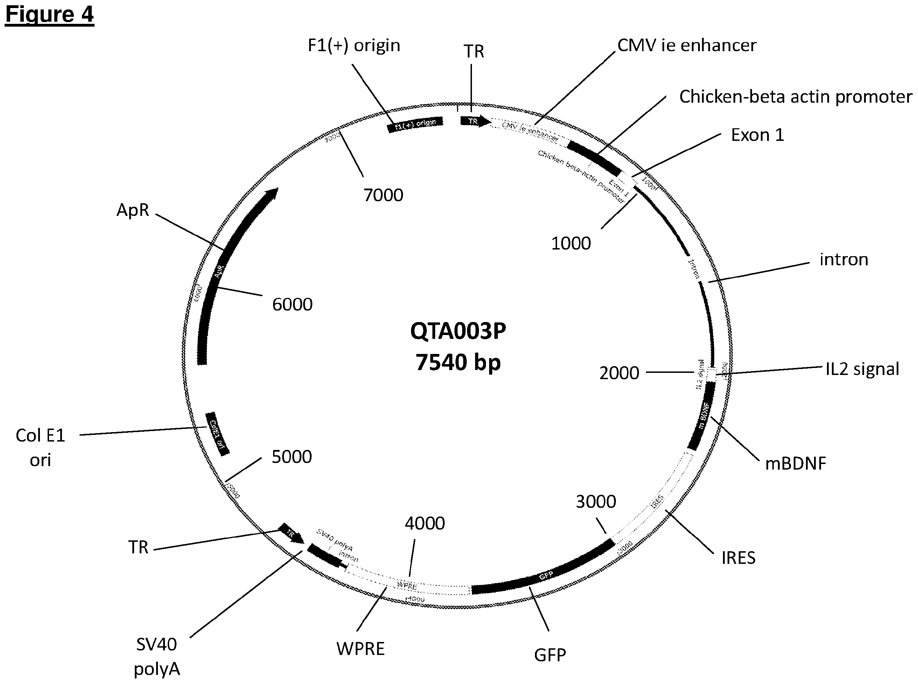

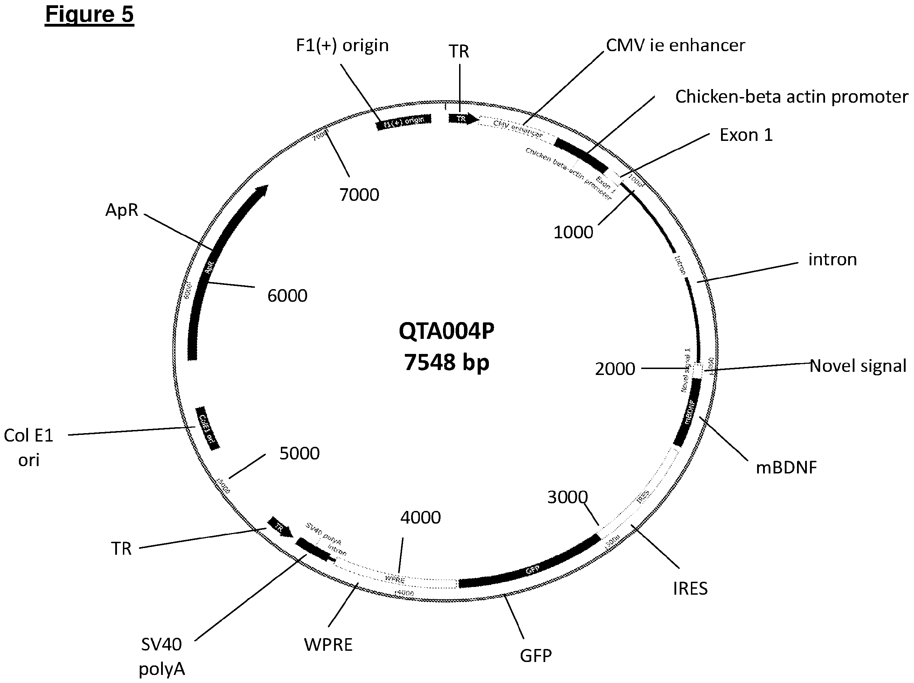

Figures 2-5 show various embodiments of expression vectors. Figure 2 shows the vector known as "Plasmid QTA001PA" containing canonical signal sequence (blue) (i.e. MTILFLTMVISYFGCMKA [SEQ ID NO:20]) plus proBDNF (red) and mBDNF (black). Figure 3 shows the vector known as "Plasmid QTA002P". It does not encode proBDNF but produces only mBDNF, and encodes the same signal sequence (blue) as QTA001PA. Figure 4 shows the vector known as "Plasmid QTA003P" which also does not encode proBDNF but produces only mBDNF. Instead of the canonical signal sequence for mBDNF, it comprises an IL-2 signal sequence (blue). Finally, Figure 5 shows the vector known as "Plasmid QTA004P". It does not encode proBDNF but instead produces only mBDNF. It also encodes a novel signal sequence (blue), [SEQ ID NO: 32].

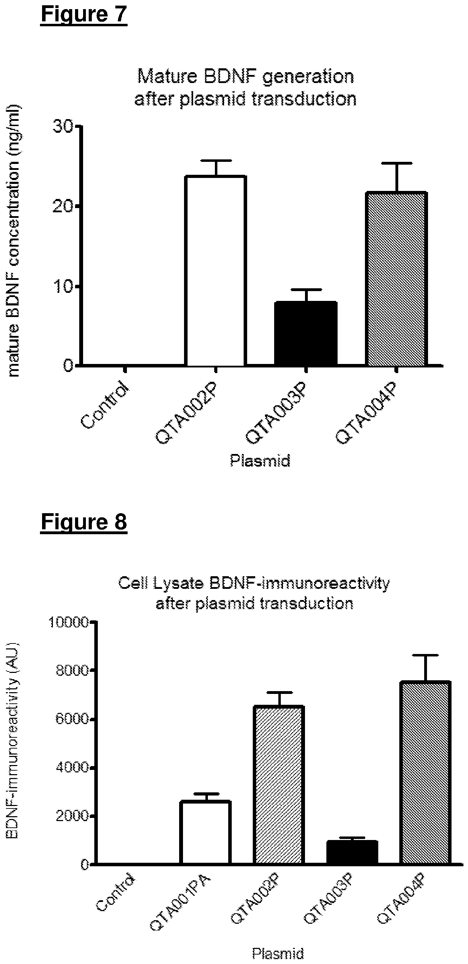

The inventors have produced and investigated the construct and vector relating to the glaucoma gene therapy concept starting with the mature BDNF (mBDNF) element. They have clearly demonstrated production and release of mBDNF from HEK293 cells following lipofectamine transduction with a plasmid which contains the BDNF sequence without the proBDNF coding region (QTA002P, see Figure 3) (see Figure 7). The mBDNF released from the cells is the predicted 14kDa monomer (measured using Western blotting and a commercially available antibody for BDNF) and there is no evidence for protein aggregates, as has been reported by several groups attempting to generate commercial amounts of mBDNF using yeast and other cell-based manufacturing approaches¹. The mBDNF is therefore released in a form which can allow the protein molecules to form non-covalent dimers in order to activate TrkB receptors.

Using an ELISA for BDNF (which does not differentiate between mBDNF and the larger extended proBDNF protein), the inventors have also demonstrated that it is possible to substitute the DNA sequence coding for the endogenous canonical 18-amino acid signal peptide sequence (MTILFLTMVISYFGCMKA) with a novel peptide sequence (QTA004P - see Figure 5) and release equivalent levels of BDNF into the HEK293 incubation medium following lipofectamine transduction of the cells with plasmids containing the BDNF gene (see Figure 7).

Substitution of the endogenous signal peptide with the sequence coding for the interleukin-2 signal peptide (QTA003P - see Figure 4) was less effective in releasing BDNF from the medium. Levels of BDNF released into the medium are currently around 1 - 2nM and concentrations of this agonist are sufficient to maximally activate the specific TrkB receptors (IC50 of around 0.9nM),. Levels of BDNF release are approximately 35-fold higher (876 ± 87 ng/mL BDNF) with the plasmid QTA001PA (see Figure 2) which contains the combined proBDNF and mBDNF sequences and which also includes the 18-amino acid canonical signal peptide as compared to the plasmids QTA002P (see Figure 3) and QTA004P (see Figure 5).

Measurements of BDNF remaining in the cell by quantitative Western blotting 24 hours after lipofectamine plasmid transduction revealed lower BDNF remaining concentrations with QTA001PA than those with QTA002P and QTA004P (see Figure 8).

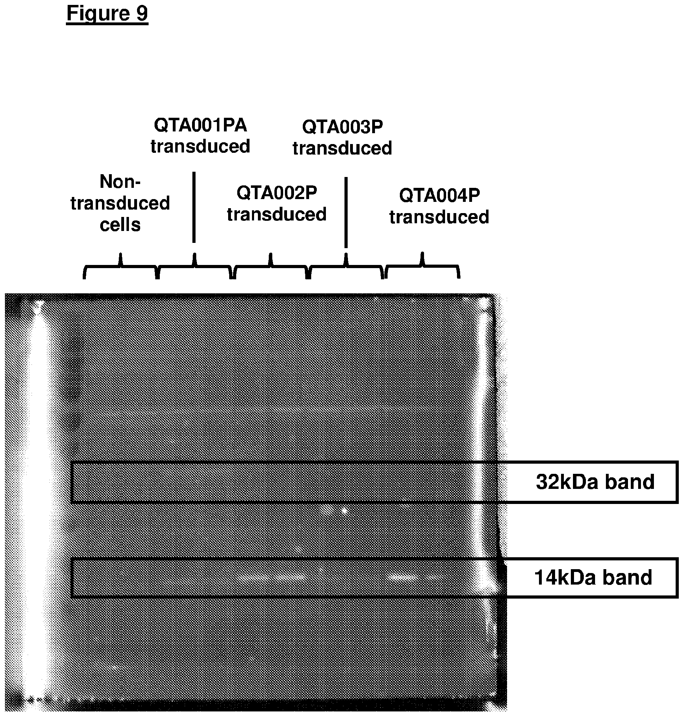

Moreover, around half of the BDNF immunoreactivity in the cell lysates transduced by QTA001PA was in the form of the proBDNF (molecular weight band at 32kDa) whereas the proBDNF band was absent in the lysates of cells transduced with QTA002P, QTA003P and QTA004P (see Figure 9), probably because these plasmids do not contain a proBDNF extended coding sequence.

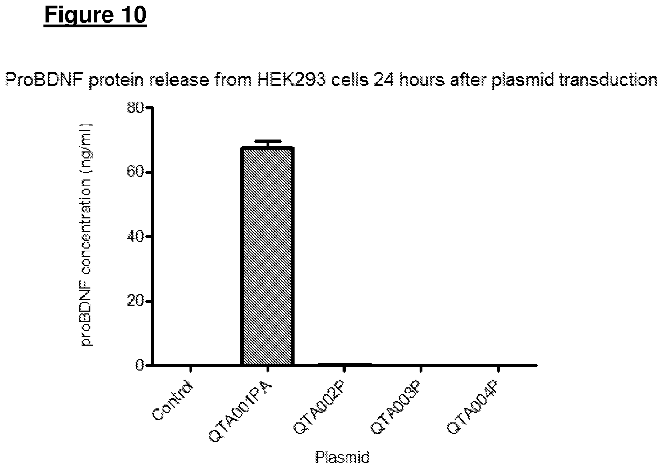

Using an ELISA specific for the proBDNF, the inventors were able to demonstrate that around 70ng/mL (2.2nM or 3.5%) of released BDNF-immunoreactivity from cells transduced by QTA001PA is in the form of proBDNF whilst the majority (96.5% or 876ng/mL / 63nM) is released as mBDNF (see Figure 10). There was no proBDNF-immunoreactivity detected from cells transduced by QTA002P, QTA003P or QTA004P which do not contain the coding sequence for the extended proBDNF.

Accordingly, it is clear that all of the plasmids are capable of producing the 14kDa mBDNF protein, but that the amounts of mBDNF released from the HEK293 cells are largely dependent on efficiency in protein storage and packaging into secretory vesicles. The extended form of the protein, containing the combined proBDNF and mBDNF sequences, as produced with plasmid QTA001PA (Figure 2) is therefore packaged into secretory vesicles and released into the incubation medium much more efficiently than with the smaller mBDNF sequences which appear to accumulate within the cell.

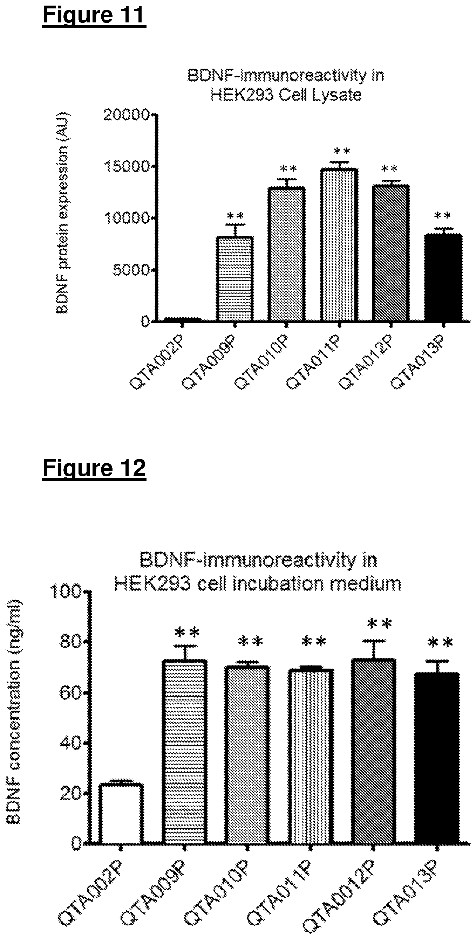

Referring to Figure 11, it shows that substitution of the coding for the endogenous canonical signal peptide sequence, as represented in plasmid QTA002P, with novel sequences included in plasmids QTA009P to QTA013P increases the concentration of BDNF in HEK293 cells 24 hours after transduction with plasmids. Figure 12 demonstrates that substitution of the endogenous canonical signal peptide coding sequence included in plasmid QTA002P with novel sequences (plasmids QTA009P to QTA013P) increases release of BDNF (as measured by ELISA) from HEK293 cells, as measured 24 hours after transduction with plasmids.

As shown in Figure 13, the addition of the viral-2A peptide sequence results in efficient processing of the coding sequence for the large precursor protein into two transgenes, eGFP and BDNF. The Western blots show HEK293 cells 24 hours after they were transduced with plasmids: (i) QTA015P (expressing BDNF and eGFP separated by an IRES spacer), (ii) QTA021P (expressing BDNF followed by eGFP separated by a functional viral-2A peptide sequence), (iii) QTA022P (expressing BDNF followed by eGFP separated by a non-functional viral-2A peptide sequence) and (iv) QTA023P (expressing eGFP followed by coding for BDNF separated by a functional viral-2A peptide sequence).

The coding sequence of QTA021P (plasmid containing codon optimised sequence for mBDNF-viral-2A peptide-eGFP) is referred to here as SEQ ID No: 104, as follows:

The coding sequence of QTA022P (plasmid containing codon optimised sequence for mBDNF-non-functional viral-2A peptide-eGFP) is referred to here as SEQ ID No: 105, as follows:

The coding sequence of QTA023P (plasmid containing codon optimised sequence for eGFP-viral-2A peptide-mBDNF) is referred to here as SEQ ID No: 106, as follows:

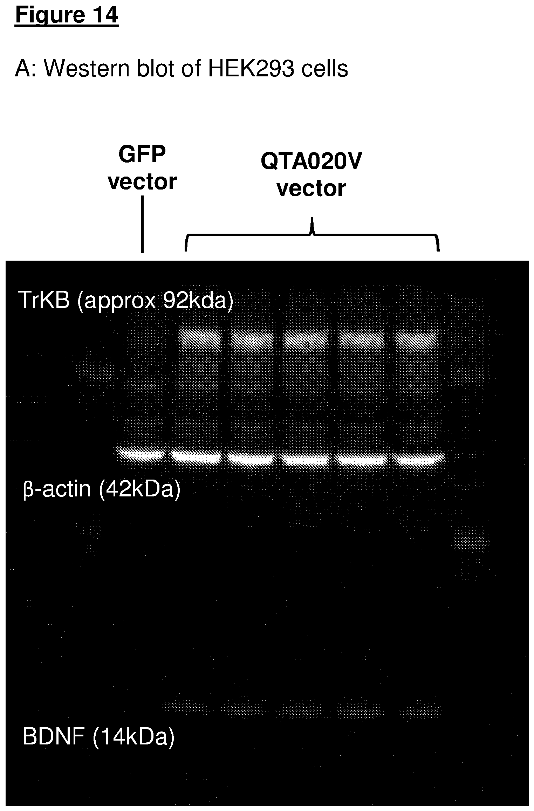

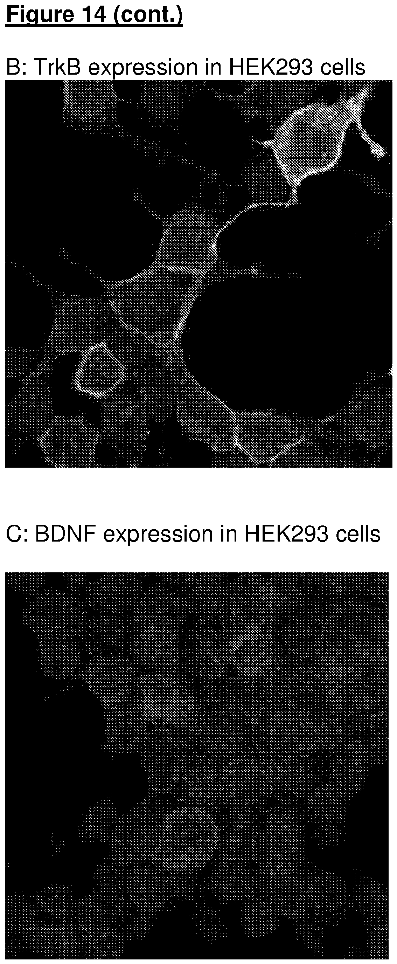

Referring to Figure 14A, there is shown a Western blot of HEK293 cell homogenates 48 hours after transfection with the QTA020V vector. It shows efficient processing of the large precursor coding region which includes the TrkB receptor and BDNF separated by the viral-2A peptide sequence. The two TrkB and mBDNF-immunoreactive transgenes are within in the predicted correct molecular weight sizes. A lack of staining of large precursor protein above the TrkB receptor band should be noted, indicating almost complete or complete processing of the precursor protein in five repeats. Figures 14B and 14C show that the transgene proteins produced after vial-2A peptide cleavage have been transported to the correct intracellular compartments in HEK293 cells after processing (TrkB receptors to the cell surface and BDNF to storage vesicles prior to release).

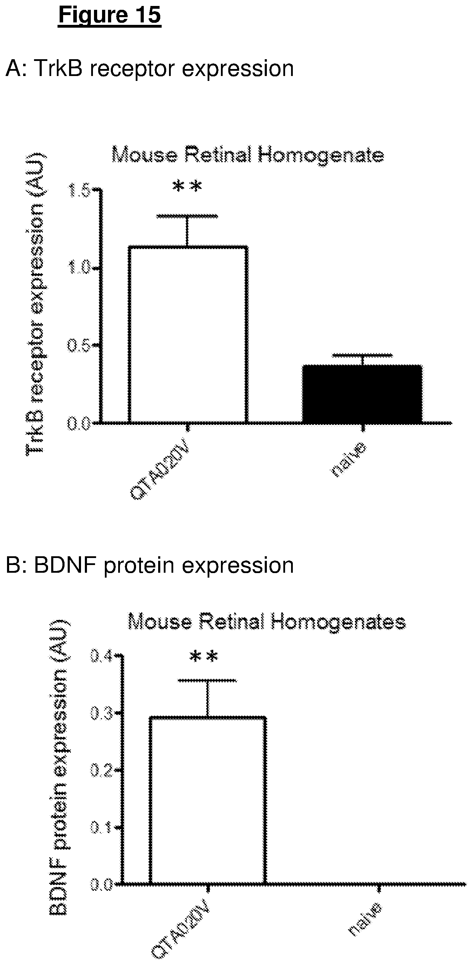

Figure 15 shows that addition of the viral-2A peptide sequence separating the two coding regions for the TrkB receptor and BDNF results in efficient processing into the two transgenes in mouse retina following intravitreal injection of the rAAV2 vector, QTA020V.

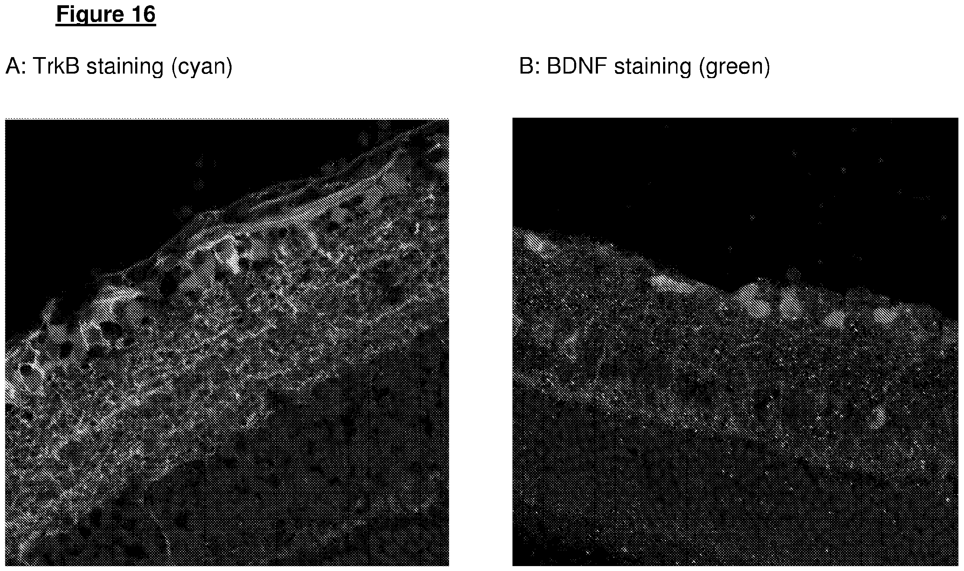

Figure 16 shows the expression of transgenes in mouse retinal ganglion cell layer as shown by immunocytochemistry following injection of QTA020V, a rAAV2 vector containing the coding for the TrkB receptor and BDNF, separated by the viral-2A peptide sequence. Target retinal ganglion cell bodies are stained red with anti-Brn3A antibodies and cell nuclei are counter-stained blue with DAPI to distinguish the retinal layers.

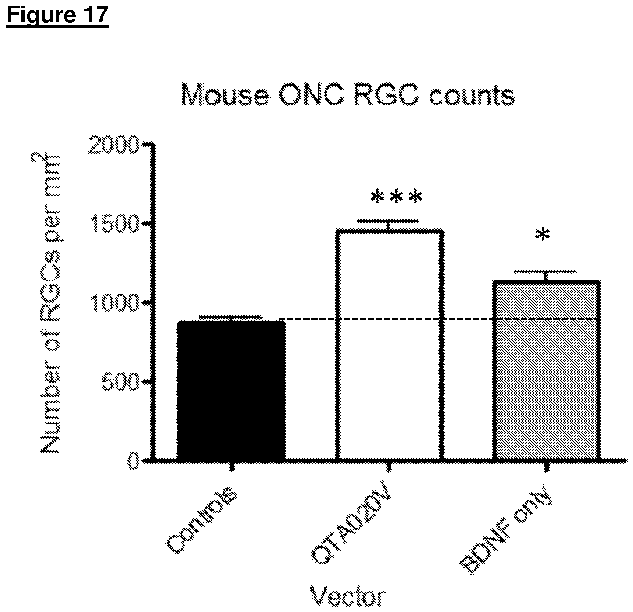

Referring to Figure 17, there is shown pre-treatment of QTA020V (containing coding for TrkB receptor and BDNF, separated by the viral-2A peptide sequence) via intravitreal injection (2µl of 9x10¹² vector particles/ml) imparts significant neuroprotective efficacy on retinal ganglion cell survival following optic nerve crush in the mouse versus control animals treated with rAAV2-CAG-eGFP vector. The level of neuroprotection by the QTA020V vector was also greater than that provided by a vector expressing only BDNF. All three groups of animals were subjected to optic nerve crush procedure and the number of retinal ganglion cells measured 7 days after the insult. Retinal ganglion cells were reduced by 71% in controls (black bars) versus animals subject to sham crush (data not shown).

### The Neuroprotective effects of the constructs

Referring to Figure 18, there are shown the expression of the BDNF transgenes (see Figure 18A) and the TrkB transgenes (see Figure 18B) in undifferentiated human SH-SY5Y neuroblastoma cell homogenates by Western blotting following transfection with rAAV2 viral vectors which express no transgenes (Null virus), BDNF only (QTA027V), TrkB only (QTA025V) and both BDNF and TrkB (QTA020V). It is clear that good levels of expression are achieved.

Referring to Figure 18C, there is shown the level of activated phosphorylated TrkB receptors in the SH-SY5Y cells in Western blots following transfection with the viral vectors Null, QTA020V, QTA025V or QTA027V. Only QTA020V vector which expresses both BDNF and TrkB was found to significantly increase the activation of TrkB receptors, as compared to untransfected cells. As such, it has been shown that the constructs of the invention effectively express both transgenes and result in activated phosphorylated TrkB receptors in the neuroblastoma SH-SY5Y cells, indicating that neurodegenerative disorders, such as Alzheimer's disease, or stroke, can be treated.

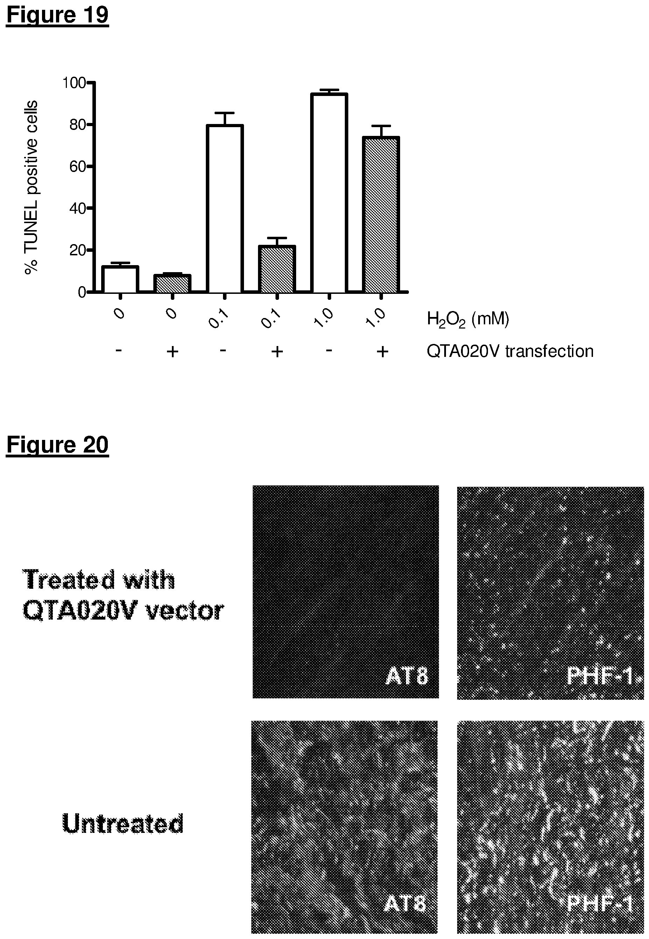

Referring to Figure 19, there is shown the level of apoptotic cell death of undifferentiated neuroblastoma SH-SY5Y cells in culture following exposure to oxidative stress produced by addition of hydrogen peroxide (H₂O₂ at either 0.1mM or 1.0 mM) by TUNEL staining. Cells transfected with the rAAV2 vector QTA020V, which expresses both BDNF and TrkB receptors, prior to addition of the hydrogen peroxide, were surprisingly found to be significantly protected against apoptosis versus untreated cells. Again, these data support the notion that the constructs of the invention can be used in the treatment, prevention or amelioration of a neurodegenerative disorder or stroke.

Referring now to Figure 20, there are shown representative immunocytochemical images of optic nerves obtained from P301S mutant human Tau transgenic mice and stained with antibodies which recognise phosphorylated Tau at positions serine 396/serine 404 (PHF-1) or serine 202/serine 205 (AT8).

P301S transgenic mice develop neuronal loss and brain atrophy by eight months, principally in the hippocampus but spreading to other brain regions, including the neocortex and entorhinal cortex. They develop widespread neurofibrillary tangle-like inclusions in the neocortex, amygdala, hippocampus, brain stem, and spinal cord. Tangle pathology is accompanied by microgliosis and astrocytosis, but not amyloid plaques [56, 57,58].

Mice were treated via intravitreal injection with QTA020V which expresses both TrkB receptors and BDNF in target retinal ganglion cells and their axons. The images in Figure 20 illustrate that the degree of Tau hyperphosphorylation, using PHF-1 and AT-8, is significantly reduced in the axons that constitute the optic nerve. These in *vivo* data show that increased expression of TrkB and BDNF, using the constructs of the invention, can significantly reduce Tau phosphorylation in neurones, which is one of the pathophysiological features associated with Alzheimer brains.

### Conclusions

It will be appreciated that for Alzheimer's disease, there is no single pre-clinical model, which is generally regarded as a surrogate for the disease and where a gene therapy may be tested with a degree of predictability towards a clinical outcome. What are available, however, are animals models in which modifications to their genome have resulted in the introduction of one of the defining genetic/neurochemical or biochemical changes into rodents which have been identified in humans with the disease. These changes include the excessive production of Aβ and formation of plaques [59] generation of a hyper-phosphorylated tau protein within neuronal cell bodies and axons which are thought to mediate axonal transport [60] and the reduction in both BDNF and its cognate receptor, TrkB [11-14, 27]

Based on human post-mortem tissue and the ability of various agents which can successfully remove beta-amyloid from both experimental animals through blockade of the BACE-1 enzyme responsible for its generation (verubecestat; Merck) or through antibody neutralisation (e.g. solenezumab; Eli Lilly and bapineuzumab; Pfizer/J&J), both of these approaches have failed to produce significant clinical benefit in Phase-III clinical studies. Therefore, of the widely described post-mortem changes in human brains diagnosed with Alzheimer's disease, loss in BDNF signalling and the presence of neurofibrillary tangles associated with hyper-phosphorylated tau are the only untested approaches to restoring or slowing pathophysiological changes associated with this neurological condition.

Using significant inventive endeavour, the inventors have addressed the problem of overcoming the loss in BDNF signalling using a novel construct which is simultaneously able to both express and up-regulate both TrkB receptors and BDNF, both of which have been reported to be reduced in this disease (see references cited above).

As BDNF has a short half-life, regular administration of recombinant BDNF, which may require several injections per day into the brain or through constant infusion, is clinically not feasible and would probably be associated with TrkB receptor down-regulation. Moreover, the inventors have also demonstrated in Figure 18C that in SHSY-5Y cells, an rAAV2 expressing TrkB receptors alone is not sufficient to significantly increase the activity of this receptor, as measured by the levels of active p-Y⁵¹⁵-TrkB staining. The constructs of the invention, which have been specifically designed to accommodate the large coding sequences of both TrkB receptor and BDNF through a number of inventive steps including: (i) loss of pro-BDBF coding, (ii) introduction of a novel signal peptide to overcome the issues associated with intracellular transport and normal protein folding of BDNF due to omission of the important Pro-BDNF sequence, (iii) constructing a single transgene containing a viral-2A peptide sequence which facilitates translational 'skipping' between the ribosomal production of TrkB and the BDNF sequences, and (iv) finally abbreviated WPRE and polyA sequences. Therefore, the inventors have provided evidence that the novel construct which expresses two transgenes, BDNF, and its cognate receptor, BDNF, is far superior to simply up-regulating TrkB receptors alone. The inventors have also demonstrated that the novel gene therapy constructs are able to provide optimal activity, as has been previously demonstrated [56], but without the requirement for additional (regular) injections of BDNF.

The inventor's main objective was to develop a gene therapy which is capable of addressing the low levels of BDNF/TrkB signalling which the examples provided clearly demonstrate. What was unexpected was that the novel gene therapy construct is capable of a major reduction in the density of hyper-phosphorylated Tau protein (measured using two antibodies which recognise several phosphorylated serine residues along the Tau protein length), as shown in Figure 20. Tau is a ubiquitous protein found in brain and other neural tissues, such as the optic nerve. Using the optic nerve as a model system, increased BDNF signalling in the eye was found to reduce the proposed pathological level of this protein isoform. Therefore, the ability to up-regulate the BDNF/TrkB signalling in the P301S transgenic mouse strain and observe such a profound reduction in the density of phosphorylated-Tau was not anticipated.

### References

1. Brookmeyer R, Johnson E, Ziegler-Graham K, Arrighi HM. Forecasting the global burden of Alzheimer's disease (2007). Alzheimers. Dement., vol. 3(3) PP:186-191.
2. Braak H, Braak E. (1991). Neuropathological stageing of Alzheimer-related changes. Acta Neuropathol., vol. 82(4), PP:239-259.
3. Dekosky ST, Scheff SW. (1990). Synapse loss in frontal cortex biopsies in Alzheimer's disease: correlation with cognitive severity. Ann. Neurol., vol. 27(5), PP:457-464.
4. Chao MV (2003). Neurotrophins and their receptors: A convergence point for many signalling pathways. Nature Rev. Neurosci. vol. 4, PP: 299-309.
5. Dawbarn D, Allen SJ, (2003). Neurotrophins and neurodegeneration. Neuropathol. Appl. Neurobiol., vol.29, PP: 211-230.
6. Phillips HS, Hains JM, Laramee GR, Rosenthal A, Winslow JW (1990). Widespread expression of BDNF but not NT3 by target areas of basal forebrain cholinergic neurons. Science, vol. 250(4978), PP: 290-294.
7. Wetmore C, Ernfors P, Persson H, Olson L, (1990). Localization of brain-derived neurotrophic factor mRNA to neurons in the brain by in situ hybridization. Exp. Neurol., vol. 109(2), PP: 141-152
8. Ghosh A, Carnahan J, Greenberg ME, (1994). Requirement for BDNF in activity-dependent survival of cortical neurons. Science, vol. 263(5153), PP: 1618-1623.
9. Phillips HS, Hains JM, Armanini M, Laramee GR, Johnson SA, Winslow JW, (1991). BDNF mRNA is decreased in the hippocampus of individuals with Alzheimer's disease. Neuron, vol. 7(5), PP: 695-702.
10. Holsinger RM, Schnarr J, Henry P, Castelo VT, Fahnestock M, (2000). Quantitation of BDNF mRNA in human parietal cortex by competitive reverse transcription-polymerase chain reaction: decreased levels in Alzheimer's disease. Brain Res. Mol. Brain Res., vol. 76(2), PP: 347-354.
11. Narisawa-Saito M, Wakabayashi K, Tsuji S, Takahashi H, Nawa H, (1996). Regional specificity of alterations in NGF, BDNF and NT-3 levels in Alzheimer's disease. Neuroreport, vol. 7(18), PP: 2925-2928.
12. Connor B, Young D, Yan Q, Faull R L, Synek B, Dragunow M, (1997). Brain-derived neurotrophic factor is reduced in Alzheimer's disease. Brain Res. Mol. Brain Res., vol. 49(1-2), PP: 71-81.
13. Ferrer I, Marin C, Rey M J, Ribalta T, Goutan E, Blanco R, Tolosa E, Marti E, (1999). BDNF and full-length and truncated TrkB expression in Alzheimer disease. Implications in therapeutic strategies. J. Neuropathol. Exp. Neurol., vol. 58(7), PP: 729-739.
14. Savaskan E, Muller-Spahn F, Olivieri G, Bruttel S, Otten U, Rosenberg C, Hulette C, Hock C, (2000). Alterations in trk A, trk B and trk C receptor immunoreactivities in parietal cortex and cerebellum in Alzheimer's disease. Eur. Neurol., vol. 44(3), PP: 172-180.
15. Peng S, Wuu J, Mufson E J, Fahnestock M, (2005). Precursor form of brain-derived neurotrophic factor and mature brain-derived neurotrophic factor are decreased in the pre-clinical stages of Alzheimer's disease. J. Neurochem., vol. 93(6) PP: 1412-1421.
16. Michalski B, Fahnestock M, (2003). Pro-brain-derived neurotrophic factor is decreased in parietal cortex in Alzheimer's disease. Brain Res. Mol. Brain Res., vol. 111(1-2), PP: 148-154.
17. Qin XY, Cao C, Cawley NX, Liu TT, Yuan J, Loh YP, Cheng Y, (2016). Decreased peripheral brain-derived neurotrophic factor levels in Alzheimer's disease: A meta-analysis study (N = 7277). Mol. Psychiatry, vol. 22, PP: 312-320.
18. Forlenza OV, Diniz BS, Teixeira AL, Radanovic M, Talib LL, Rocha NP, Gattaz WF, (2015). Lower Cerebrospinal Fluid Concentration of Brain-Derived Neurotrophic Factor Predicts Progression from Mild Cognitive Impairment to Alzheimer's Disease. Neuromolecular Med., vol. 17(3), PP: 326-332.
19. Feher A, Juhasz A, Rimanoczy A, Kalman J, Janka Z, (2009). Association between BDNF Val66Met polymorphism and Alzheimer disease, dementia with Lewy bodies, and Pick disease. Alzheimer Dis. Assoc. Disord., vol. 23(3) PP: 224-228.
20. Peng S, Wuu J, Mufson EJ, Fahnestock M, (2005). Precursor form of brain-derived neurotrophic factor and mature brain-derived neurotrophic factor are decreased in the pre-clinical stages of Alzheimer's disease. J. Neurochem., vol. 93, PP: 1412-1421.
21. Michalski B, Corrada MM, Kawas CH, Fahnestock M, (2015). Brain-derived neurotrophic factor and TrkB expression in the "oldest-old", the 90+ study: Correlation with cognitive status and levels of soluble amyloid-β. Neurobiol. Aging, vol. 36, PP: 3130-3139.
22. Elliott E, Atlas R, Lange A, Ginzburg I, (2005). Brain-derived neurotrophic factor induces a rapid dephosphorylation of tau protein through a PI-3 kinase signalling mechanism. Eur. J. Neurosci., vol. 22, PP: 1081-1089.
23. Jiao S-S, Shen L-L, Zhu C, Bu X-L, Liu Y-H, Liu C-H, Yao X-Q, Zhang L-L, Zhou H-D, Walker DG, Tan J, Gortz J, Zhou X-F, Wang Y-J, (2016). Brain-derived neurotrophic factor protects against tau-related neurodegeneration of Alzheimer's disease. Transl. Psychiatry, vol. 6, e907.
24. Rosa E, Mahendram S, Ke YD, Ittner LM, Ginsberg SD, Fahnestock M, (2016). Tau downregulates BDNF expression in animal and cellular models of Alzheimer's disease. Neurobiol. Aging, vol. 48,PP: 135-142.
25. Lim JY, Reighard CP, Crowther DC, (2015). The pro-domains of neurotrophins, including BDNF, are linked to Alzheimer's disease through a toxic synergy with Aβ. Hum. Mol. Genet., vol., 24(14) PP:3929-3938.
26. Hashimoto R, Hirata Y, Asada T, Yamashita F, Nemoto K, Mori T, Moriguchi Y, Kunugi H, Arima K, Ohnishi T, (2009). Effect of the brain-derived neurotrophic factor and the apolipoprotein E polymorphisms on disease progression in preclinical Alzheimer's disease. Genes Brain Behav., vol. 8(1) PP: 43-52.
27. Allen S J, Wilcock G K, Dawbarn D, (1999). Profound and selective loss of catalytic TrkB immunoreactivity in Alzheimer's disease. Biochem. Biophys. Res. Commun., vol. 264(3) PP: 648-651.
28. Kemppainen S, Rantamaki T, Jeronimo-Santos A, Lavasseur G, Autio H, Karpova N, Karkkainen E, Staven S,Vicente Miranda H, Outeiro TF, Diógenes MJ, Laroche S, Davis S, Sebastião AM, Castrén E, Tanila H, (2012). Impaired TrkB receptor signaling contributes to memory impairment in APP/PS1 mice. Neurobiol. Aging, vol. 33, e1123-e1139.
29. Devi L, Ohno M (2015) TrkB reduction exacerbates Alzheimer's disease-like signalling aberrations and memory deficits without affecting β-amyloidosis in 5XFAD mice. Transl. Psychiatry, vol. 5, e562.
30. Tong L, Balazs R, Thornton PL, Cotman CW, (2004). β-Amyloid peptide at sublethal concentrations downregulates brain-derived neurotrophic factor functions in cultured cortical neurons. J. Neurosci., vol. 24, PP:6799-6809.
31. Liu XH, Geng Z, Yan J, Li T, Chen Q, Zhang QY, Chen ZY, (2015). Blocking GSK3β-mediated dynamin1 phosphorylation enhances BDNF-dependent TrkB endocytosis and the protective effects of BDNF in neuronal and mouse models of Alzheimer's disease. Neurobiol. Dis., vol. 74, PP:377-391.
32. Poon WW, Blurton-Jones M, Tu CH, Feinberg LM, Chabrier MA, Harris JW, Jeon NL, Cotman CW, (2011). β-amyloid impairs axonal BDNF retrograde trafficking. Neurobiol. Aging, vol. 32, PP: 821-833.
33. Gong B, Cao Z, Zheng P, Vitolo OV, Liu S, Staniszewski A, Moolman D, Zhang H, Shelanski M, Arancio O, (2006). Ubiquitin hydrolase Uch-L1 rescues β-amyloid-induced decreases in synaptic function and contextual memory. Cell, vol. 126, PP: 775-788.
34. Gan KJ, Silverman MA, (2015). Dendritic and axonal mechanisms of Ca2+ elevation impair BDNF transport in aβ oligomer-treated hippocampal neurons. Mol. Biol. Cell, vol. 26, PP: 1058-1071.
35. Zhang L, Fang Y, Lian Y, Chen Y, Wu T, Zheng Y, Zong H, Sun L, Zhang R, Wang Z, Xu Y, (2015). Brain-derived neurotrophic factor ameliorates learning deficits in a rat model of Alzheimer's disease induced by aβ1-42., PLoS One, vol. 10(4), eo122415.
36. Wu Y, Luo X, Liu X, Liu D, Wang X, Guo Z, Zhu L, Tian Q, Yang X, Wang JZ, (2015). Intraperitoneal Administration of a Novel TAT-BDNF Peptide Ameliorates Cognitive Impairments via Modulating Multiple Pathways in Two Alzheimer's Rodent Models. Sci. Rep., vol. 5, PP: 15032.
37. Nagahara AH, Merrill DA, Coppola G, Tsukada S, Schroeder BE, Shaked GM, Wang L, Blesch A, Kim A, Conner JM, Rockenstein E, Chao MV, Koo EH, Geschwind D, Masliah E, Chiba AA, Tuszynski MH, (2009). Neuroprotective effects of brain-derived neurotrophic factor in rodent and primate models of Alzheimer's disease. Nat. Med., vol. 15(3), PP: 331-337.
38. Mey J, Thanos S, (1993). Intravitreal injections of neurotrophic factors support the survival of axotoinized retinal ganglion cells in adult rats in vivo. Brain Res., vol. 602, PP: 304-317.
39. Mansour-Rabaey S, Clarke DB, Wang Y-C, Bray GM, Aguayo AJ, (1994). Effects of ocular injury and administration of brain-derived neurotrophic factor on survival and regrowth of axotomized retinal ganglion cells. Proc. Natl. Acad. Sci. USA., vol. 91, PP: 1632-1636.
40. Peinado-Ramon P, Salvador M, Viϋegas-Perez MP, Vidal-Sanz M, (1996). Effects of axotomy and intraocular administration of NT-4, NT-3 and brain-derived neurotrophic factor on the survival of adult rat retinal ganglion cells. A quantitative in vivo study. Invest Ophthalmol. Vis. Sci., vol. 37, PP: 489-500.
41. Di Polo A, Aigner LJ, Dunn RJ, Bray GM, Aguayo AJ, (1998). Prolonged delivery of brain-derived neurotrophic factor by adenovirus- infected Müller cells temporarily rescues injured retinal ganglion cells. Proc. Natl. Acad. Sci. USA., vol. 95, PP: 3978-3983.
42. Klocker N, Kermer P, Weishaupt JH, Labes M, Ankerhold R, Bähr M, (2000). Brain-derived neurotrophic factor-mediated neuroprotection of adult rat retinal ganglion cells in vivo does not exclusively depend on phosphatidyl-inositol-3'-kinase/protein kinase B signaling. J. Neurosci., vol. 20, PP: 6962-6967.
43. Ko ML, Hu DN, Ritch R, Sharma SC, Chen CF, (2001). Patterns of retinal ganglion cell survival after brain-derived neurotrophic factor administration in hypertensive eyes of rats. Neurosci. Lett., vol. 305, PP: 139-142.
44. Chen H, Weber AJ, (2001). BDNF enhances retinal ganglion cell survival in cats with optic nerve damage. Invest Ophthamol. Vis. Sci., vol. 42, PP: 966-974.
45. Pórez MTR, Caminos E, (1995). Expression of brain-derived neurotrophic factor and its functional receptor in neonatal and adult rat retina. Neurosci. Lett., vol. 183, PP: 96-99.
46. Vecino E, Ugarte M, Nash MS, Osborne NN. (1999). NMDA induces BDNF expression in the albino rat retina in vivo. Neuroreport., vol.10, PP: 1103-1106.
47. Mowla SJ, Farhadi HF, Pareek S, Atwal JK, Morris SJ, Seidah NG, Murphy RA, (2001). Biosynthesis and post-translational processing of the precursor to brain-derived neurotrophic factor. J. Biol. Chem.,, vol. 276, PP: 12660-12666.
48. Gupta VK, You Y, Gupta VB, Klistorner A, Graham SL, (2013). TrkB receptor signalling: Implications in neurodegenerative, psychiatric and proliferative disorders. Int. J. Mol. Sci., vol.14, PP: 10122-10142
49. Teng HK, Teng KK, Lee R, Wright S, Tevar S, Almeida RD, Kermani P, Torkin R, Chen ZY, Lee FS, Kraemer RT, Nykjaer A, Hempstead BL, (2005). ProBDNF induces neuronal apoptosis via activation of a receptor complex of P75NTR and sortilin. J. Neurosci., vol. 25, PP: 5455-5463.
50. Wei Y, Zhang F, Zao J, Jiang X, Lu Q, Gao E, Wand N, (2012). Enhanced protein expression of proBDNF and proNGF in elevated intraocular pressure-induced rat retinal ischemia. Chin. Med. J., vol. 125, PP: 3875-3879.
51. Woo NH, Teng HK, Siao C-J, Chiaruttini C, Pang PT, Milner TA, Hempstead BL,Lu B, (2005). Activation of P75NTR by proBDNF facilitates hippocampal long-term depression. Nature Neurosci., vol. 8, PP: 1069-1077.
52. Lebrun-Julien F, Bertrand MJ, De Backer O, Stellwagen D, Morales CR, Di Polo A, Barker PA, (2010). ProNGF induces TNFalpha-dependent death of retinal ganglion cells through a P75NTR non-cell-autonomous signaling pathway. Proc. Natl. Acad. Sci. USA. , vol. 107, PP: 3817-3822.
53. Quigley HA, McKinnon SJ, Zack DJ, Pease ME, Kerrigan-Baumrind LA, Kerrigan DF, Mitchell RS, (2000). Retrograde axonal transport of BDNF in retinal ganglion cells is blocked by acute IOP elevation in rats. Invest. Ophthalmol. Vis. Sci., vol. 41, PP: 3460-3466.
54. Pease ME McKinnon SJ, Quigley HA, Kerrigan-Baumrind LA, Zack DJ, (2000). Obstructed axonal transport of BDNF and its receptor TRKB in experimental glaucoma. Invest. Ophthalmol. Vis. Sci., vol. 41, PP: 764-774.
55. Wei Y, Wang N, Lu Q, Zhang N, Zheng D, Li J, (2007). Enhanced protein expressions of sortilin and P75NTR in retina of rat following elevated intraocular pressure-induced retinal ischemia. Neurosci. Lett., vol. 429, PP: 169-174.
56. Cheng L, Sapieha P, Kittlerova P, Hauswith WW, Di Polo A, (2002). TrkB gene transfer protects retinal ganglion cells from axotomy-induced death in vivo. J. Neurosci., vol. 22(10), PP: 3977-3986.
57. YoshiyamaY, Higuchi M, Zhang B, Huang SM, Iwata N, Saido TC, Maeda J, Suhara T, Trojanowski TC, Lee VM, (2007).. Synapse loss and microglial activation precede tangles in a P301S Tauopathy mouse model. Neuron, vol. 53, PP: 337-351.
58. Takeuchi H, Iba M, Inoue H, Higuchi M, Takao K, Tsukita K, Karatsu Y, Iwamoto Y, Miyakama T, Suhara T, Trojanowski JQ, Lee VM, Takahashi R, (2011). P301S mutant human Tau transgenic mice manifest early symptoms of human tauopathies with dementia and altered sensorimotor gating. PLoS One, vol. 6(6), 021050.
59. Hardy J, Allsop D, (1991). Amyloid deposition as the central event in the aetiology of Alzheimer's disease, Trends Pharmacol. Sci., vol. 12(10), PP:383-388.
60. Mudher A, Lovestone S, (2002). Alzheimer's disease - do tauists and baptists finally shake hands?. Trends Neurosci., vol. 25(1), PP: 22-26.

## Claims

1. A recombinant vector comprising a genetic construct comprising a promoter operably linked to a first coding sequence, which encodes the tyrosine kinase receptor B (TrkB), and a second coding sequence, which encodes an agonist of the TrkB receptor, wherein the agonist is mature BDNF or mature NT-4, wherein the second coding sequence comprises a nucleotide sequence encoding a signal peptide which boosts secretion of the agonist of the TrkB receptor, and wherein the genetic construct comprises a spacer sequence disposed between the first and second coding sequences, which spacer sequence encodes a peptide spacer that is configured to be digested to thereby produce the TrkB receptor and agonist as separate molecules, for use in the treatment, prevention or amelioration of a neurodegenerative disorder.

2. A recombinant vector, for use according to claim 1, wherein the genetic construct comprises a nucleotide sequence encoding Woodchuck Hepatitis Virus Post-transcriptional Regulatory Element (WHPE), optionally wherein the WHPE comprises a nucleic acid sequence substantially as set out in SEQ ID No: 57 or 58, or a fragment or variant with at least 65% sequence identity to SEQ ID No: 57 or 58, and/or wherein the construct comprises a nucleotide sequence encoding a polyA tail, optionally wherein
the polyA tail comprises a nucleic acid sequence substantially as set out in SEQ ID No: 59, or a fragment or variant with at least 65% sequence identity to SEQ ID No: 59.

3. A recombinant vector, for use according to any preceding claim, wherein:
i) the promoter is the human synapsin I (SYN I) promoter, optionally wherein the promoter comprises a nucleotide acid sequence substantially as set out in SEQ ID No: 1, or a fragment or variant with at least 65% sequence identity to SEQ ID No: 1; or
ii) the promoter is the CAG promoter, optionally wherein the promoter comprises a nucleotide acid sequence substantially as set out in SEQ ID No: 2, 3 or 48, or a fragment or variant thereof at least 65% sequence identity to SEQ ID No: 2, 3 or 48.

4. A recombinant vector, for use according to any preceding claim, wherein the spacer sequence comprises and encodes a viral peptide spacer sequence, more preferably a viral 2A peptide spacer sequence.

5. A recombinant vector, for use according to any preceding claim, wherein:
i) the peptide spacer sequence comprises an amino acid sequence as set out in SEQ ID NO.4, or a fragment or variant with at least 65% sequence identity to SEQ ID No: 4;
ii) the spacer sequence comprises a nucleotide sequence as set out in SEQ ID NO.5, or a fragment or variant thereof with at least 65% sequence identity to SEQ ID No: 5;
iii) the peptide spacer sequence comprises an amino acid sequence as set out in SEQ ID NO.6, or a fragment or variant with at least 65% sequence identity to SEQ ID No: 6;
iv) the spacer sequence comprises a nucleotide sequence as set out in SEQ ID NO.7, or a fragment or variant with at least 65% sequence identity to SEQ ID No: 7; or
v) the peptide spacer sequence comprises an amino acid sequence as set out in SEQ ID NO.8, or a fragment or variant with at least 65% sequence identity to SEQ ID No: 8.

6. A recombinant vector, for use according to any preceding claim, wherein the first coding sequence comprises a nucleotide sequence encoding the human canonical isoform of TrkB, wherein the canonical isoform of TrkB comprises an amino acid sequence as set out in SEQ ID NO.9, or a fragment or variant at least 65% sequence identity to SEQ ID No: 9, and/or wherein the first coding sequence comprises a nucleotide sequence as set out in SEQ ID NO. 10, or a fragment or variant with at least 65% sequence identity to SEQ ID No: 10.

7. A recombinant vector, for use according to any preceding claim, wherein the first coding sequence comprises a nucleotide sequence which encodes isoform 4 of TrkB, optionally wherein isoform 4 of TrkB comprises an amino acid sequence as set out in SEQ ID NO. 11, or a fragment or variant with at least 65% sequence identity to SEQ ID No: 11.

8. A recombinant vector, for use according to either claim 7, wherein the first coding sequence comprises a nucleotide sequence as set out in SEQ ID NO. 12, or a fragment or variant with at least 65% sequence identity to SEQ ID No: 12.

9. A recombinant vector, for use according to any preceding claim, wherein the first coding sequence comprises a nucleotide sequence according to SEQ ID No: 9, wherein one or more tyrosine residue at position 516, 701, 705, 706 and/or 816 of SEQ ID No: 9 is modified to a different amino acid residue, optionally wherein at least two, three or four tyrosine residues at position 516, 701, 705, 706 and/or 816 of SEQ ID No: 9 are modified tc a different amino acid residue.

10. A recombinant vector, for use according to claim 9, wherein all five tyrosine residues at position 516, 701, 705, 706 and/or 816 of SEQ ID No: 9 are modified to a different amino acid residue, and/or wherein the or each tyrosine residue is modified to a glutamic acid.

11. A recombinant vector, for use according to either claim 9 or claim 10, wherein the modified form of the TrkB receptor comprises an amino acid sequence as set out in SEQ ID NO. 13, or a fragment or variant with at least 65% sequence identity to SEQ ID No: 13, optionally wherein the first coding sequence comprises a nucleotide sequence as set out in SEQ ID NO. 14, or a fragment or variant with at least 65% sequence identity to SEQ ID No: 14.

12. A recombinant vector, for use according to any preceding claim, wherein the second coding sequence encodes neurotrophin-4 (NT-4), which comprises an amino acid sequence as set out in SEQ ID NO: 49 or 55, or a fragment or variant with at least 65% sequence identity to SEQ ID No: 49 or 55, and/or the second coding sequence comprises a nucleotide sequence as set out in SEQ ID No: 50 or 56, or a fragment or variant with at least 65% sequence identity to SEQ ID No: 50 or 56.

13. A recombinant vector, for use according to any preceding claim, wherein the second coding sequence comprises a nucleotide sequence which encodes mature BDNF.

14. A recombinant vector, for use according to claim 13, wherein mature BDNF comprises an amino acid sequence as set out in SEQ ID NO. 18, or a fragment or variant with at least 65% sequence identity to SEQ ID No: 18, optionally wherein the second coding sequence comprises a nucleotide sequence as set out in SEQ ID NO. 19, or a fragment or variant with at least 65% sequence identity to SEQ ID No: 19.

15. A recombinant vector, for use according to any preceding claim, wherein the second coding sequence comprises a nucleotide sequence encoding a signal peptide for the agonist of the TrkB receptor, most preferably a signal peptide for BDNF, optionally wherein:
i) the nucleotide sequence encodes the canonical signal peptide for BDNF, wherein the second coding sequence comprises a nucleotide sequence which encodes a signal peptide comprising an amino acid sequence as set out in SEQ ID NO. 20, or a fragment or variant with at least 65% sequence identity to SEQ ID No: 20; or
ii) the second coding sequence comprises a nucleotide sequence as set out in SEQ ID NO. 21, or a fragment or variant with at least 65% sequence identity to SEQ ID No: 21.

16. A recombinant vector, for use according to any preceding claim, wherein the second coding sequence comprises a nucleotide sequence encoding a signal sequence peptide as set out in any one of SEQ ID NO. 23, 25, 27 or 29, or wherein the signal peptide comprises an amino acid sequence as set out in any one of SEQ ID NO. 22, 24, 26 or 28, and/or wherein the second coding sequence comprises a nucleotide sequence encoding a signal sequence peptide as set out in any one of SEQ ID NO. 31, 33, 35, 37, 39, 41, 43, 45, 61, 63, 65, 67 69, 71, 73, 75, 77, 79, 81, 83, 85, 87, 89, 91, 93, 95, 97, 99, 101 or 103; or wherein the signal peptide comprises an amino acid sequence as set out in any one of SEQ ID NO. 30, 32, 34, 36, 38, 40, 42, 44, 60, 62, 64, 66, 68, 70, 72, 74, 76, 78, 80, 82, 84, 86, 88, 90, 92, 94, 96, 98, 100 or 102.

17. A recombinant vector, for use according to any preceding claim, wherein the construct comprises a nucleotide sequence as set out in SEQ ID No: 107 or 108, or a fragment or variant with at least 65% sequence identity to SEQ ID No: 107 or 108.

18. A recombinant vector, for use according to any preceding claim, wherein the vector is a recombinant AAV (rAAV) vector, optionally wherein the rAAV is AAV-i, AAV-2, AAV-3A, AAV-3B, AAV-4, AAV-5, AAV-6, AAV-7, AAV-8, AAV-9, AAV-10 or AAV-11, optionally wherein the rAAV is rAAV serotype-2.

19. A recombinant vector, for use according to any one of claims 1-18, wherein the neurodegenerative disorder is selected from a group consisting of: Alexander's disease, Alper's disease, Alzheimer's Disease, amyotrophic lateral sclerosis (ALS), ataxia telangiectasia, neuronal ceroid lipofuscinoses, Batten disease, bovine spongiform encephalopathy (BSE), Canavan disease, cerebral palsy, Cockayne syndrome, corticobasal degeneration, Creutzfeldt-Jakob disease, frontotemporal lobar degeneration, Gaucher's disease, Huntington's disease, HIV-associated dementia, Kennedy's disease, Krabbe's disease, Lewy body dementia, lysosomal storage disorders, neuroborreliosis, Machado-Joseph disease, motor neurone disease, multiple system atrophy, multiple sclerosis, multiple sulfatase deficiency, mucolipidoses, narcolepsy, Niemann-Pick type C, Niemann Pick disease, Parkinson's Disease, Pelizaeus-Merzbacher Disease, Pick's disease, Pompe disease, primary lateral sclerosis, prion diseases, progressive supranuclear palsy, Refsum's disease, Sandhoff disease, Schilder's disease, subacute combined degeneration of spinal cord secondary to pernicious anaemia, Spielmeyer-Vogt-Sjogren-Batten disease, spinocerebellar ataxia, spinal muscular atrophy, Steele-Richardson-Olszewski disease, Tabes dorsalis, and Tay-Sachs disease.

20. A recombinant vector, for use according to any one of claims 1-19, wherein the neurodegenerative disorder is Alzheimer's disease, optionally wherein Tau phosphorylation in neurones is reduced.

## Patentansprüche

1. Rekombinanter Vektor, umfassend ein genetisches Konstrukt, umfassend einen Promotor, der funktionsfähig mit einer ersten codierenden Sequenz verbunden ist, die den Tyrosinkinase-Rezeptor B (TrkB) codiert, und einer zweiten codierenden Sequenz, die einen Agonisten des TrkB-Rezeptors codiert, wobei der Agonist reifer BDNF oder reifes NT-4 ist, wobei die zweite codierende Sequenz eine Nukleotidsequenz umfasst, die ein Signalpeptid codiert, das Sekretion des Agonisten des TrkB-Rezeptors verstärkt, und wobei das genetische Konstrukt eine Spacersequenz umfasst, die zwischen der ersten und der zweiten codierenden Sequenz angeordnet ist, wobei die Spacersequenz einen Peptidspacer codiert, der konfiguriert ist, um verdaut zu werden, um dadurch den TrkB-Rezeptor und den Agonisten als separate Moleküle zu erzeugen, zur Verwendung bei der Behandlung, Prävention oder Besserung einer neurodegenerativen Störung.

2. Rekombinanter Vektor zur Verwendung nach Anspruch 1, wobei das genetische Konstrukt eine Nukleotidsequenz umfasst, die das posttranskriptionelle regulatorische Element des Woodchuck-Hepatitis-Virus (WHPE) codiert, wobei optional das WHPE eine Nukleinsäuresequenz umfasst, die im Wesentlichen ist wie dargelegt in SEQ ID No: 57 oder 58, oder ein Fragment oder eine Variante mit mindestens 65% Sequenzidentität zu SEQ ID No: 57 oder 58, und/oder wobei das Konstrukt eine Nukleotidsequenz umfasst, die einen PolyA-Schwanz codiert, wobei optional der PolyA-Schwanz eine Nukleinsäuresequenz umfasst, die im Wesentlichen ist wie dargelegt in SEQ ID No: 59, oder ein Fragment oder eine Variante mit mindestens 65% Sequenzidentität zu SEQ ID No: 59.

3. Rekombinanter Vektor zur Verwendung nach einem vorhergehenden Anspruch, wobei:
i) der Promotor der humane Synapsin-I-(SYN I-)Promotor ist, wobei optional der Promotor eine Nukleotidsäuresequenz umfasst, die im Wesentlichen ist wie dargelegt in SEQ ID No: 1, oder ein Fragment oder eine Variante mit mindestens 65% Sequenzidentität zu SEQ ID No: 1; oder
ii) der Promotor der CAG-Promotor ist, wobei optional der Promotor eine Nukleotidsäuresequenz umfasst, die im Wesentlichen ist wie dargelegt in SEQ ID No: 2, 3 oder 48, oder ein Fragment oder eine Variante davon mindestens 65% Sequenzidentität zu SEQ ID No: 2, 3 oder 48.

4. Rekombinanter Vektor zur Verwendung nach einem vorhergehenden Anspruch, wobei die Spacersequenz eine virale Peptid-Spacersequenz, bevorzugter eine virale 2A-Peptid-Spacersequenz, umfasst und codiert.

5. Rekombinanter Vektor zur Verwendung nach einem vorhergehenden Anspruch, wobei:
i) die Peptid-Spacersequenz eine Aminosäuresequenz wie dargelegt in SEQ ID NO. 4, oder ein Fragment oder eine Variante mit mindestens 65% Sequenzidentität zu SEQ ID No: 4 umfasst;
ii) die Spacersequenz eine Nukleotidsequenz wie dargelegt in SEQ ID NO. 5, oder ein Fragment oder eine Variante davon mit mindestens 65% Sequenzidentität zu SEQ ID No: 5 umfasst;
iii) die Peptid-Spacersequenz eine Aminosäuresequenz wie dargelegt in SEQ ID NO. 6, oder ein Fragment oder eine Variante mit mindestens 65% Sequenzidentität zu SEQ ID No: 6 umfasst;
iv) die Spacersequenz eine Nukleotidsequenz wie dargelegt in SEQ ID NO. 7, oder ein Fragment oder eine Variante mit mindestens 65% Sequenzidentität zu SEQ ID No: 7 umfasst; oder
v) die Peptid-Spacersequenz eine Aminosäuresequenz wie dargelegt in SEQ ID NO. 8, oder ein Fragment oder eine Variante mit mindestens 65% Sequenzidentität zu SEQ ID No: 8 umfasst.

6. Rekombinanter Vektor zur Verwendung nach einem vorhergehenden Anspruch, wobei die erste codierende Sequenz eine Nukleotidsequenz umfasst, welche die humane kanonische Isoform von TrkB codiert, wobei die kanonische Isoform von TrkB eine Aminosäuresequenz wie dargelegt in SEQ ID NO. 9, oder ein Fragment oder eine Variante mit mindestens 65% Sequenzidentität zu SEQ ID No: 9 umfasst, und/oder wobei die erste codierende Sequenz eine Nukleotidsequenz wie dargelegt in SEQ ID NO. 10, oder ein Fragment oder eine Variante mit mindestens 65% Sequenzidentität zu SEQ ID No: 10 umfasst.

7. Rekombinanter Vektor zur Verwendung nach einem vorhergehenden Anspruch, wobei die erste codierende Sequenz eine Nukleotidsequenz umfasst, die Isoform 4 von TrkB codiert, wobei optional Isoform 4 von TrkB eine Aminosäuresequenz wie dargelegt in SEQ ID NO. 11, oder ein Fragment oder eine Variante mit mindestens 65% Sequenzidentität zu SEQ ID No: 11 umfasst.

8. Rekombinanter Vektor zur Verwendung nach Anspruch 7, wobei die erste codierende Sequenz eine Nukleotidsequenz wie dargelegt in SEQ ID NO. 12, oder ein Fragment oder eine Variante mit mindestens 65% Sequenzidentität zu SEQ ID No: 12 umfasst.

9. Rekombinanter Vektor zur Verwendung nach einem vorhergehenden Anspruch, wobei die erste codierende Sequenz eine Nukleotidsequenz gemäß SEQ ID No: 9 umfasst, wobei ein oder mehrere Tyrosinreste an Position 516, 701, 705, 706 und/oder 816 von SEQ ID No: 9 zu einem anderen Aminosäurerest modifiziert sind, wobei optional mindestens zwei, drei oder vier Tyrosinreste an Position 516, 701, 705, 706 und/oder 816 von SEQ ID No: 9 zu einem anderen Aminosäurerest modifiziert sind.

10. Rekombinanter Vektor zur Verwendung nach Anspruch 9, wobei alle fünf Tyrosinreste an Position 516, 701, 705, 706 und/oder 816 von SEQ ID No: 9 zu einem anderen Aminosäurerest modifiziert sind, und/oder wobei der oder jeder Tyrosinrest zu einer Glutaminsäure modifiziert ist.

11. Rekombinanter Vektor zur Verwendung nach Anspruch 9 oder Anspruch 10, wobei die modifizierte Form des TrkB-Rezeptors eine Aminosäuresequenz wie dargelegt in SEQ ID NO. 13, oder ein Fragment oder eine Variante mit mindestens 65% Sequenzidentität zu SEQ ID No: 13 umfasst, wobei optional die erste codierende Sequenz eine Nukleotidsequenz wie dargelegt in SEQ ID NO. 14, oder ein Fragment oder eine Variante mit mindestens 65% Sequenzidentität zu SEQ ID No: 14 umfasst.

12. Rekombinanter Vektor zur Verwendung nach einem vorhergehenden Anspruch, wobei die zweite codierende Sequenz Neurotrophin-4 (NT-4) codiert, das eine Aminosäuresequenz wie dargelegt in SEQ ID NO: 49 oder 55, oder ein Fragment oder eine Variante mit mindestens 65% Sequenzidentität zu SEQ ID No: 49 oder 55 umfasst, und/oder die zweite codierende Sequenz eine Nukleotidsequenz wie dargelegt in SEQ ID No: 50 oder 56, oder ein Fragment oder eine Variante mit mindestens 65% Sequenzidentität zu SEQ ID No: 50 oder 56 umfasst.

13. Rekombinanter Vektor zur Verwendung nach einem vorhergehenden Anspruch, wobei die zweite codierende Sequenz eine Nukleotidsequenz umfasst, die reifen BDNF codiert.

14. Rekombinanter Vektor zur Verwendung nach Anspruch 13, wobei reifer BDNF eine Aminosäuresequenz wie dargelegt in SEQ ID NO. 18, oder ein Fragment oder eine Variante mit mindestens 65% Sequenzidentität zu SEQ ID No: 18 umfasst, wobei optional die zweite codierende Sequenz eine Nukleotidsequenz wie dargelegt in SEQ ID NO. 19, oder ein Fragment oder eine Variante mit mindestens 65% Sequenzidentität zu SEQ ID No: 19 umfasst.

15. Rekombinanter Vektor zur Verwendung nach einem vorhergehenden Anspruch, wobei die zweite codierende Sequenz eine Nukleotidsequenz umfasst, die ein Signalpeptid für den Agonisten des TrkB-Rezeptors codiert, am meisten bevorzugt ein Signalpeptid für BDNF, wobei optional:
i) die Nukleotidsequenz das kanonische Signalpeptid für BDNF codiert, wobei die zweite codierende Sequenz eine Nukleotidsequenz umfasst, die ein Signalpeptid codiert, das eine Aminosäuresequenz wie dargelegt in SEQ ID NO. 20, oder ein Fragment oder eine Variante mit mindestens 65% Sequenzidentität zu SEQ ID No: 20 umfasst; oder
ii) die zweite codierende Sequenz eine Nukleotidsequenz wie dargelegt in SEQ ID NO. 21, oder ein Fragment oder eine Variante mit mindestens 65% Sequenzidentität zu SEQ ID No: 21 umfasst.

16. Rekombinanter Vektor zur Verwendung nach einem vorhergehenden Anspruch, wobei die zweite codierende Sequenz eine Nukleotidsequenz, die ein Signalsequenzpeptid codiert, wie dargelegt in einer der SEQ ID NO. 23, 25, 27 oder 29 umfasst, oder wobei das Signalpeptid eine Aminosäuresequenz wie dargelegt in einer der SEQ ID NO. 22, 24, 26 oder 28 umfasst, und/oder wobei die zweite codierende Sequenz eine Nukleotidsequenz, die ein Signalsequenzpeptid codiert, wie dargelegt in einer der SEQ ID NO. 31, 33, 35, 37, 39, 41, 43, 45, 61, 63, 65, 67, 69, 71, 73, 75, 77, 79, 81, 83, 85, 87, 89, 91, 93, 95, 97, 99, 101 oder 103 umfasst; oder wobei das Signalpeptid eine Aminosäuresequenz wie dargelegt in einer der SEQ ID NO. 30, 32, 34, 36, 38, 40, 42, 44, 60, 62, 64, 66, 68, 70, 72, 74, 76, 78, 80, 82, 84, 86, 88, 90, 92, 94, 96, 98, 100 oder 102 umfasst.

17. Rekombinanter Vektor zur Verwendung nach einem vorhergehenden Anspruch, wobei das Konstrukt eine Nukleotidsequenz wie dargelegt in SEQ ID No: 107 oder 108, oder ein Fragment oder eine Variante mit mindestens 65% Sequenzidentität zu SEQ ID No: 107 oder 108 umfasst.

18. Rekombinanter Vektor zur Verwendung nach einem vorhergehenden Anspruch, wobei der Vektor ein rekombinanter AAV-(rAAV-)Vektor ist, wobei optional der rAAV AAV-1, AAV-2, AAV-3A, AAV-3B, AAV-4, AAV-5, AAV-6, AAV-7, AAV-8, AAV-9, AAV-10 oder AAV-11 ist, wobei optional der rAAV rAAV-Serotyp-2 ist.

19. Rekombinanter Vektor zur Verwendung nach einem der Ansprüche 1-18, wobei die neurodegenerative Störung ausgewählt ist aus einer Gruppe bestehend aus: Alexander-Krankheit, Alper-Krankheit, Alzheimer-Krankheit, amyotropher Lateralsklerose (ALS), Ataxie telangiectasia, neuronalen Ceroid-Lipofüszinosen, Batten-Krankheit, boviner spongiformer Enzephalopathie (BSE), Canavan-Krankheit, Zerebralparese, Cockayne-Syndrom, kortikobasaler Degeneration, Creutzfeldt-Jakob-Krankheit, frontotemporaler Lobärdegeneration, Gaucher-Krankheit, Huntington-Krankheit, HIV-assoziierter Demenz, Kennedy-Krankheit, Krabbe-Krankheit, Lewy-Körper-Demenz, lysosomalen Speicherkrankheiten, Neuroborreliose, Machado-Joseph-Krankheit, Motoneuronerkrankung, Multisystematrophie, multipler Sklerose, multipler Sulfatasedefizienz, Mukolipidosen, Narkolepsie, Niemann-Pick Typ C, Niemann-Pick-Krankheit, Parkinson-Krankheit, Pelizaeus-Merzbacher-Krankheit, Pick-Krankheit, Pompe-Krankheit, primärer Lateralsklerose, Prionkrankheiten, progressiver supranukleärer Blickparese, Refsum-Krankheit, Sandhoff-Krankheit, Schilder-Krankheit, subakuter kombinierter Degeneration des Rückenmarks sekundär zu perniziöser Anämie, Spielmeyer-Vogt-Sjogren-Batten-Krankheit, spinozerebellärer Ataxie, spinaler Muskelatrophie, Steele-Richardson-Olszewski-Krankheit, Tabes dorsalis und Tay-Sachs-Krankheit.

20. Rekombinanter Vektor zur Verwendung nach einem der Ansprüche 1-19, wobei die neurodegenerative Störung Alzheimer-Krankheit ist, wobei optional Tau-Phosphorylierung in Neuronen reduziert ist.

## Revendications

1. Vecteur recombinant comprenant une construction génétique comprenant un promoteur lié de manière fonctionnelle à une première séquence codante, qui code le récepteur de tyrosine kinase B (TrkB), et à une seconde séquence codante, qui code un agoniste du récepteur TrkB, ledit agoniste étant le BDNF mature ou la NT-4 mature, ladite seconde séquence codante comprenant une séquence nucléotidique codant un peptide signal qui stimule la sécrétion de l'agoniste du récepteur TrkB, et ladite construction génétique comprenant une séquence d'espacement disposée entre les première et seconde séquences codantes, laquelle séquence d'espacement code un espaceur peptidique qui est conçu pour être digéré afin de produire ainsi le récepteur TrkB et l'agoniste sous forme de molécules distinctes, pour une utilisation dans le traitement, la prévention ou l'amélioration d'un trouble neurodégénératif.

2. Vecteur recombinant, pour une utilisation selon la revendication 1, ladite construction génétique comprenant une séquence nucléotidique codant un élément régulateur post-transcriptionnel du virus de l'hépatite de la marmotte commune (WHPE), éventuellement ledit WHPE comprenant une séquence d'acide nucléique essentiellement telle qu'indiquée dans la SEQ ID n° : 57 ou 58, ou un fragment ou variant présentant une identité de séquence d'au moins 65 % avec la SEQ ID n° : 57 ou 58, et/ou ladite construction comprenant une séquence nucléotidique codant une queue polyA, éventuellement ladite queue polyA comprenant une séquence d'acides nucléiques sensiblement telle qu'indiquée dans la SEQ ID n° : 59, ou un fragment ou variant présentant une identité de séquence d'au moins 65 % avec la SEQ ID n° : 59.

3. Vecteur recombinant, pour utilisation selon l'une quelconque des revendications précédentes :
i) ledit promoteur étant le promoteur de la synapsine I humaine (SYN I), éventuellement ledit promoteur comprenant une séquence d'acides nucléotidiques sensiblement telle qu'indiquée dans la SEQ ID n° : 1, ou un fragment ou variant présentant une identité de séquence d'au moins 65 % avec la SEQ ID n° : 1 ; ou
ii) ledit promoteur étant le promoteur CAG, éventuellement ledit promoteur comprenant une séquence d'acides nucléotidiques essentiellement telle que définie dans la SEQ ID n° : 2, 3 ou 48, ou un fragment ou un variant de celle-ci présentant une identité de séquence d'au moins 65 % avec la SEQ ID n° : 2, 3 ou 48.

4. Vecteur recombinant, pour une utilisation selon l'une quelconque des revendications précédentes, ladite séquence d'espacement comprenant et codant une séquence d'espacement de peptide viral, plus préférablement une séquence d'espacement de peptide 2A viral.

5. Vecteur recombinant, pour une utilisation selon l'une quelconque des revendications précédentes :
i) ladite séquence d'espacement de peptide comprenant une séquence d'acides aminés telle qu'indiquée dans la SEQ ID n° : 4, ou un fragment ou variant présentant une identité de séquence d'au moins 65 % avec la SEQ ID n° : 4 ;
ii) ladite séquence d'espacement comprenant une séquence nucléotidique telle qu'indiquée dans la SEQ ID n° : 5, ou un fragment ou variant de celle-ci présentant une identité de séquence d'au moins 65 % avec la SEQ ID n° : 5 ;
iii) ladite séquence d'espacement de peptide comprenant une séquence d'acides aminés telle qu'indiquée dans la SEQ ID n° : 6, ou un fragment ou variant présentant une identité de séquence d'au moins 65 % avec la SEQ ID n° : 6 ;
iv) ladite séquence d'espacement comprenant une séquence nucléotidique telle qu'indiquée dans la SEQ ID n° : 7, ou un fragment ou variant présentant une identité de séquence d'au moins 65 % avec la SEQ ID n° : 7 ; ou
v) ladite séquence d'espacement de peptide comprenant une séquence d'acides aminés telle qu'indiquée dans la SEQ ID n° : 8, ou un fragment ou variant présentant une identité de séquence d'au moins 65 % avec la SEQ ID n° : 8.

6. Vecteur recombinant, pour une utilisation selon l'une quelconque des revendications précédentes, ladite première séquence codante comprend une séquence nucléotidique codant l'isoforme canonique humaine de TrkB, ladite isoforme canonique de TrkB comprenant une séquence d'acides aminés telle qu'indiquée dans la SEQ ID n° : 9, ou un fragment ou variant présentant une identité de séquence d'au moins 65 % avec la SEQ ID n° : 9, et/ou ladite première séquence codante comprenant une séquence nucléotidique telle qu'indiquée dans la SEQ ID n° : 10, ou un fragment ou variant présentant une identité de séquence d'au moins 65 % avec la SEQ ID n° : 10.

7. Vecteur recombinant, pour une utilisation selon l'une quelconque des revendications précédentes, ladite première séquence codante comprenant une séquence nucléotidique qui code l'isoforme 4 de TrkB, éventuellement ladite isoforme 4 de TrkB comprenant une séquence d'acides aminés telle qu'indiquée dans la SEQ ID n° : 11, ou un fragment ou variant présentant une identité de séquence d'au moins 65 % avec la SEQ ID n° : 11.

8. Vecteur recombinant, pour une utilisation selon la revendication 7, ladite première séquence codante comprenant une séquence nucléotidique telle qu'indiquée dans la SEQ ID n° : 12, ou un fragment ou variant présentant une identité de séquence d'au moins 65 % avec la SEQ ID n° : 12.

9. Vecteur recombinant, pour une utilisation selon l'une quelconque des revendications précédentes, ladite première séquence codante comprenant une séquence nucléotidique conformément à la SEQ ID n° : 9, un ou plusieurs résidus tyrosine en position 516, 701, 705, 706 et/ou 816 de la SEQ ID n° : 9 étant modifié en un résidu d'acide aminé différent, éventuellement au moins deux, trois ou quatre résidus tyrosine en position 516, 701, 705, 706 et/ou 816 de la SEQ ID n° : 9 étant modifiés en un résidu d'acide aminé différent.

10. Vecteur recombinant, pour une utilisation selon la revendication 9, la totalité des cinq résidus tyrosine en position 516, 701, 705, 706 et/ou 816 de la SEQ ID n° : 9 étant modifiés en un résidu d'acide aminé différent, et/ou ledit ou chaque résidu tyrosine étant modifié en un acide glutamique.

11. Vecteur recombinant, pour une utilisation selon la revendication 9 ou la revendication 10, la forme modifiée du récepteur TrkB comprenant une séquence d'acides aminés telle qu'indiquée dans la SEQ ID n° : 13, ou un fragment ou variant présentant une identité de séquence d'au moins 65 % avec la SEQ ID n° : 13, éventuellement ladite première séquence codante comprenant une séquence nucléotidique telle qu'indiquée dans la SEQ ID n° : 14, ou un fragment ou variant présentant une identité de séquence d'au moins 65 % avec la SEQ ID n° : 14.

12. Vecteur recombinant, pour une utilisation selon l'une quelconque des revendications précédentes, ladite seconde séquence codante codant la neurotrophine-4 (NT-4), qui comprend une séquence d'acides aminés telle qu'indiquée dans la SEQ ID n° : 49 ou 55, ou un fragment ou variant présentant une identité de séquence d'au moins 65 % avec la SEQ ID n° : 49 ou 55, et/ou ladite seconde séquence codante comprenant une séquence nucléotidique telle qu'indiquée dans la SEQ ID n° : 50 ou 56, ou un fragment ou variant présentant une identité de séquence d'au moins 65 % avec la SEQ ID n° : 50 ou 56.

13. Vecteur recombinant, pour une utilisation selon l'une quelconque des revendications précédentes, ladite seconde séquence codante comprenant une séquence nucléotidique qui code le BDNF mature.

14. Vecteur recombinant, pour une utilisation selon la revendication 13, ledit BDNF mature comprenant une séquence d'acides aminés telle qu'indiquée dans la SEQ ID n° : 18, ou un fragment ou variant présentant une identité de séquence d'au moins 65 % avec la SEQ ID n° : 18, éventuellement ladite seconde séquence codante comprenant une séquence nucléotidique telle qu'indiquée dans la SEQ ID n° : 19, ou un fragment ou variant présentant une identité de séquence d'au moins 65 % avec la SEQ ID n° : 19.

15. Vecteur recombinant, pour une utilisation selon l'une quelconque des revendications précédentes, ladite seconde séquence codante comprenant une séquence nucléotidique codant un peptide signal pour l'agoniste du récepteur TrkB, idéalement un peptide signal pour le BDNF, éventuellement :
i) ladite séquence nucléotidique codant le peptide signal canonique pour le BDNF, ladite seconde séquence codante comprenant une séquence nucléotidique qui code un peptide signal comprenant une séquence d'acides aminés telle qu'indiquée dans la SEQ ID n° : 20, ou un fragment ou variant présentant une identité de séquence d'au moins 65 % avec la SEQ ID n° : 20 ; ou
ii) ladite seconde séquence codante comprenant une séquence nucléotidique telle qu'indiquée dans la SEQ ID n° : 21, ou un fragment ou variant présentant une identité de séquence d'au moins 65 % avec la SEQ ID n° : 21.

16. Vecteur recombinant, pour une utilisation selon l'une quelconque des revendications précédentes, ladite seconde séquence codante comprenant une séquence nucléotidique codant un peptide de séquence signal tel qu'indiquée dans l'une quelconque des SEQ ID n° : 23, 25, 27 ou 29, ou ledit peptide signal comprenant une séquence d'acides aminés telle qu'indiquée dans l'une quelconque des SEQ ID n° : 22, 24, 26 ou 28, et/ou ladite seconde séquence codante comprenant une séquence nucléotidique codant un peptide de séquence signal telle qu'indiquée dans l'une quelconque des SEQ ID n° : 31, 33, 35, 37, 39, 41, 43, 45, 61, 63, 65, 67, 69, 71, 73, 75, 77, 79, 81, 83, 85, 87, 89, 91, 93, 95, 97, 99, 101 ou 103; ou ledit peptide signal comprenant une séquence d'acides aminés telle qu'indiquée dans l'une quelconque des SEQ ID n° : 30, 32, 34, 36, 38, 40, 42, 44, 60, 62, 64, 66, 68, 70, 72, 74, 76, 78, 80, 82, 84, 86, 88, 90, 92, 94, 96, 98, 100 ou 102.

17. Vecteur recombinant, pour une utilisation selon l'une quelconque des revendications précédentes, ladite construction comprenant une séquence nucléotidique telle qu'indiquée dans la SEQ ID n° : 107 ou 108, ou un fragment ou variant présentant une identité de séquence d'au moins 65 % avec la SEQ ID n° : 107 ou 108.

18. Vecteur recombinant, pour une utilisation selon l'une quelconque des revendications précédentes, ledit vecteur étant un vecteur AAV recombinant (rAAV), éventuellement ledit rAAV étant AAV-1, AAV-2, AAV-3A, AAV-3B, AAV-4, AAV-5, AAV-6, AAV-7, AAV-8, AAV-9, AAV-10 ou AAV-11, éventuellement ledit rAAV étant le rAAV de sérotype-2.

19. Vecteur recombinant, pour une utilisation selon l'une quelconque des revendications 1 à 18, ledit trouble neurodégénératif étant choisi dans le groupe constitué par : la maladie d'Alexander, la maladie d'Alper, la maladie d'Alzheimer, la sclérose latérale amyotrophique (SLA), l'ataxie-télangiectasie, la céroïde-lipofuscinose neuronale, la maladie de Batten, l'encéphalopathie spongiforme bovine (ESB), la maladie de Canavan, la paralysie cérébrale, le syndrome de Cockayne, la dégénérescence corticobasale, la maladie de Creutzfeldt-Jakob, la dégénérescence lobaire fronto-temporale, la maladie de Gaucher, la maladie de Huntington, la démence associée au VIH, la maladie de Kennedy, la maladie de Krabbe, la démence à corps de Lewy, les troubles de stockage lysosomal, la neuroborréliose, la maladie de Machado-Joseph, la maladie du motoneurone, l'atrophie multi-systématisée, la sclérose en plaques, le déficit multiple en sulfatases, les mucolipidoses, la narcolepsie, la maldie de Niemann-Pick de type C, la maladie de Niemann Pick, la maladie de Parkinson, la maladie de Pelizaeus-Merzbacher, la maladie de Pick, la maladie de Pompe, la sclérose latérale primaire, les maladies à prion, la paralysie supranucléaire progressive, la maladie de Refsum, la maladie de Sandhoff, la maladie de Schilder, la dégénérescence combinée subaiguë de la moelle épinière consécutive à une anémie pernicieuse, la maladie de Spielmeyer-Vogt-Sjögren-Batten, une ataxie spinocérébelleuse, une amyotrophie spinale, la maladie de Steele-Richardson-Olszewski, le tabès dorso-lombaire et la maladie de Tay-Sachs.

20. Vecteur recombinant, pour une utilisation selon l'une quelconque des revendications 1 à 19, ledit trouble neurodégénératif étant la maladie d'Alzheimer, éventuellement la phosphorylation de Tau dans les neurones étant réduite.
